# EUROPÄISCHE PATENTANMELDUNG

(11) **EP 1 770 086 A1**
(43) Veröffentlichungstag der Anmeldung: **04.04.2007**
(21) Anmeldenummer: 05021236.4
(22) Anmeldetag: 29.09.2005
(51) Int. Cl.: C07D 249/12, C07D 243/10, C07D 403/12, C07D 401/12

(54) **Ausgewählte CGRP-Antagonisten, Verfahren zu deren Herstellung sowie deren Verwendung als Arzneimittel**

(71) Anmelder: Boehringer Ingelheim Pharma GmbH & Co. KG, 55216 Ingelheim (DE)
(72) Erfinder: Die Erfindernennung liegt noch nicht vor

(57) **Zusammenfassung**

Gegenstand der vorliegenden Erfindung sind die CGRP-Antagonisten der allgemeinen Formel I 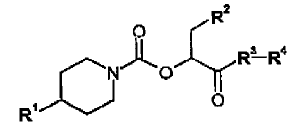 in der **R¹, R², R³** und **R⁴** wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, sowie diejenigen Verbindungen der allgemeinen Formel I, in denen ein oder mehrere Wasserstoffatome durch Deuterium ausgetauscht sind, diese Verbindungen enthaltende Arzneimittel, deren Verwendung und Verfahren zu ihrer Herstellung.

## Beschreibung

Gegenstand der vorliegenden Erfindung sind die CGRP-Antagonisten der allgemeinen Formel I 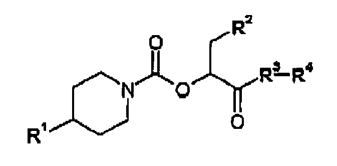 in der **R¹, R², R³** und **R⁴** wie in Anspruch 1 definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Satze mit anorganischen oder organischen Säuren oder Basen, sowie diejenigen Verbindungen der allgemeinen Formel I, in denen ein oder mehrere Wasserstoffatome durch Deuterium ausgetauscht sind, diese Verbindungen enthaltende Arzneimittel, deren Verwendung und Verfahren zu ihrer Herstellung,

### STAND DER TECHNIK

In den internationalen Patentanmeldungen PCT/EP97/04862 und PCT/EP04/000087 werden bereits CGRP-Antagonisten zur Behandlung von Migräne beschrieben.

### DETAILLIERTE BESCHREIBUNG DER ERFINDUNG

In der obigen allgemeinen Formel I bedeuten in einer ersten Ausführungsform
- **R¹**: eine Gruppe ausgewählt aus 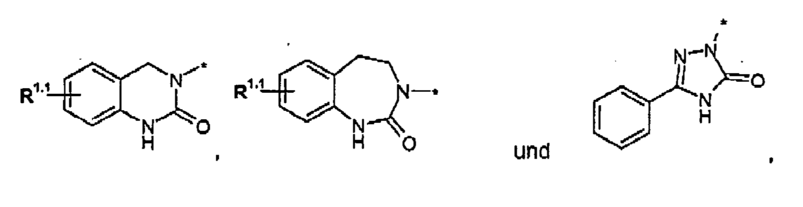 worin
**R^{1.1}** H oder H₃C-O- darstellt,
- **R²**: eine Gruppe der allgemeinen Formel II 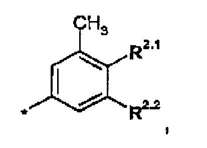 worin
**R^{2.1}** HO-, H₃CO-, H-C(O)-O- oder H₃C-C(O)-O- und
**R^{2.2}** C₁₋₂-Alkyl- oder H₃CO- darstellt,
- **R³**: **R^{3.1}**-C₁₋₈-alkylenyl-NH- und
**R^{3.1}** H, H₂N-, C₁₋₃-Alkyl-NH-, (C₁₋₃-Alkyl)₂-N- oder C₁₋₆-Alkyl-O-C(O)-NH-darstellt, oder
- **R³**: eine Gruppe der allgemeinen Formeln III 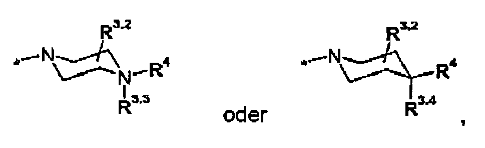 worin
**R^{3.2}** H oder C₁₋₃-Alkyl-,
**R^{3.3}** ein freies Elektronenpaar oder, wenn R⁴ nicht H darstellt oder über ein Kohlenstoffatom an R³ gebunden ist, das Sauerstoffatom,
**R^{3,4}** H oder C₁₋₃-Alkyl- darstellt, und
- **R⁴**: H, C₁₋₆-Alkyl-, H₂N-C₂₋₆-alkylenyl-, C₁₋₃-Alkyl-NH-C₂₋₆-alkylenyl-, (C₁₋₃-Alkyl)₂-N-C₁₋₆-alkylenyl-, C₃₋₆-Cycloalkyl-C₁₋₃-alkylenyl-, NH₂-, C₁₋₃-Alkyl-NH- oder (C₁₋₃-Alkyl)₂-N-, oder
- **R⁴**: eine Gruppe ausgewählt aus 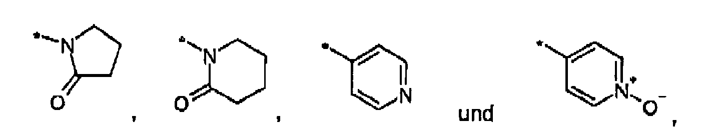 oder
- **R⁴**: eine Gruppe der allgemeinen Formeln **IV** 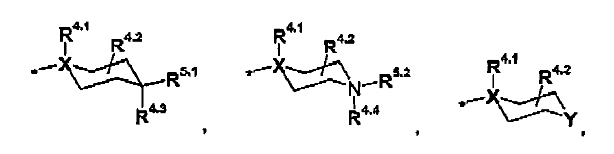 worin
**X** C und
**R^{4.1}** H, OH oder C₁₋₃-Alkyl-, oder
**X** N und
**R^{4.1}** ein freies Elektronenpaar oder das Sauerstoffatom darstellt, mit der Maßgabe, dass **X** nicht N ist, wenn **R⁴** über N an den Rest **R³** gebunden ist,
Y O, S, S(O), S(O)₂, wenn X = N ist, oder
**Y** S, S(O), S(O)₂, wenn X = C ist,
**R^{4.1}** H oder C₁₋₃-Alkyl-,
**R^{4.2}** H oder C₁₋₃-Alkyl-,
**R^{4.3}** H oder C₁₋₃-Alkyl-,
**R^{4.4}** ein freies Elektronenpaar oder, wenn R^{5.2} nicht H ist, das Sauerstoffatom,
**R^{5.1}** H, CN, OH, C₁₋₃-Alkyl-, C₁₋₃-Alkyl-C(O)-O-, C₁₋₃-Alkyl-O-, R^{5.1.1}-O-C(O)-, R^{5.1.1}-O-C(O)-C₂₋₄-alkylenyl- oder R^{5.1.1}-O-C(O)-C₁₋₃-alkylenyl-O-,
**R^{5.1.1}** H, C₁₋₆-Alkyl-, H₂N-C(O)C₁₋₃-alkylenyl-, C₁₋₃-Alkyl-NH-C(O)-C₁₋₃-alkylenyl-, (C₁₋₃-Alkyl)₂-N-C(O)-C₁₋₃alkylenyl- oder C₁₋₃-Alkyl-O-C(O)-O-C₁₋₃-alkylenyl-,
**R^{5.2}** H, C₁₋₃-Alkyl-, C₃₋₆-Cycloalkyl-, C₃₋₆-Cycloalkyl-C₁₋₃-alkylenyl-, C₁₋₃-Alkyl-C(O)-, R^{5.2.1}-O-C(O)-C₁₋₃-alkylenyl- oder R^{5.2.2}-C₂₋₄-alkylenyl-O-C(O)-C₁₋₃-alkylenyl-,
**R^{5.2.1}** H, C₁₋₆-Alkyl-, C₁₋₆-Alkyl-C(O)-O-C₁₋₃-alkylenyl-, C₁₋₃-Alkyl-O-C(O)-O-C₁₋₃-alkylenyl-, C₃₋₇-Cycloalkyl-O-C(O)-O-C₁₋₃-arkylenyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder R^{5.2.1.1}-C(O)-C₁₋₃-alkylenyl- und
**R^{5.2.1.1}** eine Gruppe ausgewählt aus 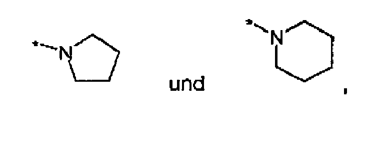 oder
**R^{5.2.1}** eine Gruppe ausgewählt aus 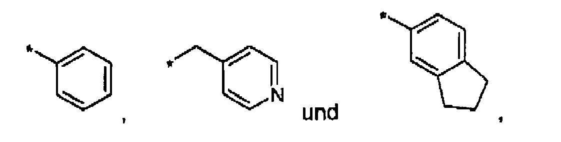 **R^{5.2.2}** H₂N, C₁₋₃-Alkyl-NH-, (C₁₋₃-Alkyl)₂-N-, C₁₋₃-Alkyl-O- oder C₁₋₃-Alkyl-O-C₂₋₄-alkylenyl-O-, oder
**R^{5.2.2}** eine Gruppe darstellt ausgewählt aus 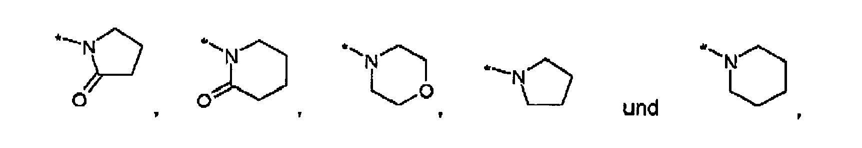
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

In der obigen allgemeinen Formel I bedeuten in einer zweiten Ausführungsform
- **R¹**: eine Gruppe ausgewählt aus 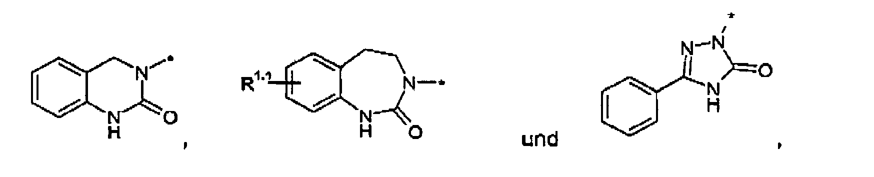 worin
**R^{1.1}** H oder H₃C-O- darstellt,
- **R²**: eine Gruppe der allgemeinen Formel II 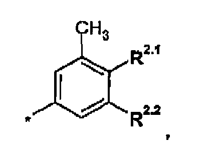 worin
**R^{2.1}** HO-, H₃CO-, H-C(O)-O- oder H₃C-C(O)-O- und
**R^{2.2}** C₁₋₂-Alkyl- oder H₃CO- darstellt,
- **R³**: R^{3.1}-C₂₋₈-alkylenyl-NH- und
**R^{3.1}** H₂N-, C₁₋₃-Alkyl-NH- oder (C₁₋₃-Alkyl)₂-N-, C₁₋₆-Alkyl-O-C(O)-NH- darstellt, oder
- **R³**: eine Gruppe der allgemeinen Formeln III 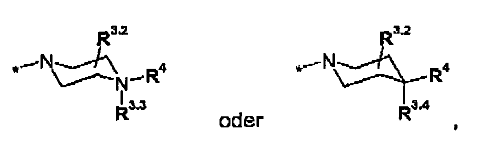 worin
**R^{3.2}** H,
**R^{3.3}** ein freies Elektronenpaar oder, wenn **R⁴** nicht H darstellt oder über ein Kohlenstoffatom an R³ gebunden ist, das Sauerstoffatom,
**R^{3.4}** H oder C₁₋₃-Alkyl- darstellt, und
- **R⁴**: H, C₁₋₆-Alkyl-, H₂N-C₂₋₆-alkylenyl-, C₁₋₃-Alkyl-NH-C₂₋₆-alkylenyl-, (C₁₋₃-Alkyl)₂-N-C₁₋₆-alkylenyl-, C₃₋₆Cycloalkyl-C₁₋₃-alkylenyl-, H₂N-, C₁₋₃-Alkyl-NH- oder (C₁₋₃-Alkyl)₂-N-, oder
- **R⁴**: eine Gruppe ausgewählt aus 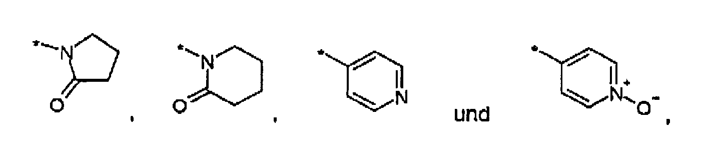 oder
- **R⁴**: eine Gruppe der allgemeinen Formeln IV 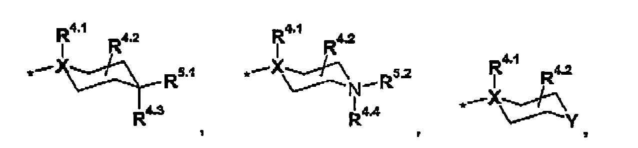 worin
**X** C und
**R^{4.1}** H, OH, C₁₋₃-Alkyl- oder
**X** N und
**R^{4.1}** ein freies Elektronenpaar oder das Sauerstoffatom darstellt, mit der Maßgabe, dass **X** nicht N ist, wenn **R⁴** über N an den Rest **R³** gebunden ist,
**Y** O, S, S(O), S(O)₂, wenn X = N ist, oder
**Y** S, S(O), S(O)₂, wenn **X** = C ist,
**R^{4.1}** H oder C₁₋₃-Alkyl-,
**R^{4.2}** H,
**R^{4.3}** H oder C₁₋₃-Alkyl-,
**R^{4.4}** ein freies Elektronenpaar oder, wenn **R^{5.2}** nicht H ist, das Sauerstoffatom,
**R^{5.1}** H, CN, OH, C₁₋₃-Alkyl-, C₁₋₃-Alkyl-C(O)-O-, C₁₋₃-Alkyl-O-, **R^{5.1.1}**-O-C(O)-, R^{5.1.1}-O-C(O)-C₂₋₄-alkylenyl- oder R^{5.1.1}-O-C(O)-C₁₋₃-alkylenyl-O-,
**R^{5.1.1}** H, C₁₋₆-Alkyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder C₁₋₃-Alkyl-O-C(O)-O-C₁₋₃-alkylenyl-,
**R^{5.2}** H, C₁₋₃-Alkyl-, C₃₋₆-Cycloalkyl-, C₃₋₈-Cycloalkyl-C₁₋₃-alkylenyl-, C₁₋₃-Alkyl-C(O)-, R^{5.2.1}-O-C(O)-C₁₋₃-alkylenyl- oder R^{5.2.2}-C₂₋₄-alkylenyl-O-C(O)-C₁₋₃-alkylenyl-,
**R^{5.2.1}** H, C₁₋₆-Alkyl-, C₁₋₆-Alkyl-C(O)-O-C₁₋₃-alkylenyl-, C₁₋₃-Alkyl-O-C(O)-O-C₁₋₃-alkylenyl-, C₃₋₇-Cycloalkyl-O-C(O)-O-C₁₋₃-alkylenyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder **R^{5.2.1.1}**-C(O)-C₁₋₃-alkylenyl- und
**R^{5.2.1.1}** eine Gruppe ausgewählt aus 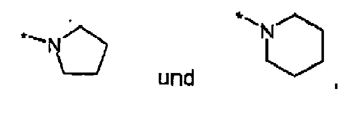 oder
**R^{5.2.1}** eine Gruppe ausgewählt aus 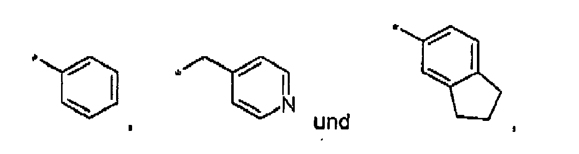 **R^{6.2.2}** H₂N, C₁₋₃-Alkyl-NH-, (C₁₋₃-Alkyl)₂-N-, C₁₋₃-Alkyl-O- oder C₁₋₃-Alkyl-O-C₂₋₄-alkylenyl-O-, oder
**R^{6.2.2}** eine Gruppe darstellt ausgewählt aus 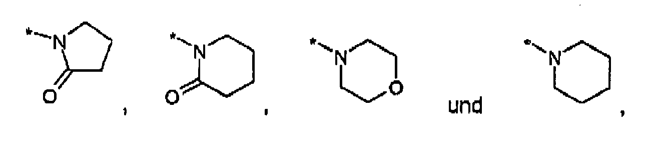
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel I, in denen
- **R¹**: eine Gruppe ausgewählt aus 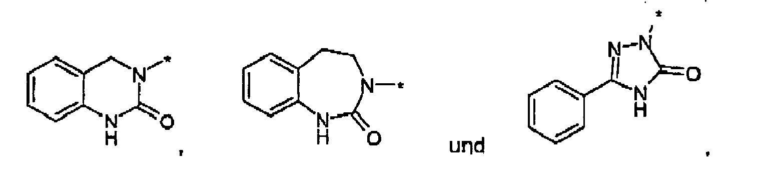
- **R²**: eine Gruppe ausgewählt aus 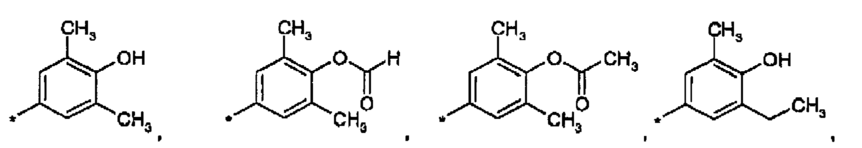 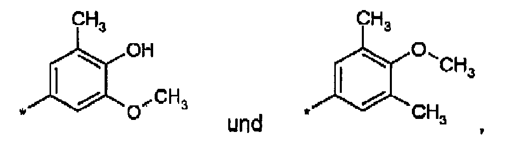
- **R³**: **R^{3.1}**-C₁₋₈-alkylenyl-NH- und
**R^{3.1}** H, H₂N-, (C₁₋₃-Alkyl)₂-N- oder C₁₋₄-Alkyl-O-C(O)-NH- darstellt, oder
- **R³**: eine Gruppe ausgewählt aus 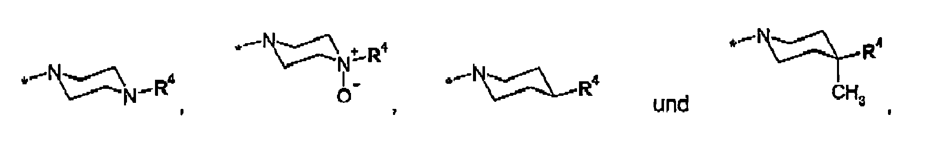 und
- **R⁴**: H, C₁₋₆-Alkyl-, (C₁₋₃-Alkyl)₂-N-C₁₋₆-alkylenyl-, C₃₋₆-Cycloalkyl-C₁₋₃-alkylenyl- oder (C₁₋₃-Alkyl)₂-N-, oder
- **R⁴**: eine Gruppe ausgewählt aus 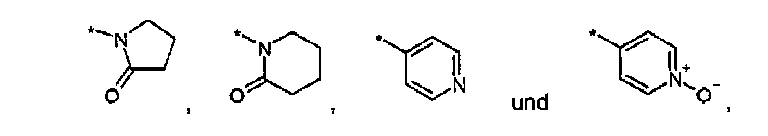 oder
- **R⁴**: eine Gruppe ausgewählt aus 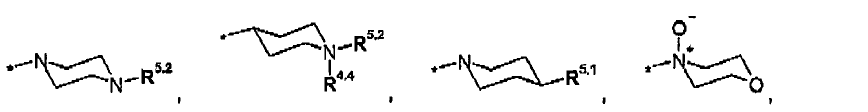 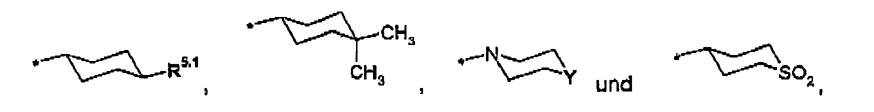 worin
**Y** O, S, S(O), S(O)₂.
**R^{5.1}** H, CN, OH, C₁₋₃-Alkyl-, C₁₋₃-Alkyl-C(O)-O-, C₁₋₃-Alkyl-O-, R^{5.1.1}-O-C(O)-, R^{6.1.1}-O-C(O)-C₂₋₄-alkylenyl- oder R^{5.1.1}-O-C(O)-C₁₋₃-alkylenyl-O-,
**R^{5.1.1}** H, C₁₋₆-Alkyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder C₁₋₃-Alkyl-O-C(O)-O-C₁₋₃-alkylenyl-,
**R^{5.2}** H, C₁₋₃-Alkyl-, Cyclopropyl-, Cyclopropyl-methylenyl-, C₁₋₃-Alkyl-C(O)-, R^{5.2.1}-O-C(O)-C₁₋₃-alkylenyl- oder R^{5.2.2}-C₂₋₄-alkylenyl-O-C(O)-C₁₋₃-alkylenyl-,
**R^{5.2.1}** H, C₁₋₆-Alkyl-, C₁₋₆-Alkyl-C(O)-O-C₁₋₃-alkylerlyl-, C₁₋₃Alkyl-O-C(O)-O-C₁₋₃-alkylenyl-, (C₁₋₃-Alkyl)₂-N-C(O)-C₁₋₃-alkylenyl- oder R^{5.2.1.1}-C(O)-C₁₋₃-alkylenyl- und
**R^{5.2.1.1}** eine Gruppe ausgewählt aus 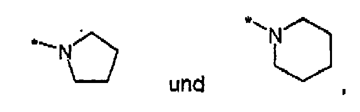 oder
**R^{5.2.1}** eine Gruppe ausgewählt aus 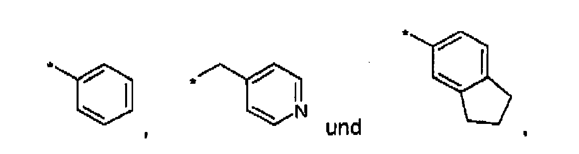 **R^{5.2.2}** (C₁₋₃-Alkyl)₂-N-, C₁₋₃-Alkyl-O- oder C₁₋₃-Alkyl-O-C₂₋₄-arkylenyl-O-, oder
**R^{5.2.2}** eine Gruppe darstellt ausgewählt aus 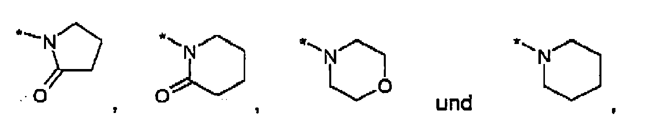 mit der Maßgabe, dass R³ und R⁴ nicht gleichzeitig Ober N aneinander gebunden sind,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel seien beispielsweise folgende Verbindungen genannt:

| **Nr.** | **Struktur** |
|---|---|
| (1) | 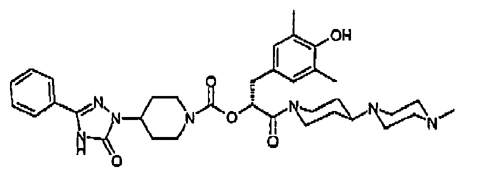 |
| (2) | 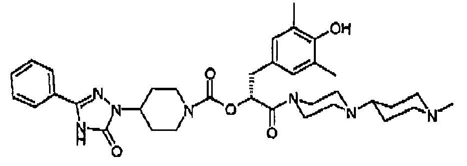 |
| (3) | 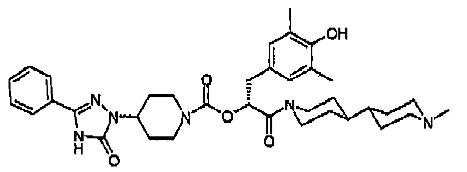 |
| (4) | 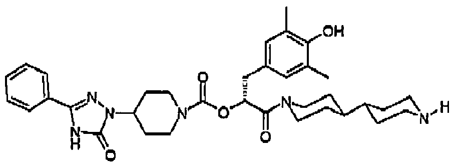 |
| (5) | 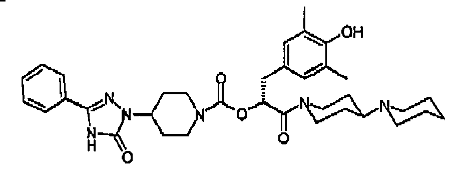 |
| (6) | 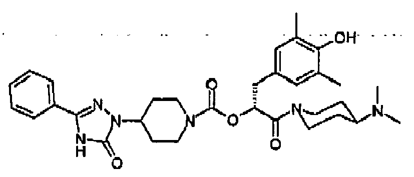 |
| (7) | 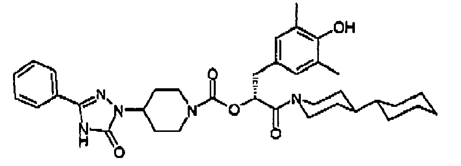 |
| (8) | 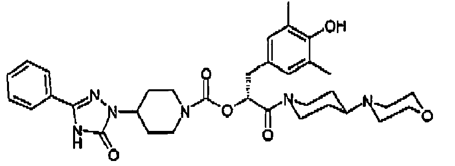 |
| (9) | 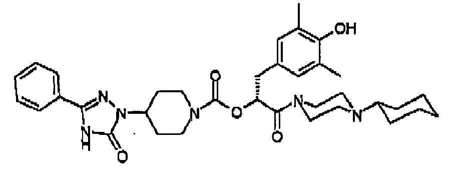 |
| (10) | 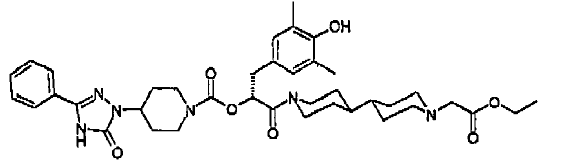 |
| (11) | 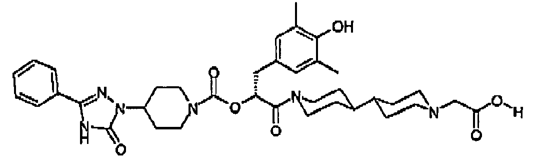 |
| (12) | 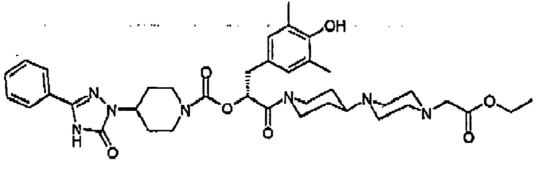 |
| (13) | 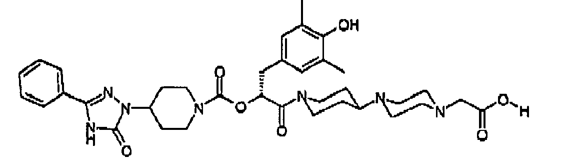 |
| (14) | 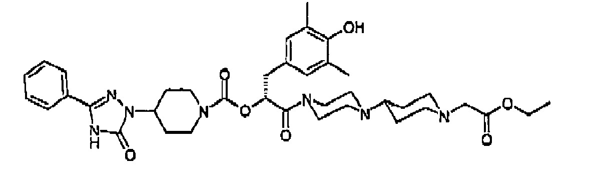 |
| (15) | 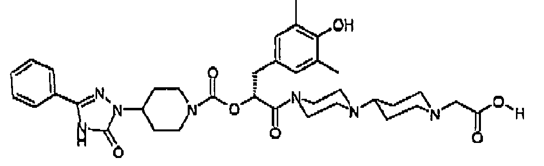 |
| (16) | 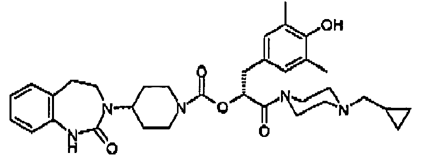 |
| (17) | 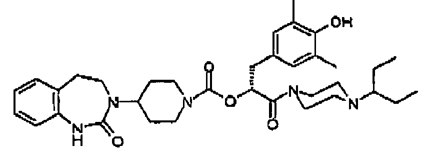 |
| (18) | 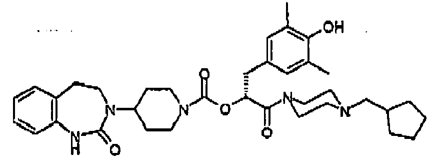 |
| (19) | 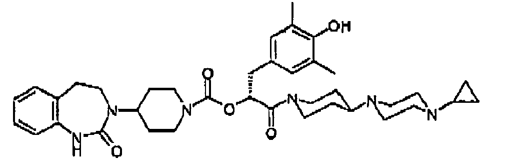 |
| (20) | 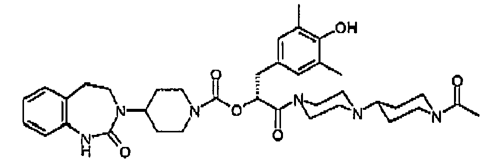 |
| (21) | 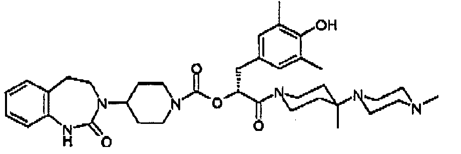 |
| (22) | 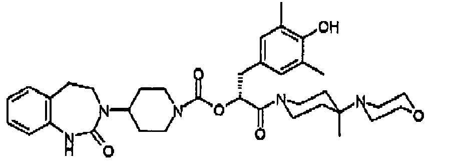 |
| (23) | 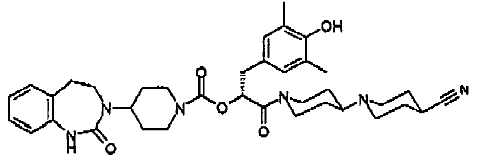 |
| (24) | 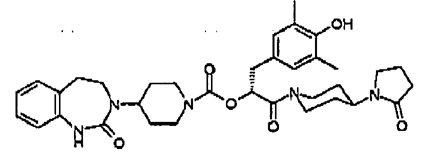 |
| (25) | 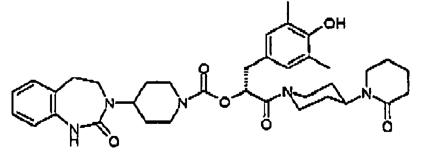 |
| (26) | 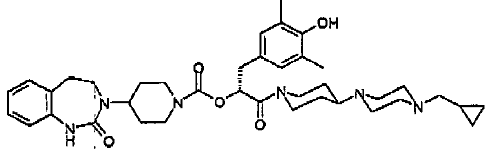 |
| (27) | 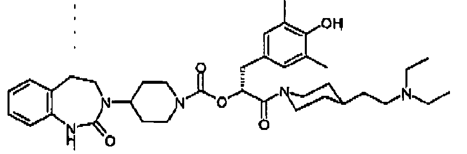 |
| (28) | 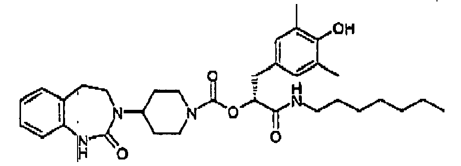 |
| (29) | 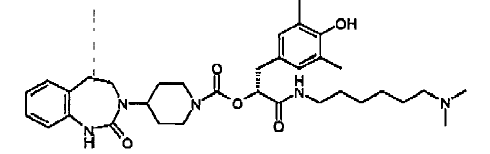 |
| (30) | 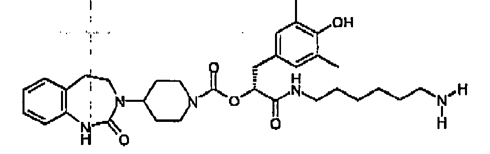 |
| (31) | 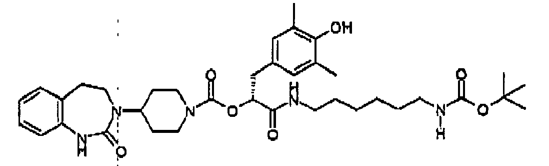 |
| (32) | 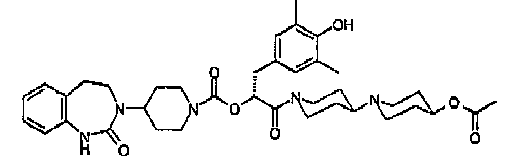 |
| (33) | 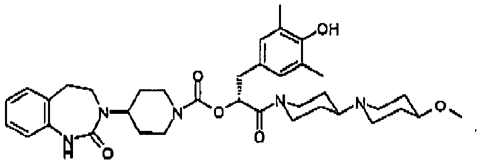 |
| (34) | 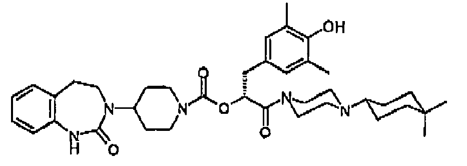 |
| (35) | 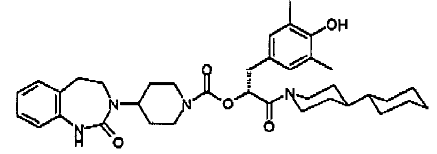 |
| (36) | 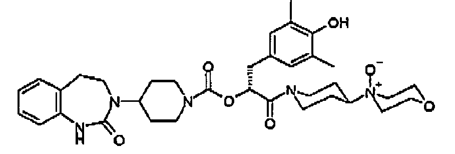 |
| (37) | 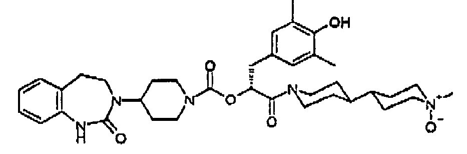 |
| (38) | 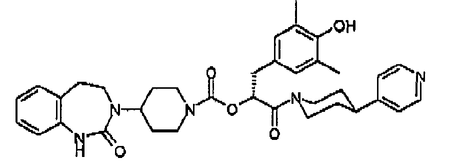 |
| (39) | 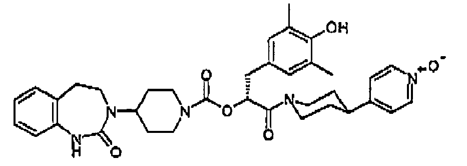 |
| (40) | 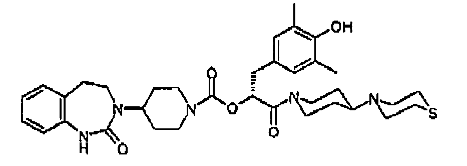 |
| (41) | 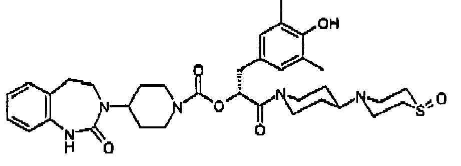 |
| (42) | 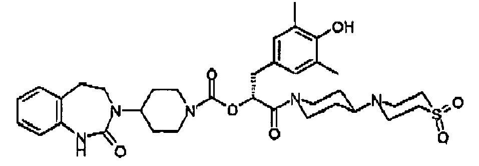 |
| (43) | 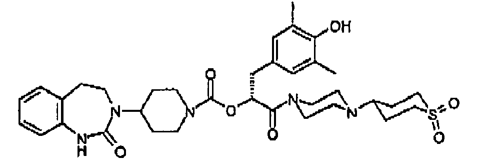 |
| (44) | 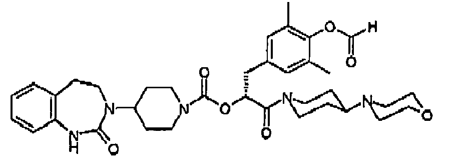 |
| (45) | 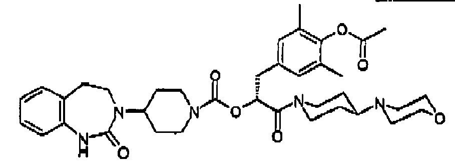 |
| (46) | 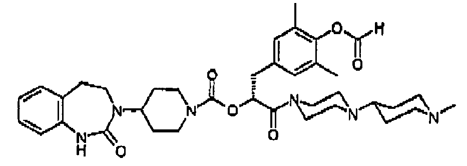 |
| (47) | 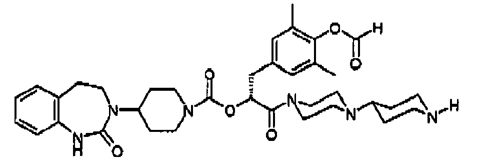 |
| (48) | 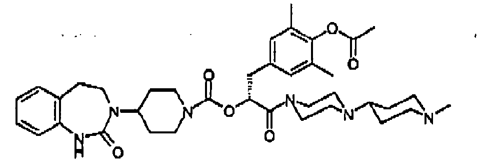 |
| (49) | 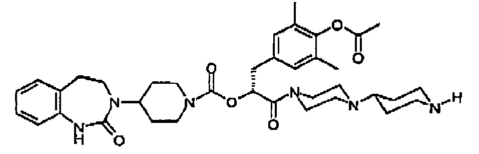 |
| (50) | 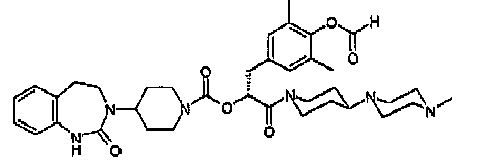 |
| (51) | 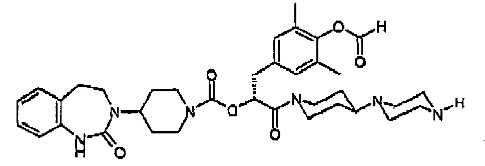 |
| (52) | 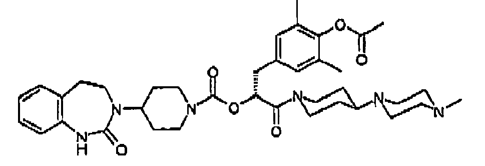 |
| (53) | 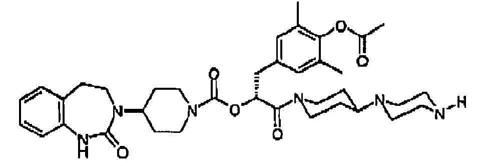 |
| (54) | 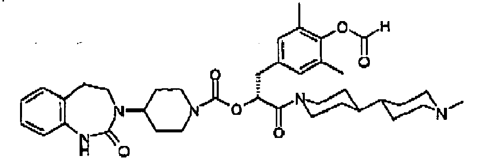 |
| (55) | 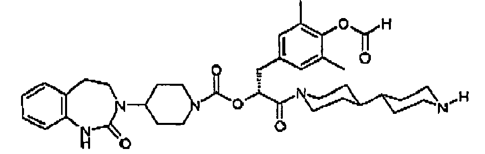 |
| (56) | 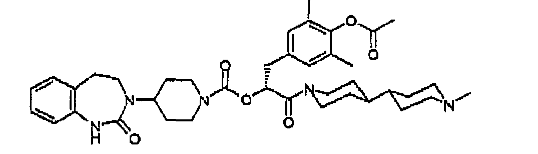 |
| (57) | 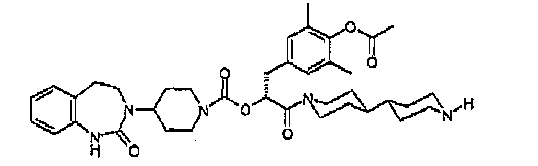 |
| (58) | 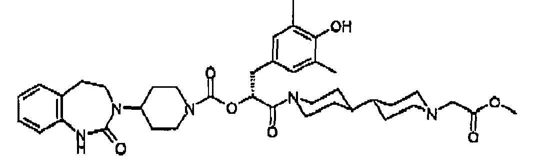 |
| (59) | 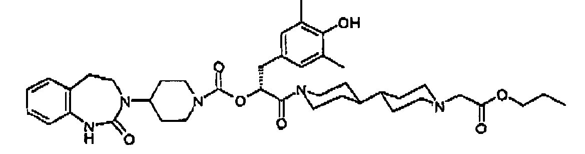 |
| (60) | 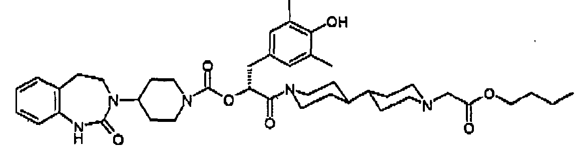 |
| (61) | 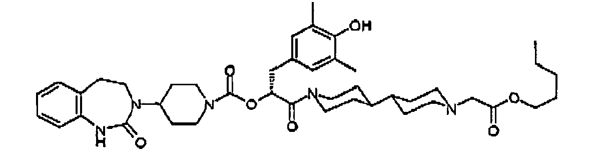 |
| (62) | 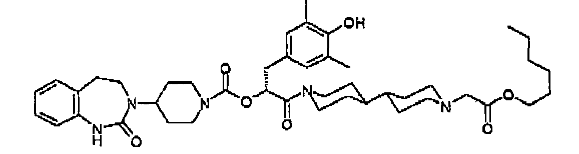 |
| (63) | 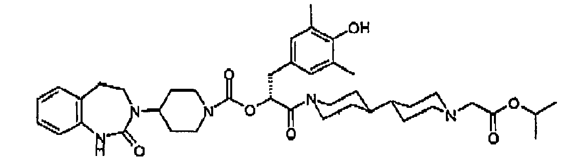 |
| (64) | 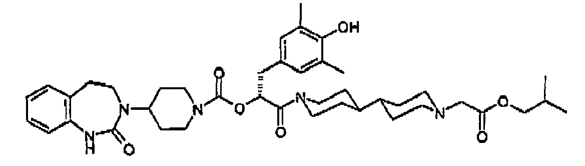 |
| (65) | 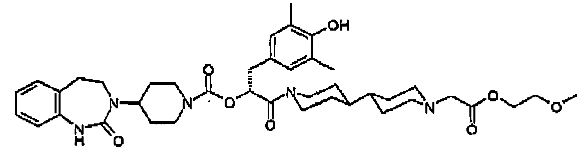 |
| (66) | 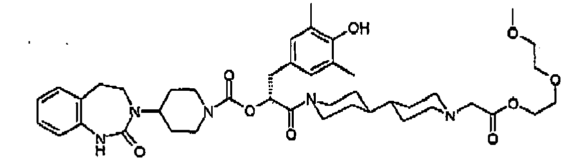 |
| (67) | 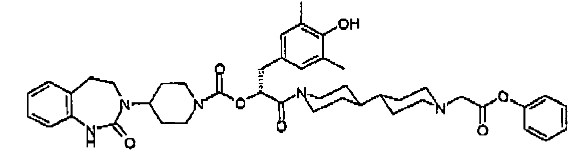 |
| (68) | 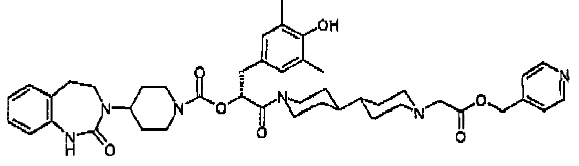 |
| (69) | 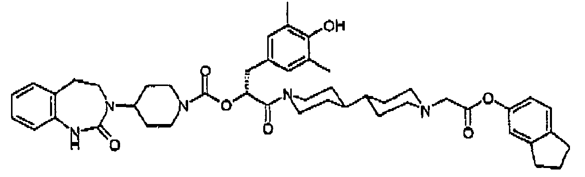 |
| (70) | 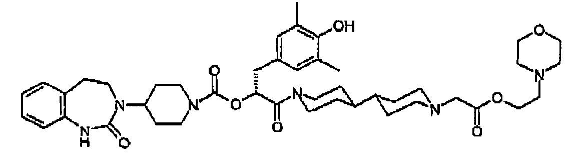 |
| (71) | 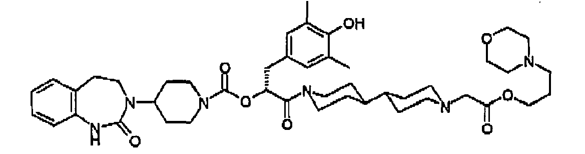 |
| (72) | 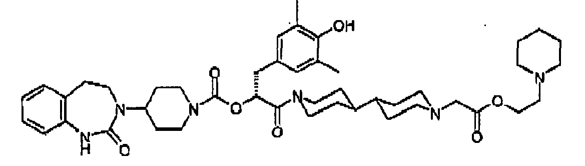 |
| (73) | 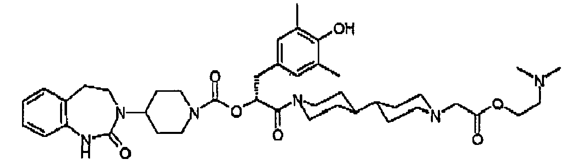 |
| (74) | 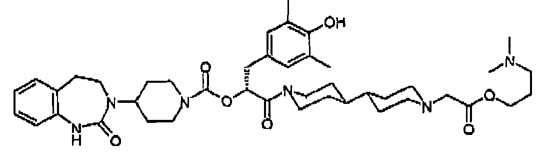 |
| (75) | 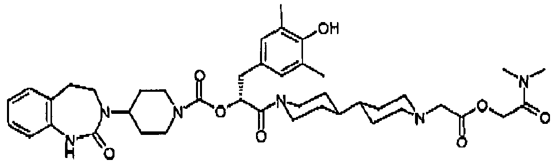 |
| (76) | 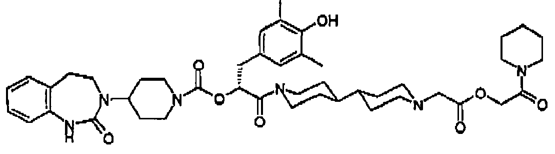 |
| (77) | 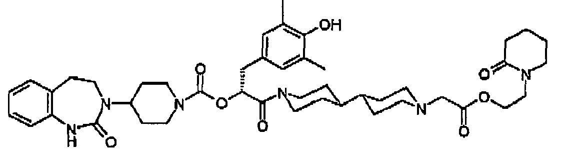 |
| (78) | 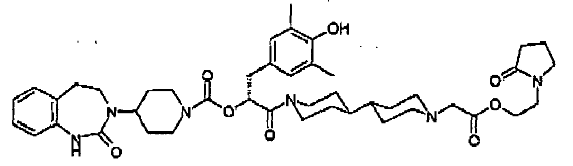 |
| (79) | 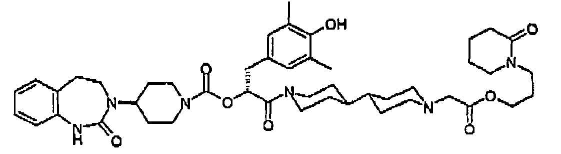 |
| (80) | 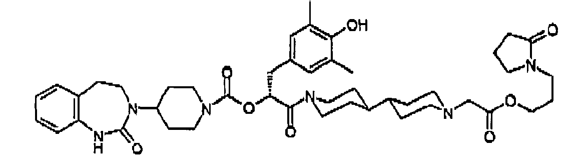 |
| (81) | 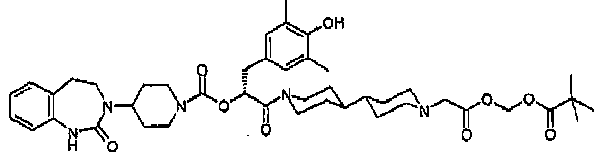 |
| (82) | 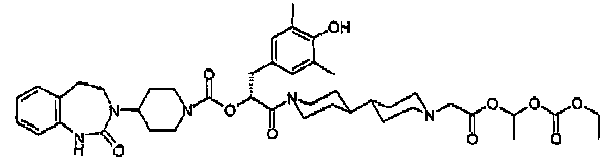 |
| (83) | 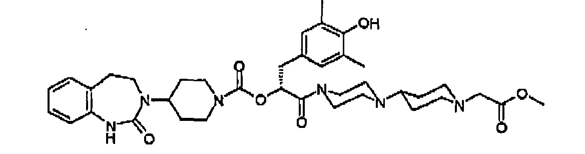 |
| (84) | 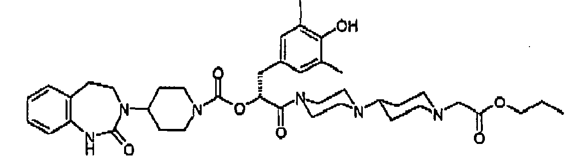 |
| (85) | 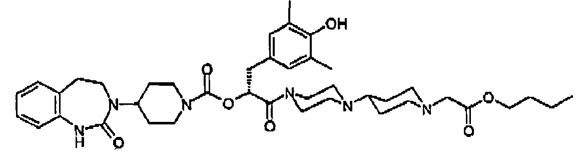 |
| (86) | 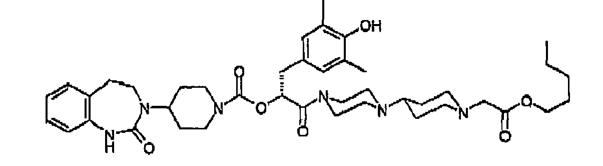 |
| (87) | 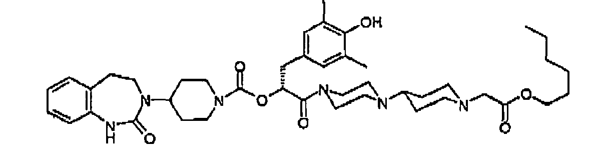 |
| (88) | 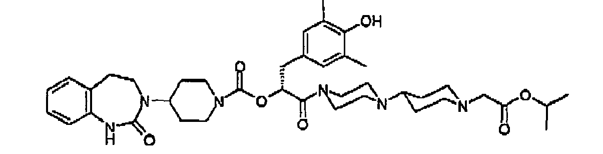 |
| (89) | 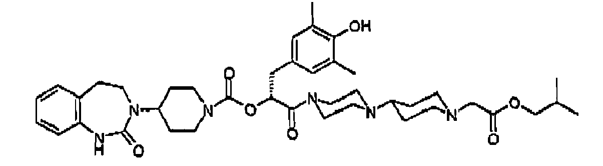 |
| (90) | 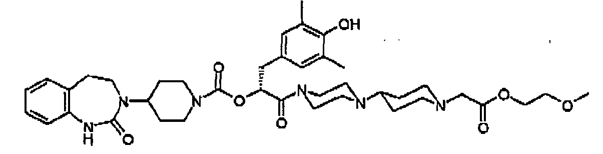 |
| (91) | 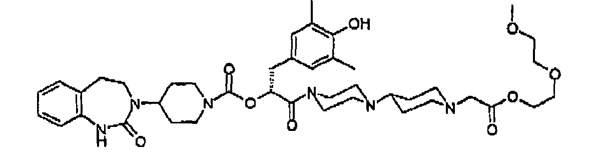 |
| (92) | 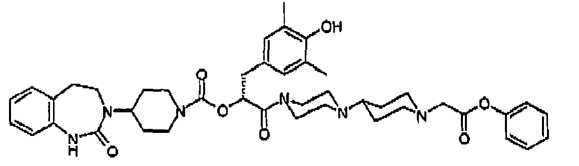 |
| (93) | 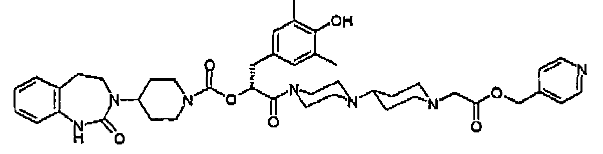 |
| (94) | 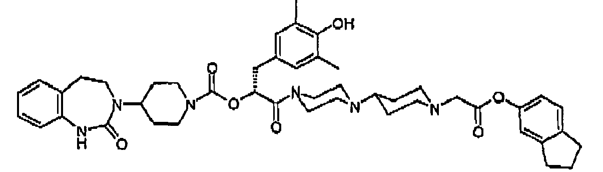 |
| (95) | 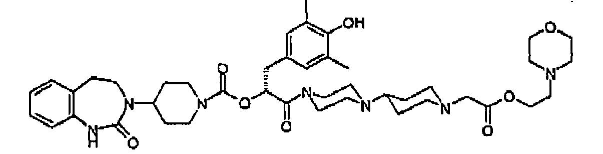 |
| (96) | 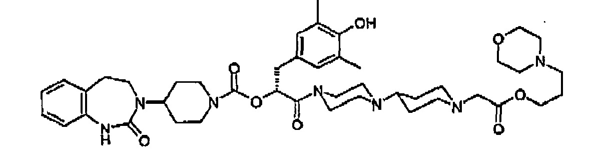 |
| (97) | 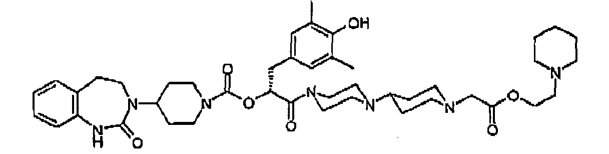 |
| (98) | 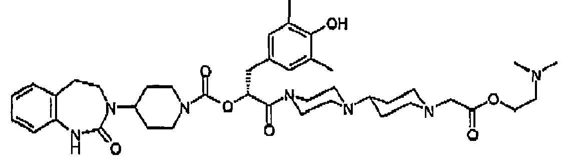 |
| (99) | 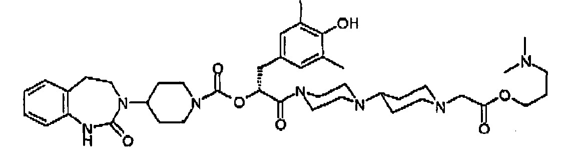 |
| (100) | 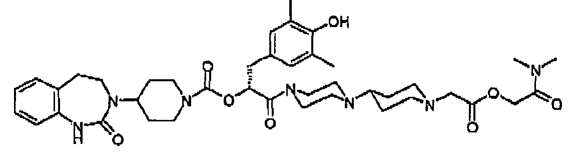 |
| (101) | 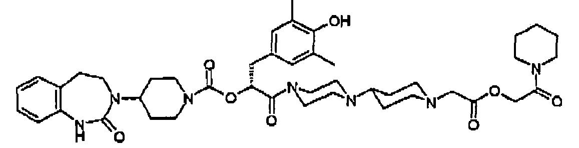 |
| (102) | 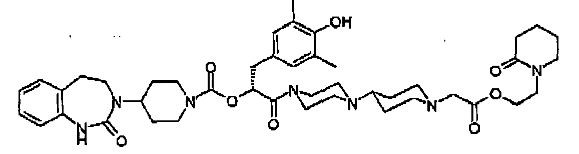 |
| (103) | 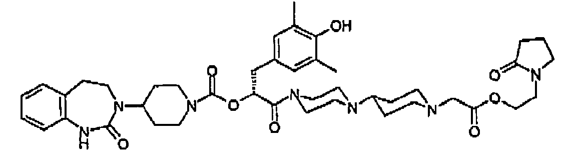 |
| (104) | 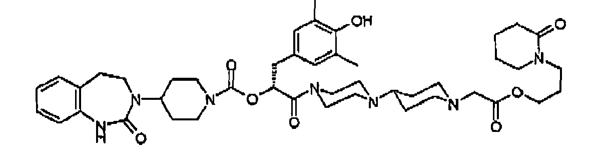 |
| (105) | 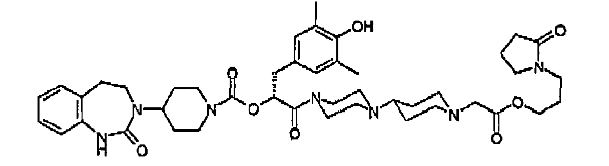 |
| (106) | 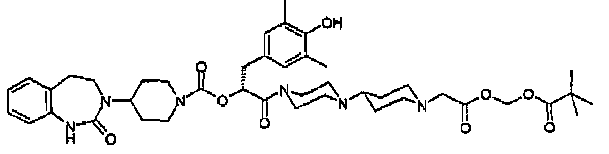 |
| (107) | 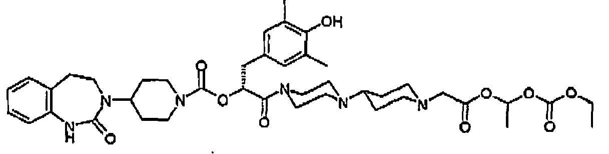 |
| (108) | 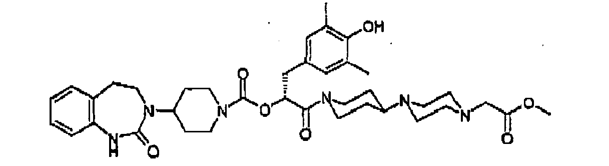 |
| (109) | 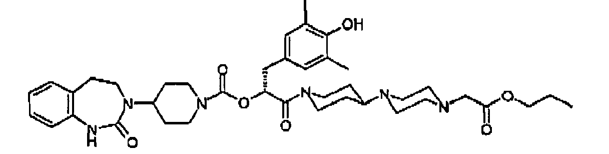 |
| (110) | 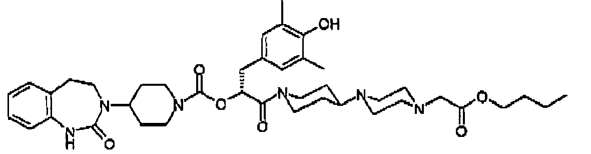 |
| (111) | 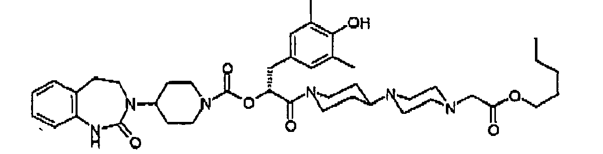 |
| (112) | 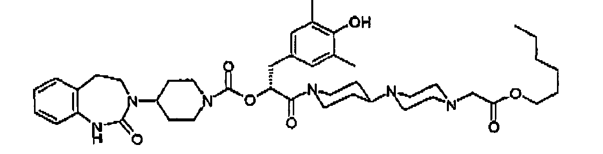 |
| (113) | 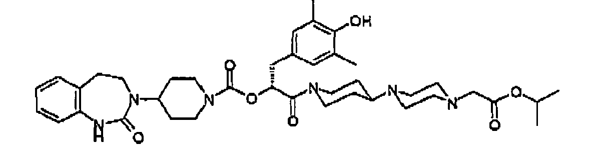 |
| (114) | 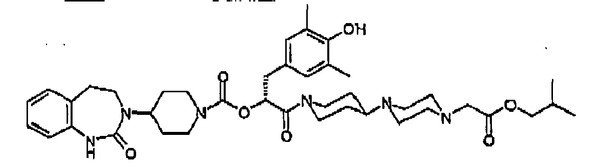 |
| (115) | 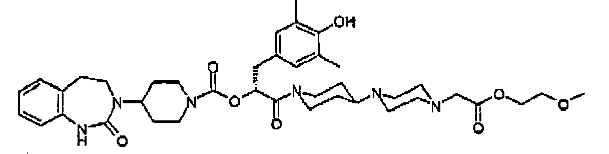 |
| (116) | 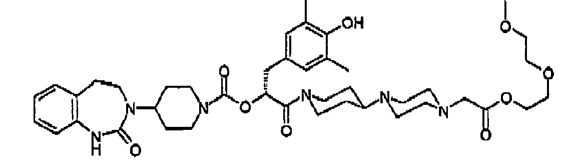 |
| (117) | 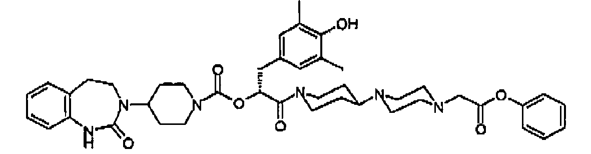 |
| (118) | 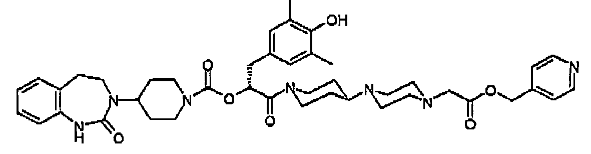 |
| (119) | 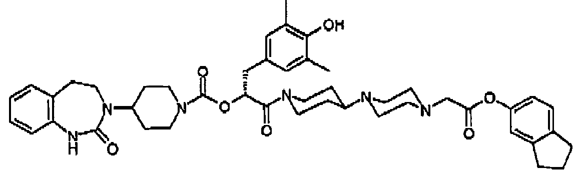 |
| (120) | 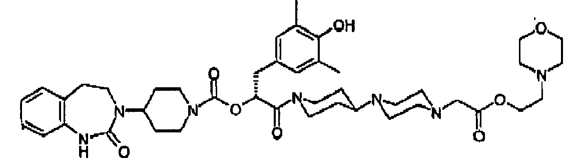 |
| (121) | 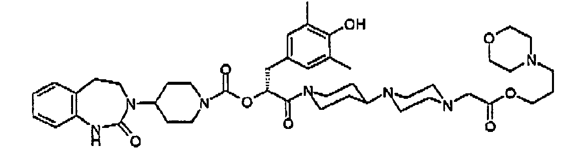 |
| (122) | 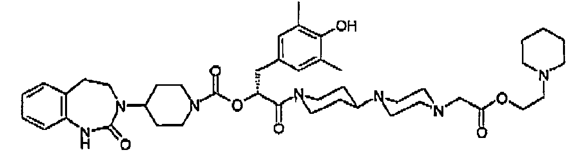 |
| (123) | 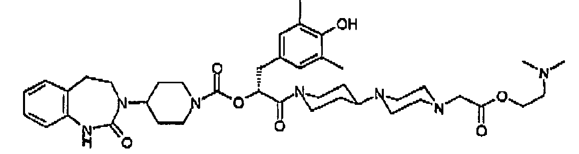 |
| (124) | 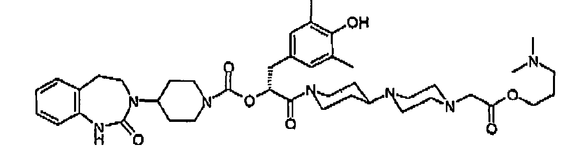 |
| (125) | 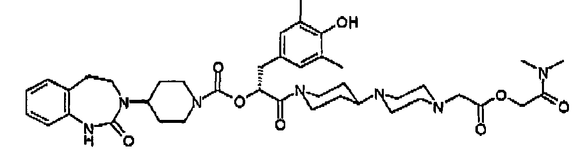 |
| (126) | 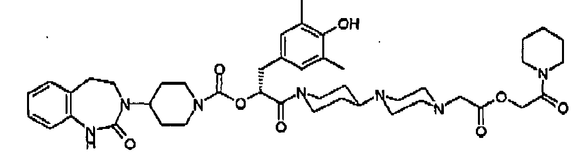 |
| (127) | 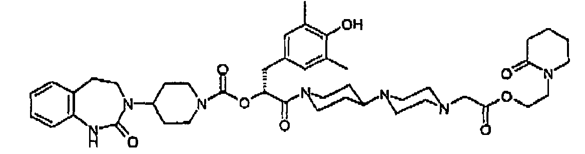 |
| (128) | 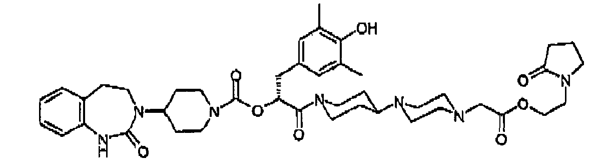 |
| (129) | 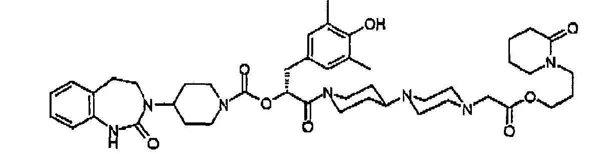 |
| (130) | 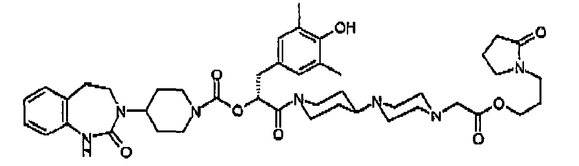 |
| (131) | 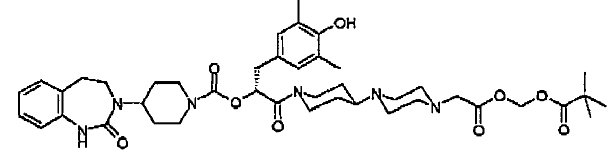 |
| (132) | 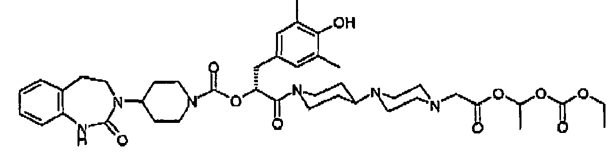 |
| (133) | 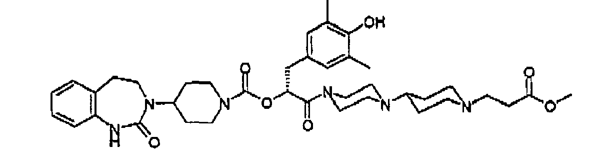 |
| (134) | 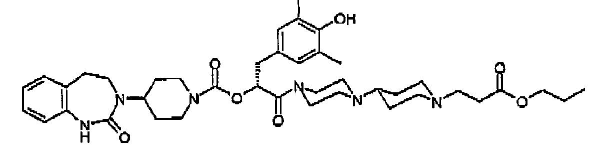 |
| (135) | 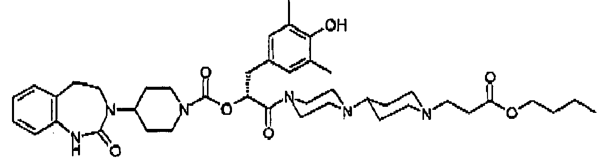 |
| (136) | 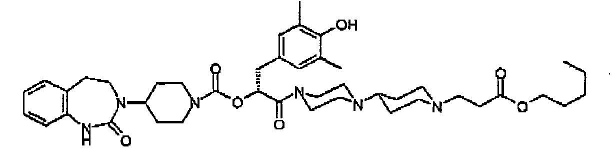 |
| (137) | 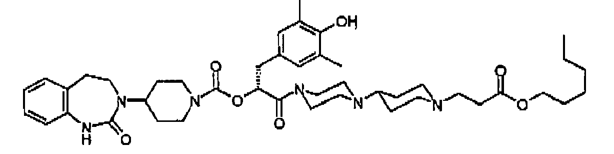 |
| (138) | 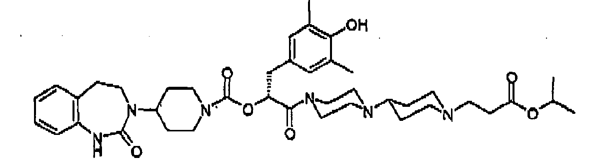 |
| (139) | 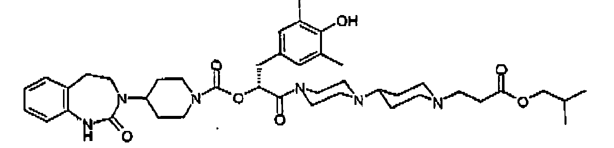 |
| (140) | 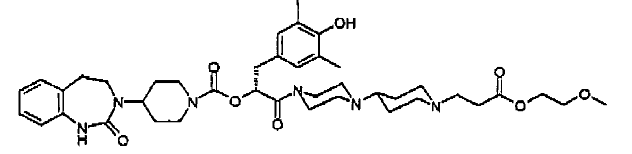 |
| (141) | 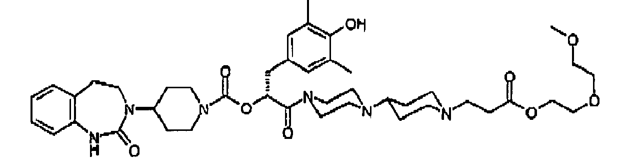 |
| (142) | 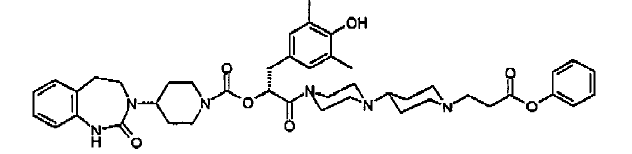 |
| (143) | 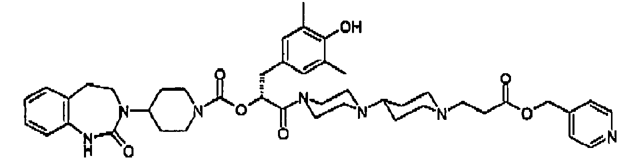 |
| (144) | 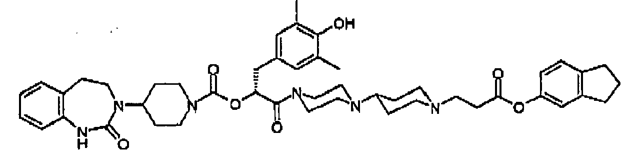 |
| (145) | 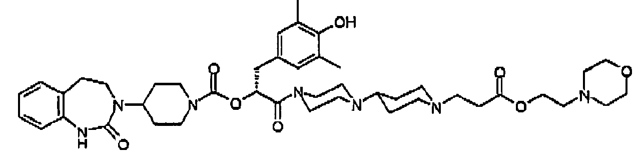 |
| (146) | 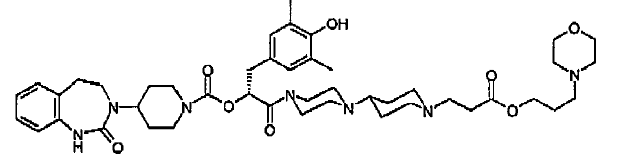 |
| (147) | 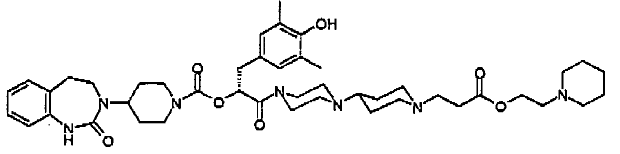 |
| (148) | 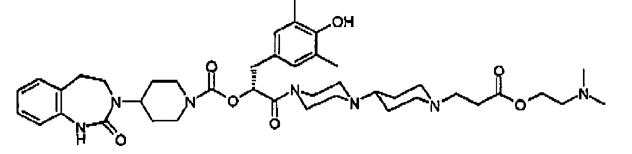 |
| (149) | 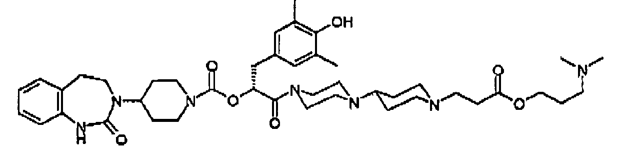 |
| (150) | 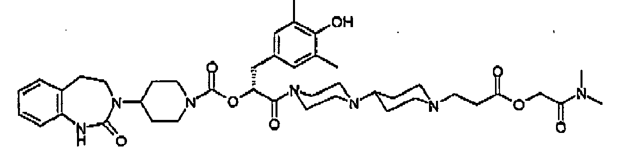 |
| (151) | 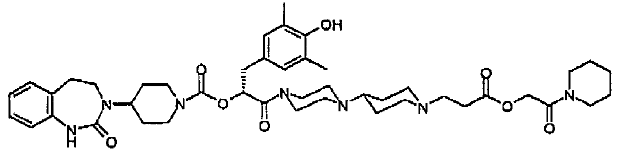 |
| (152) | 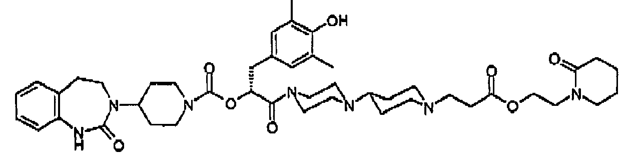 |
| (153) | 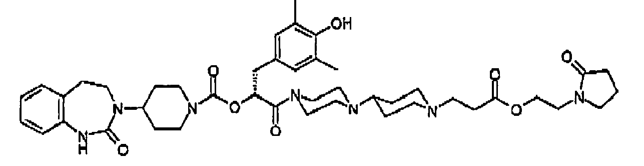 |
| (154) | 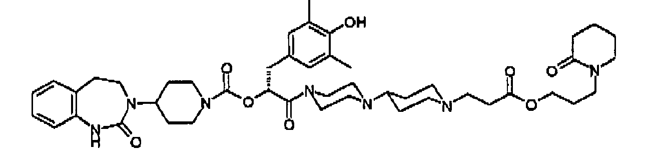 |
| (155) | 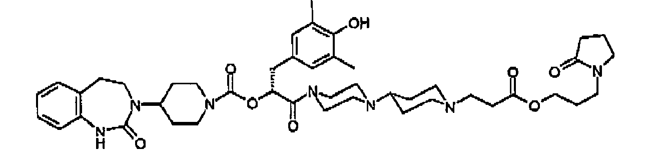 |
| (156) | 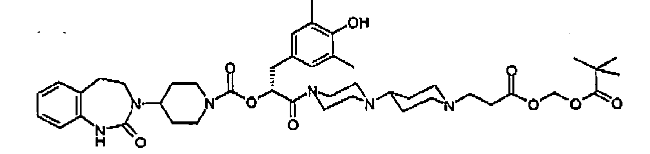 |
| (157) | 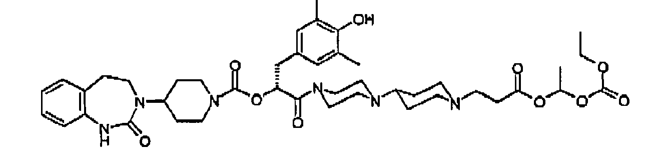 |
| (158) | 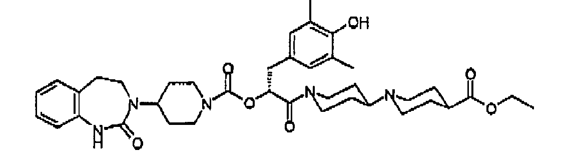 |
| (159) | 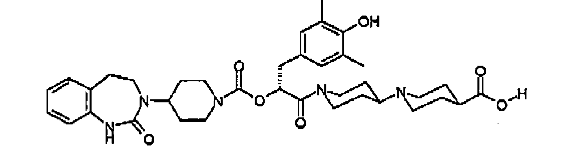 |
| (160) | 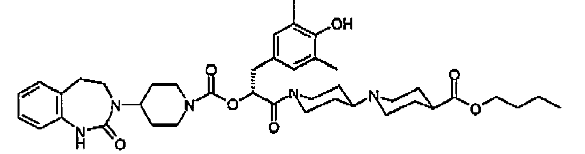 |
| (161) | 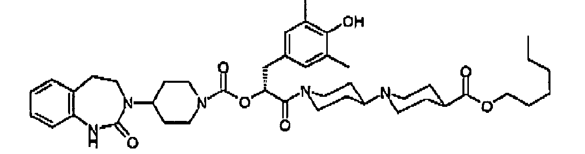 |
| (162) | 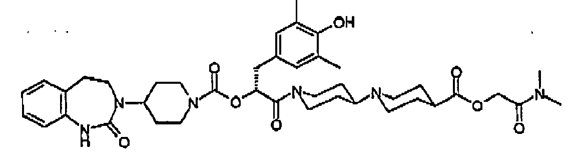 |
| (163) | 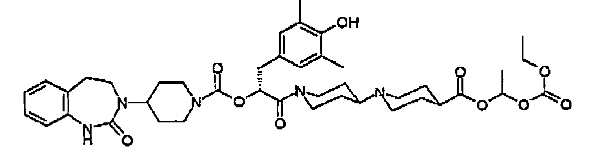 |
| (164) | 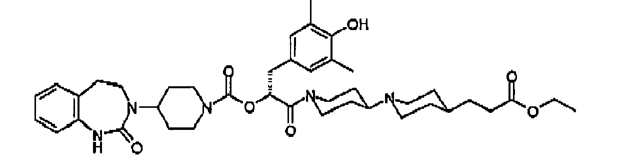 |
| (165) | 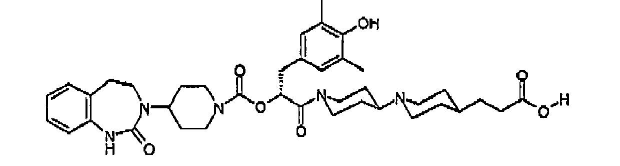 |
| (166) | 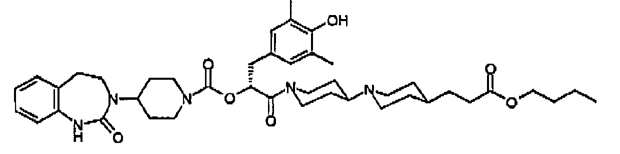 |
| (167) | 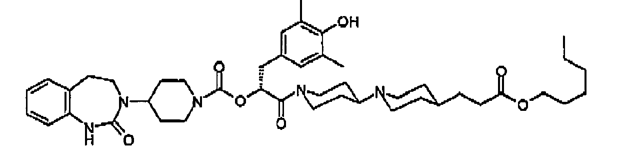 |
| (168) | 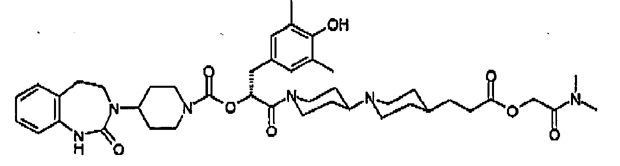 |
| (169) | 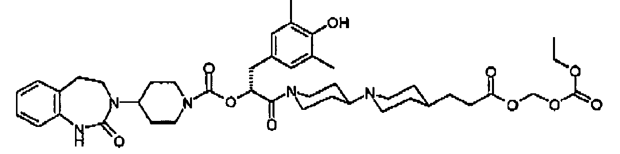 |
| (170) | 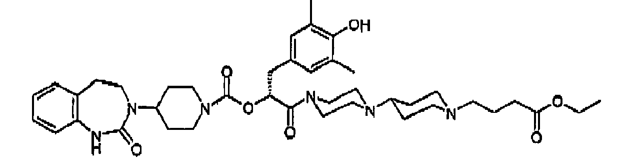 |
| (171) | 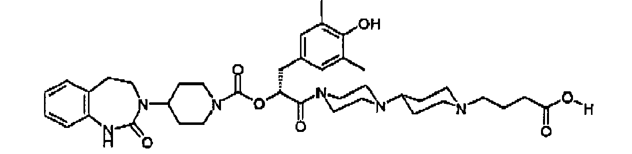 |
| (172) | 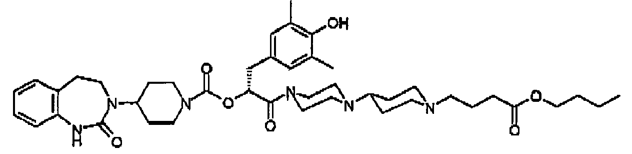 |
| (173) | 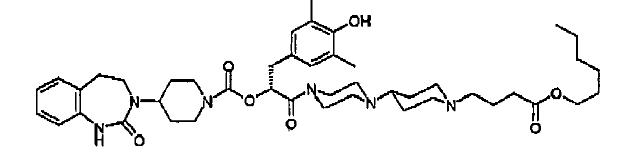 |
| (174) | 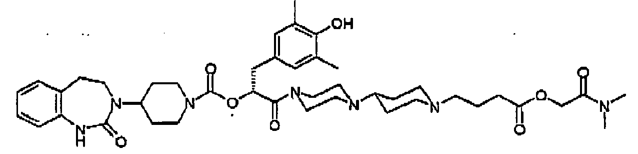 |
| (175) | 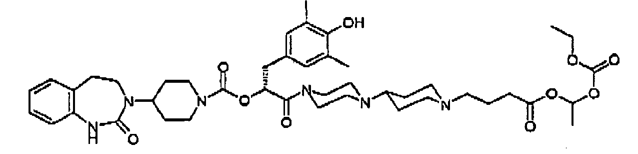 |
| (176) | 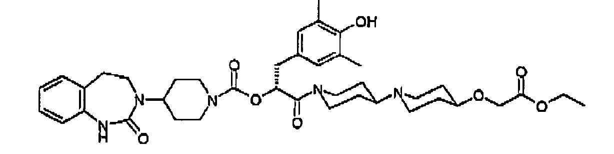 |
| (177) | 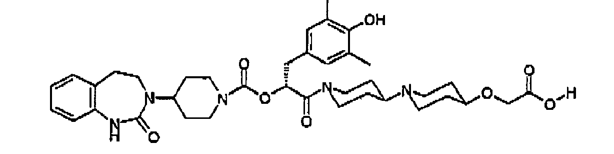 |
| (178) | 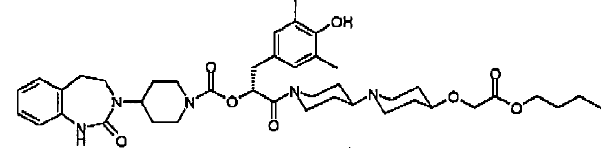 |
| (179) | 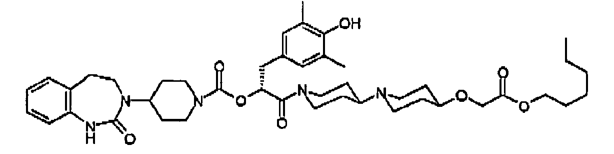 |
| (180) | 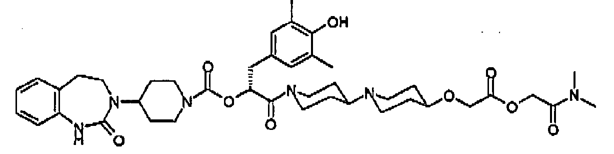 |
| (181) | 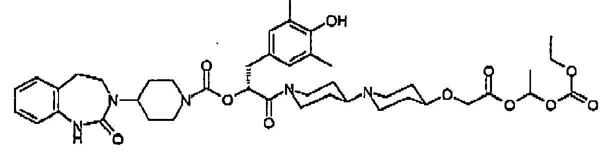 |
| (182) | 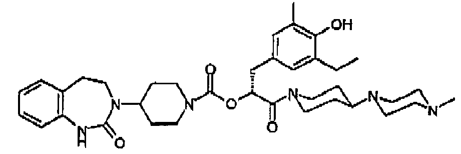 |
| (183) | 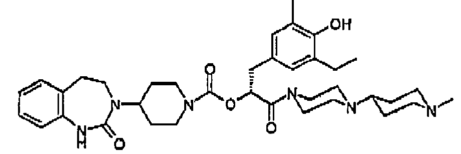 |
| (184) | 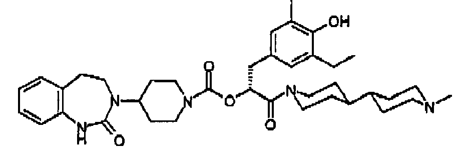 |
| (185) | 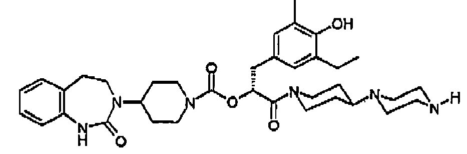 |
| (186) | 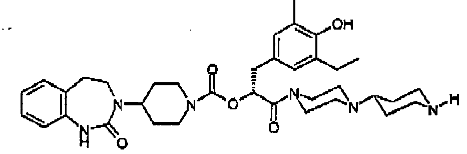 |
| (187) | 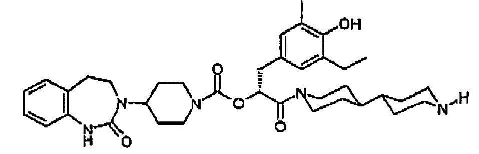 |
| (188) | 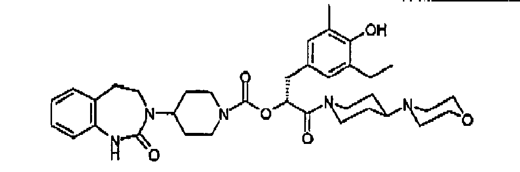 |
| (189) | 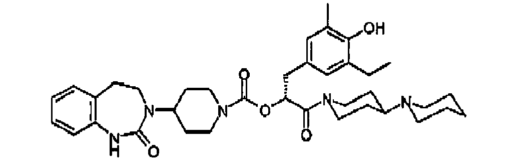 |
| (190) | 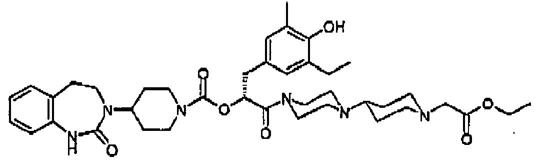 |
| (191) | 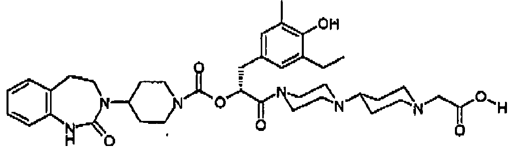 |
| (192) | 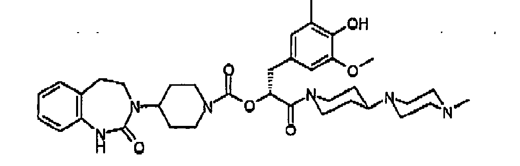 |
| (193) | 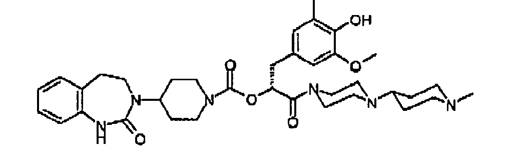 |
| (194) | 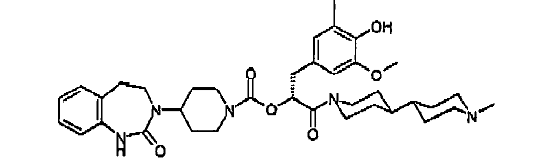 |
| (195) | 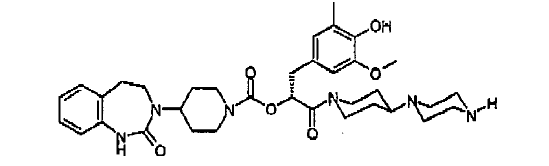 |
| (196) | 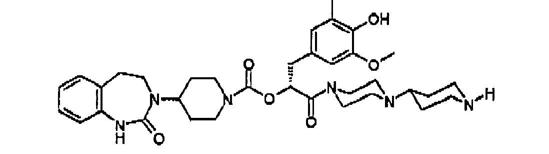 |
| (197) | 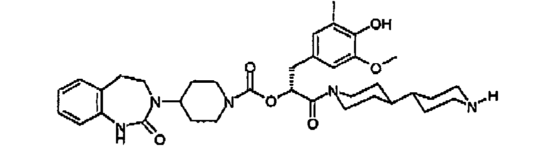 |
| (198) | 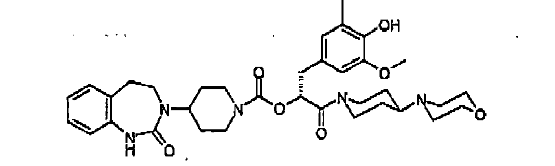 |
| (199) | 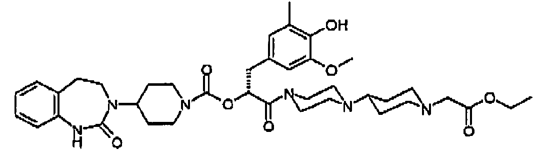 |
| (200) | 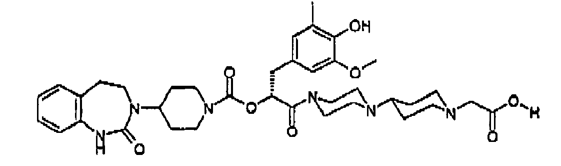 |
| (201) | 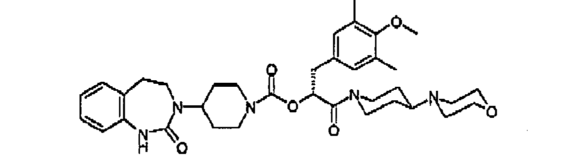 |
| (202) | 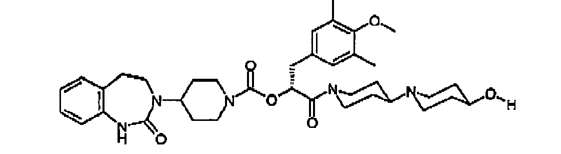 |
| (203) | 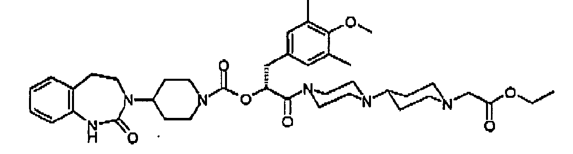 |
| (204) | 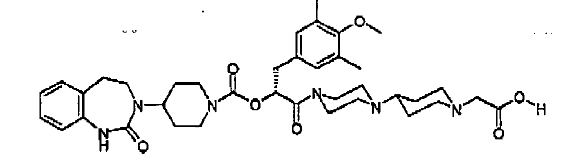 |
| (205) | 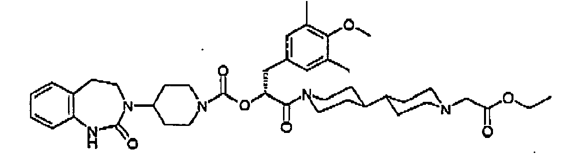 |
| (206) | 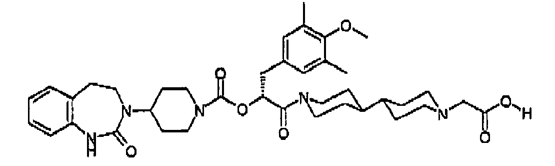 |
| (207) | 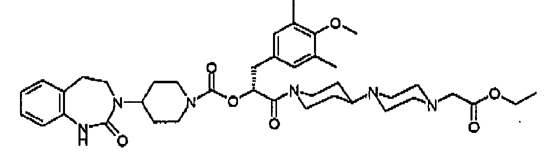 |
| (208) | 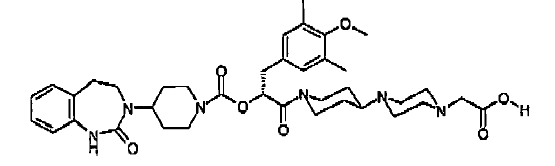 |
| (209) | 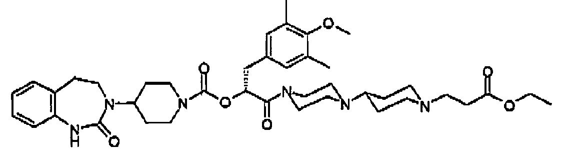 |
| (210) | 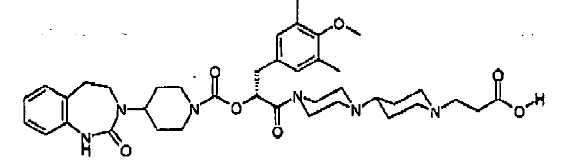 |
| (211) | 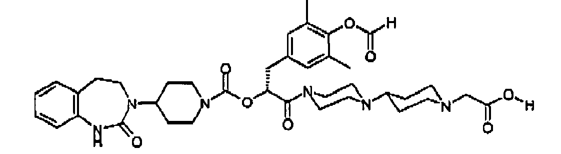 |
| (212) | 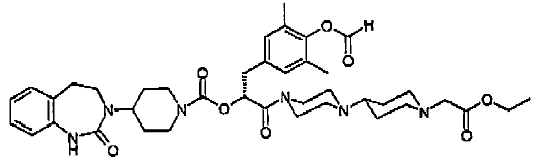 |
| (213) | 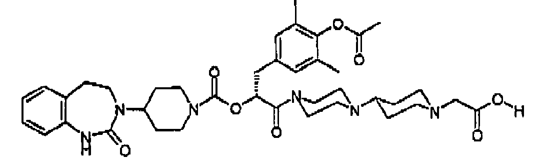 |
| (214) | 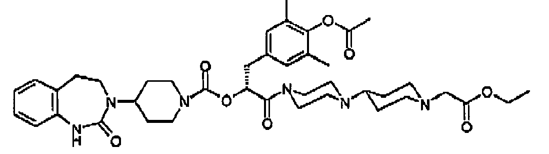 |
| (215) | 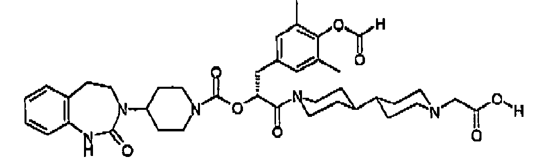 |
| (216) | 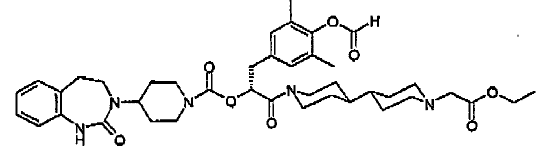 |
| (217) | 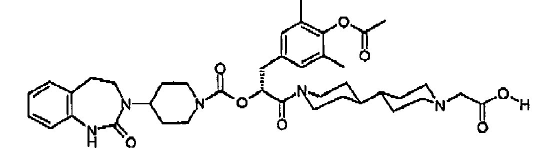 |
| (218) | 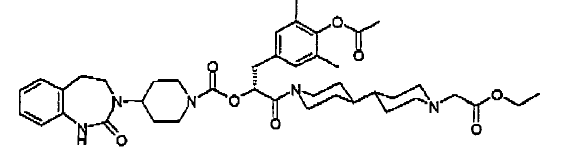 |

deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Soweit nicht anders angegeben, sind alle Substituenten voneinander unabhängig. Sollten an einer Gruppe z.B. mehrere C₁₋₆-Alkylgruppen als Substituenten sein, so könnte im Fall von drei Substituenten C₁₋₆-Alkyl unabhängig voneinander einmal Methyl, einmal *n*-Propyl und einmal *tert*-Butyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₂-Alkyl" (auch soweit sie Bestandteil anderer Reste sind) werden Alkylgruppen mit 1 oder 2 Kohlenstoffatomen verstanden, unter dem Begriff "C₁₋₃-Alkyl" verzweigte und unverzweigte Alkylgruppen mit 1 bis 3 Kohlenstoffatomen und unter dem Begriff "C₁₋₆-Alkyl" verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, *n*-Pentyl, *iso*-Pentyl, neo-Pentyl oder Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl, Butyl, Pentyl und Hexyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₃-Alkylen" (auch soweit sie Bestandteil anderer Reste sind) werden verzweigte und unverzweigte Alkylengruppen mit 1 bis 3 Kohlenstoffatomen, unter dem Begriff "C₂₋₄-Alkylen" verzweigte und unverzweigte Alkylengruppen mit 2 bis 4 Kohlenstoffatomen, unter dem Begriff "C₁₋₆-Alkylen" verzweigte und unverzweigte Alkylengruppen mit 1 bis 6 Kohlenstoffatomen, unter dem Begriff "C₂₋₆-Alkylen" verzweigte und unverzweigte Alkylengruppen mit 2 bis 6 Kohlenstoffatomen und unter dem Begriff "C₁₋₈-Alkylen" verzweigte und unverzweigte Alkylengruppen mit 1 bis 8 Kohlenstoffatomen verstanden. Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Diemthylpropylen, Hexylen, Heptylen oder Octylen, Sofern nicht anders beschrieben, umfassen die Definitionen Propylen, Butylen, Pentylen, Hexylen, Heptylen und Octylen alle denkbaren isomeren Formen der gleichen Kohlenstoffanzahl. So umfasst beispielsweise Propylen auch 1-Methylethylen und Butylen umfasst 1-Methylpropylen, 1,1-Dimathylethylen, 1,2-Dimethylethylen.

Unter dem Begriff "C₃₋₆-Cycloalkyl" (auch soweit sie Bestandteil anderer Reste sind) werden cyclische Alkylgruppen mit 3 bis 6 Kohlenstoffatomen und unter dem Begriff "C₃₋₇Cycloalkyl" cyclische Alkylgruppen mit 3 bis 7 Kohlenstoffatomen verstanden. Beispielsweise werden hierfür genannt: Cyclopropyl, Cyclobutyl, Cyclopentyl. Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Verbindungen der allgemeinen Formel I können Säuregruppen besitzen, hauptsächlich Carboxylgruppen, und/oder basische Gruppen wie z.B. Aminofunktionen. Verbindungen der allgemeinen Formel I können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansutfonsäure, Benzolsulfonsäure, *p*-Toluolsulfonsäure oder organischen Säuren wie beispielsweise Äpfelsäure, Bernsteinsäure. Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Wie vorstehend genannt, können die Verbindungen der Formel I in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel I mit anorganischen oder organischen Säuren vorliegen. Andererseits kann die Verbindung der Formel I im Falle von R gleich Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure. Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel I in der R Wasserstoff bedeutet, kommen vorzugsweise die Alkali- und Erdalkalihydroxide und -hydride in Betracht, wobei die Hydroxide und Hydride der Alkalimetalle, besonders des Natriums und Kaliums bevorzugt, Natrium- und Kaliumhydroxid besonders bevorzugt sind.

Gegebenenfalls können die Verbindungen der allgemeinen Formel I in ihre Salze, insbesondere für die pharmazeutische Anwendung, in ihre pharmakologisch unbedenklichen Säureadditionssalze mit einer anorganischen oder organischen Säure, überführt werden. Als Säuren kommen hierfür beispielsweise Bernsteinsäure, Bromwasserstoffsäure, Essigsäure, Fumarsäure, Maleinsäure, Methansulfonsäure, Milchsäure, Phosphorsäure, Salzsäure, Schwefelsäure, Weinsäure oder Zitronensäure in Betracht. Ferner können Mischungen der vorgenannten Säuren eingesetzt werden.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren -wie beispielsweise Säureadditionssalze mit Halogenwassersfioffsäuren - beispielsweise Chlor- oder Bromwasserstoffsäure - oder organische Säuren - wie beispielsweise Oxal-, Fumar-, Diglykol- oder Methansulfonsäure

Die erfindungsgemäßen Verbindungen können als Racemate vortiegen, sofern sie nur ein Chiralitätselement besitzen, sie können aber auch als reine Enantiomere, d.h. in (R)- oder (*S*)-Form gewonnen werden. Bevorzugt sind Verbindungen die als Racemate bzw. als (*R*)-Form vorliegen.

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren - wie beispielsweise Säureadditionssalze mit Halogenwasserstoffsäuren - beispielsweise Chlor- oder Bromwasserstoffsäure - oder organische Säuren - wie beispielsweise Oxal-, Fumar-, Diglycol- oder Methansulfonsäure.

### HERSTELLVERFAHREN

Die Verbindungen der allgemeinen Formel I werden nach prinzipiell bekannten Methoden hergestellt. Die folgenden Verfahren haben sich zur Herstellung der erfindungsgemäßen Verbindungen der allgemeinen Formel I besonders bewährt:

(a) Zur Herstellung von Verbindungen der allgemeinen Formel I, in der alle Reste wie eingangs erwähnt definiert sind:
Kupplung einer Carbonsäure der allgemeinen Formel V 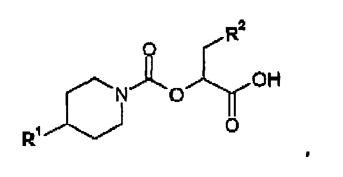 in der **R¹** und **R²** wie eingangs erwähnt definiert sind, mit einem Amin der allgemeinen Formel **VI**

   **H-R³-R⁴**

   in der **R³** und **R⁴** wie eingangs definiert sind, wobei die Verknüpfung über das Stickstoffatom von **R³** erfolgt.
Vor Durchführung der Reaktion können in den Resten des Amins der Formel **H-R³-R⁴** gegebenenfalls vorhandene Carbonsäurefiunktionen, primäre oder sekundäre Aminofunktionen oder Hydroxyfunktionen durch übliche Schutzreste geschützt und gegebenenfalls verwendete Schutzreste nach Durchführung der Reaktion nach für den Fachmann geläufigen Methoden wieder abgespalten werden.
Die Kupplung wird bevorzugt unter Verwendung von aus der Peptidchemie bekannten Verfahren (siehe z. B. Houben-Weyl, Methoden der Organischen Chemie, Bd. 15/2) durchgeführt, wobei zum Beispiel Carbodiimide, wie z. B, Dicyclohexylcarbodiimid (DCC), Diisopropytcarbodiimid (DIC) oder Ethyl-(3-dimathylarnino-propyl)-carbodiimid, O-(1*H*-Benzotriazol-1-yl)-*N*,*N*-*N*',*N*'-tetramethyluronium-hexa-fluorphosphat (HBTU) oder -tetrafluorborat (TBTU) oder 1*H*-Benzotriazol-1-yl-oxy-tris-(dimethytarnino)-phosphonium-hexafluorphosphat (BOP) eingesetzt werden. Durch Zugabe von 1-Hydroxybenzotriazol (HOBt) oder von 3-Hydroxy-4-oxo-3,4-dihydro-1,2,3-benzotriazin (HOOBt) kann die Reaktionsgeschwindigkeit gesteigert werden. Die Kupplungen werden normalerweise mit äquimolaren Anteilen der Kupplungskomponenten sowie des Kupplungsreagenz in Lösungsmitteln wie Dichlormethan, Tetrahydrofuran, Acetonitril, Dimethylformamid (DMF), Dimettlylacetamid (DMA), N-Methylpyrralidon (NMP) oder Gemischen aus diesen und bei Temperaturen zwischen -30°C und +30°C, bevorzugt -20°C und +25°C, durchgeführt. Sofern erforderlich wird als zusätzliche Hilfsbase N-Fthyldüsopropylamin (Honig-Base) bevorzugt.

Als weiteres Kupplungsverfahren zur Synthese von Verbindungen der allgemeinen Formel I wird das sogenannte "Anhydridverfahren" (siehe auch: M. Bodanszky, "Peptide Chemistry", Springer-Verlag 1988, S. 58-59; M. Bodanszky, "Principles of Peptide Synthesis", Springer-Verlag 1984, S. 21-27) eingesetzt. Bevorzugt wird das "gemischte Anhydridverfahren" in der Variante nach Vaughan (J.R. Vaughan Jr., J. Amer. Chem.Soc. 73, 3547 (1951)), bei der unter Verwendung von Chlorkohlensäureisobutylester in Gegenwart von Basen, wie 4-Methylmorpholin oder 4-Ethylmorpholin, das gemischte Anhydrid aus der zu kuppelnden Carbonsäure der allgemeinen Formel V und dem Kohlensäuremonoisobutylester erhalten wird. Die Herstellung dieses gemischten Anhydrids und die Kupplung mit den Aminen der allgemeinen Formel VI erfolgt im Eintopfverfahren unter Verwendung der voranstehend genannten Lösungsmittel und bei Temperaturen zwischen -20°C und +25°C, bevorzugt zwischen 0°C und +25°C.

(b) Zur Herstellung von Verbindungen der allgemeinen Formel I, in der alle Reste wie eingangs erwähnt definiert sind:
Kupplung einer Verbindung der allgemeinen Formel VII 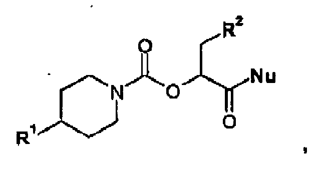 in der **R¹** und **R²** wie eingangs erwähnt definiert sind und **Nu** eine Austrittsgruppe, beispielsweise ein Halogenatom, wie das Chlor-, Brom- oder lodatom, eine Alkylsulfonyloxygruppe mit 1 bis 10 Kohlenstoffatomen im Alkylteil, eine gegebenenfalls durch Chlor- oder Bromatome, durch Methyl- oder Nitrogruppen mono-, di- oder trisubstituierte Phenylsulfonyloxy oder Naphthylsulfonyloxygruppe, wobei die Substituenten gleich oder verschieden sein können, eine 1*H*-[midazol-1-yl-, eine gegebenenfalls durch eine oder zwei Methylgruppen im Kohlenstoffgerüst substituierte 1*H*-Pyrazol-1-yl-, eine 1*H*-1,2,4-Triazol-1-yl-, 1*H*-1,2,3-Triazol-1-yl-, 1*H*-1,2,3,4-Tetrazol-1-yl-, eine Vinyl-, Propargyl-, *p*-Nitrophenyl-, 2,4-Dinitrophenyl-, Trichlorphenyl-, Pentachlorphenyl-, Pentafluorphenyl-, Pyranyl- oder Pyridinyl-, eine Dimethylaminyloxy-, 2(1*H*)-Oxopyridin-1-yl-oxy-, 2,5-Dioxo-pyrrotidin-1-yloxy-, Phthalimidyloxy-, 1*H*-Benzotnazol-1-yloxy- oder Azidgruppe darstellt, mit einem Amin der allgemeinen Formel VI

   H-R³-R⁴,
in der alle Reste wie eingangs erwähnt definiert sind und wobei die Verknüpfung über das Stickstoffatom des Amins R³ erfolgt.

Vor Durchführung der Reaktion können in den Resten des Amins der allgemeinen Formel **VI** gegebenenfalls vorhandene Carbonsäurefunktionen, primäre oder sekundäre Aminofunktionen oder Hydroxyfunktionen durch übliche Schutzreste geschützt und gegebenenfalls verwendete Schutzreste nach Durchführung der Reaktion nach für den Fachmann geläufigen Methoden wieder abgespalten werden.

Die Umsetzung wird unter Schotten-Baumann- oder Einhom-Bedingungen durchgeführt, das heißt, die Komponenten werden in Gegenwart von wenigstens einem Äquivalent einer Hilfsbase bei Temperaturen zwischen -50°C und +120°C. bevorzugt-10°C und +30°C, und gegebenenfalls in Gegenwart von Lösungsmitteln zur Reaktion gebracht. Als Hilfsbasen kommen bevorzugt Alkali- und Erdalkalihydroxide, beispielsweise Natriumhydroxid, Kaliumhydroxid oder Bariumhydroxid, Alkalicarbonate, z. B. Natriumcarbonat, Kaliumcarbonat oder Cäsiumcarbonat, Alkaliacetate, z.B. Natrium- oder Kaliumacetat, sowie tertiäre Amine, beispielsweise Pyridin, 2,4,6-Trimethylpyridin, Chinolin, Triethylamin, *N*-Ethyldiisopropylamin, *N*-Ethyldicyclohexylamin, 1,4-Di-azabicyclo[2,2.2]octan oder 1,8-Diaza-bicyclo[5,4,0]undec-7-en, als Lösungsmittel beispielsweise Dichlormethan, Tetrahydrofuran, 1.4-Dioxan, Acetonitril, Dimethylformamid, Dimethylacetamld, N-Methylpyrrolidon oder Gemische davon in Betracht; werden als Hilfsbasen Alkali- oder Erdalkalihydroxide, Alkalicarbonate oder-acetate verwendet, kann dem Reaktionsgemisch auch Wasser als Cosolvens zugesetzt werden.

Die erfindungsgemäßen neuen Verbindungen der allgemeinen Formel I enthalten ein oder mehrere Chiralitätszentren. Sind beispielsweise zwei Chiralitätszentren vorhanden, dann können die Verbindungen in Form zweier diastereomerer Antipodenpaare auftreten. Die Erfindung umfasst die einzelnen Isomeren ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulanchromatographie unter Verwendung chiraler oder bevorzugt achiraler stationärer Phasen.

Die Trennung von unter die allgemeine Formel fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (*R*)-(+)-1-Phenylethylamin, (*S*)-(-)-1-Phenylethylamin oder (S)-Brucin, entstehen. '

Nach einem üblichen Verfahren zur Isomerentrennung wird das Racemat einer Verbindung der allgemeinen Formel I mit einer der vorstehend angegebenen optisch aktiven Säuren bzw. Basen in äquimolarer Menge in einem Lösemittel umgesetzt und die erhaltanen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (*R*)- oder (*S*)-Enantiomer bzw. ein Gemisch zweier optisch aktiver, unter die allgemeine Formel I fallender, diastereomerer Verbindungen wird auch dadurch erhalten, dass man die oben beschriebenen Synthesen mit jeweils einer geeigneten (R)- bzw. (S)-konfigurierten Reaktionskomponente durchführt.

Die als Ausgangsverbindungen benötigten Carbonsäuren der allgemeinen Formel **V** sind durch Umsetzung von Piperidinen der allgemeinen Formel **VIII** 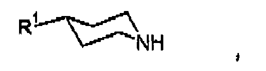 in der **R¹** wie eingangs erwähnt definiert ist, mit Kohlensäurederivaten der allgemeinen Formel **IX** 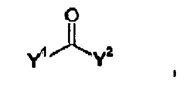 in der **Y¹** rund **Y²** nucleofuge Gruppen bedeuten, die gleich oder verschieden sein können, bevorzugt das Chloratom, die p-Nitrophenoxy- oder Trichlormethoxy-Gruppe, und mit Verbindungen der allgemeinen Formel **X** 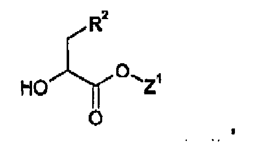 in der **R²** wie eingangs erwähnt definiert ist und **Z¹** eine Schutzgruppe für eine Carboxygruppe darstellt, beispielsweise eine C₁₋₆-Alkyl- oder eine gegebenenfalls substituierte Benzylgruppe, wobei die Alkylgruppen linear oder verzweigt sein können und die Benzylgruppe durch ein oder zwei Methoxygruppen substituiert sein kann, erhältlich.
Bevorzugt ist für **Z¹** die Methyl-, Ethyl-, *tert*-Butyl oder Benzylgruppe, Vor Durchführung der Reaktion kann im Rest **R²** einer Verbindung der Formel **VI** gegebenenfalls vorhandene Hydroxyfunktionen durch übliche Schutzreste geschützt und gegebenenfalls verwendete Schutzreste nach Durchführung der Reaktion nach dem Fachmann geläufigen Methoden wieder abgespalten werden.

In einer ersten Stufe werden die Verbindungen der allgemeinen Formel VIII in einem Lösungsmittel, beispielsweise in Dichlormethan, THF, Pyridin oder deren Mischungen, bei einer Temperatur zwischen -20°C bis 50°C in Gegenwart einer Base, beispielsweise Triethylamin, Pyridin oder Ethyldiisopropylamin, mit den Kohlensäurederivaten der allgemeinen Formel IX zur Reaktion gebracht. Die dabei entstehende Zwischenstufe kann aufgereinigt oder ohne Reinigung weiter umgesetzt werden.
Die Umsetzung dieser Zwischenstufen mit Verbindungen der allgemeinen Formel **X** erfolgt ebenfalls in einem der oben genannten Lösungsmittel, und bei den oben genannten Temperaturen, in Gegenwart einer Base, wie Triethylamin oder Pyridin, mit oder ohne Zusatz eines Aktivierungsreagenz, wie z,B, 4-Dimethylaminopyridin. Zur Aktivierung können die Verbindungen der allgemeinen Formel **X** auch mittels eines Metallhydrides, wie z.B. NaH oder KH, deprotoniert werden, wobei in diesem Fall auf die Gegenwart der Base oder des Aktivierungsreagenzes verzichtet werden kann.

Die Ausgangsverbindungen der Formel **VIII** und **IX** sjnd entweder käuflich, literaturbekannt oder können nach literatürbekannten Methoden hergestellt werden.

Ein Zugang zu Verbindungen der allgemeinen Formel **X** besteht in der Umsetzung von Aldehyden der allgemeinen Formel **XI** 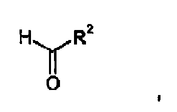 in der **R²** wie eingangs erwähnt definiert ist, mit *N*-Acetylglycin in Acetanhydrid als Lösungsmittel in Gegenwart von Alkaliacetat, bevorzugt Natrium- oder Kaliumacetat, bei geeigneter Temperatur, bevorzugt bei 80 bis 130°C.
Die primär entstehenden Azlactone werden ohne Isolierung zu den Verbindungen der allgemeinen Formel **XII** 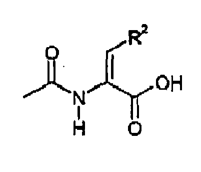 in der **R²** wie eingangs erwähnt definiert ist, hydrolysiert,
Alternativ können Enamide der allgemeinen Struktur **XIII** 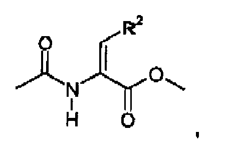 in der **R²** wie eingangs definiert ist, durch eine Kupplungsreaktion von Verbindungen der allgemeinen Formel **XIV**

**Hal-R²,**

in der **R²** wie eingangs erwähnt definiert ist und Hal das Brom- oder lodatom bedeutet und 2-Acetylaminoacrylsäuremethylester erhalten werden.
Die Umsetzung erfolgt in einem geeignetem Lösungsmittel, wie Tetrahydrofuran, Dimethylformamid, 1,4-Dioxan oder Acetonitril, bevorzugt Acetonitril, bei Temperaturen zwischen Raumtemperatur und 120°C, bevorzugt zwischen 50°C und 80°C, in Gegenwart einer geeigneten Hilfsbase, wie Triethylamin oder Ethyldüsopmpylamin, bevorzugt Triethylamin, und eines geeigneten Katalysatorsystems. Geeignete ffatalysatorsysteme stellen die Kombination einer Palladium-Spezies, wie Palladium(II)-acetat oder Bis(acetonitril)-Palladium-dichlorid, bevorzugt Palladium(II)-acetat, und eines geeigneten Phosphan-Liganden, wie Triphenyl- oder Tris-o-tolyl-phosphan, bevorzugt Tris-o-tolylphosphan dar.
Durch weitere Umsetzung von Verbindungen der allgemeinen Formeln **XII** und **XIII** in Gegenwart von wässrigen Mineralsäuren, wie beispielsweise Schwefel-, Phosphor- oder Chlorwasserstoffsäure, bevorzugt jedoch von Chlorwasserstoffsäure, werden Verbindungen der allgemeinen Formel **XV** 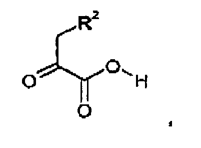 in der **R²** wie eingangs erwähnt definiert ist, erhalten,
Diese werden dann mit geeigneten Reduktionsmitteln in die Verbindungen der allgemeinen Formel **XVI** 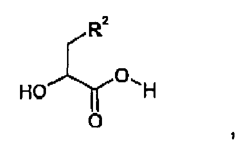 in der **R²** wie eingangs erwähnt definiert ist, überführt.
Als Reduktionsmittel können Alkaliborhydride, wie Natrium- oder Kaliumborhydrid verwendet werden. Weitere Reduktionsmittel stellen Chlordialkylborane, wie Chlordicyclohexylboran, dar. Werden chirale Chlordialkylborane, wie z.B. B-Chlordüsopinocampheylboran benutzt, können die Verbindungen der allgemeinen Formel **XIV** in enantiomerenreiner Form isoliert werden. Die weitere Umsetzung von Verbindungen der allgemeinen Formel **XIV** zu Verbindungen der allgemeinen Formel **X** erfolgt im alkoholischen Milieu, bevorzugt in Methanol oder Ethanol, in Gegenwart einer geeigneten Säure, wie Chlorwasserstoffsäure. Die Reaktion kann alternativ durch Umsetzung in alkoholischen Lösungsmitteln, bevorzugt Methanol, mit Thionychlorid erfolgen.

Alle Verbindungen der allgemeinen Formel I, die primäre oder sekundäre Amino-, Hydroxy- oder Hydroxycarbonylfunktionen enthalten, werden bevorzugt aus mit Schutzgruppen versehenen Vorstufen gewonnen. Als Schutzgruppen für Aminofunktionen kommen beispielsweise eine Benzyloxycarbonyl-, 2-Nitrobenzyloxycarbonyl-, 4-Nitrobenzyloxycarbonyl-, 4-Methoxy-benzyloxycarbonyl-, 2-Chlor-benzyloxycarbonyl-, 3-Chlor-benzyloxycarbonyl-, 4-Chlor-benzyloxycarbonyl-, 4-Biphenylyl-α,α-dimethyl-benzyloxycarbonyl- oder 3,5-Dimethoxy-α,α-dimethyl-benzyloxycarbonylgruppe, eine Alkoxycarbonylgruppe mit insgesamt 1 bis 5 Kohlenstoffatomen im Alkylteil, beispielsweise die Methoxycarbonyl-, Ethoxycarbonyl-, *n*-Propoxycarbonyl-, Isopropoxycarbonyl-, *n*-Butoxycarbonyl-, 1-Methylpropoxycarbonyl-, 2-Methylpropoxy-carbonyl- oder *tert*-Butyloxycarbonylgruppe, die Allyloxycarbonyl-, 2,2,2-Trichlor-(1,1-dimethylethoxy)carbonyl- oder 9-Fluorenylmethoxycarbonyl-Gruppe oder eine Formyl-, Acetyl- oder Trifluoracetylgruppe in Frage.

Als Schutzgruppe für Hydroxyfunktionen kommt beispielsweise eine Trimethylsilyl-, Triethylsilyl-, Trüsopropyl-, *tert*-Butyldimethylsilyl- oder *tert*-Butyldiphenylsilylgruppe, eine *tert*-Butyl-, Benzyl-, 4-Methoxybenzyl- oder 3,4-Dimethoxybenzylgruppe in Frage.
Als Schutzgruppe für Hydroxycarbonylfunktionen kommt beispielsweise eine Alkylgruppe mit insgesamt 1 bis 5 Kohlenstoffatomen, beispielsweise die Methyl-, Ethyl-, *n*-Propyl-, Isopropyl-, *n*-Butyl-, *tert*-Butyl, Allyl-, 2,2,2-Trirhlorothyl-, Benzyl- oder 4-Methoxybenzylgruppe in Frage.

Die erhaltenen Verbindungen der allgemeinen Formel 1 können, sofern sie geeignete basische Funktionen enthalten, insbesondere für pharmazeutische Anwendungen in ihre physiologisch verträglichen Salze mit anorganischen oder organischen Säuren übergeführt werden. Als Säuren kommen hierfür beispielsweise Salzsäure, Bromwasserstoffsäure, Phosphorsäure, Salpetersäure, Schwefelsäure, Methansulfonsäure, Ethansulfonsäure, Benzolsulfonsäure, *p*-Totuoisutfonsäure, Essigsäure, Fumarsäure, Bernsteinsäure, Milchsäure, Mandelsäure, Äpfelsäure, Zitronensäure, Weinsäure oder Maleinsäure in Betracht.

Außerdem lassen sich die neuen Verbindungen der Formel I, falls sie Carbonsäurefunktion enthalten, in ihre Additionssalze mit anorganischen oder organischen Basen, insbesondere für die pharmazeutische Anwendung in ihre physiologisch verträglichen Additionssalze überführen. Als Basen kommen hierfür beispielsweise Natriumhydroxid, Kaliumhydroxid, Ammoniak, Cyclohexylamin, Dicyclohexylamin, Ethanolamin, Diethanolamin und Triethanolamin in Betracht.

Die vorliegende Erfindung betrifft Racemate, sofern die Verbindungen der allgemeinen Formel I nur ein Chiralitätselement besitzen. Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enantiomeren, aus denen sich die erwähnten Racemate zusammensetzen.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Die neuen Verbindungen der allgemeinen Formel I und deren physiologisch verträgliche Salze weisen wertvolle pharmakologische Eigenschaften auf, die auf ihre selektiven CGRP-antagonistischen Eigenschaften zurückgehen. Ein weiterer Gegenstand der Erfindung sind diese Verbindungen enthaltende Arzneimittel, deren Verwendung und deren Herstellung.

Die voranstehend genannten neuen Verbindungen und deren physiologisch verträgliche Salze besitzen CGRP-antagonistische Eigenschaften und zeigen gute Affinitäten in CGRP-Rezeptorbindungsstudien. Die Verbindungen weisen in den nachstehend beschriebenen pharmakologischen Testsystemen CGRP-antagonistische Eigenschaften auf.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC-Zellen werden in "Dulbecco's modified Eagle Medium" kultiviert. Das Medium konfluenter Kulturen wird entfernt. Die Zellen werden zweimal mit PBS-Puffer (Gibco 041-04190 M) gewaschen, durch Zugabe von PBS-Puffer, versetzt mit 0.02% EDTA, abgelöst und durch Zentrifugation isoliert. Nach Resuspension in 20 ml "Balanced Salts Solution" [BSS (in mM): NaCl 120, KCl 5.4, NaHCO₃ 16.2, MgSO₄ 0.8, NaHPO₄ 1.0, CaCl₂ 1.8, D-Glucose 5.5, HEPES 30, pH 7.40] werden die Zellen zweimal bei 100 x g zentrifugiert und in BSS resuspendiert. Nach Bestimmung der Zellzahl werden die Zellen mit Hilfe eines Ultra-Turrax homogenisiert und für 10 Minuten bei 3000 x g zentrifugiert. Der Überstand wird verworfen und das Pellet in Tris-Puffer (10 mM Tris, 50 mM NaCl, 5 mM MgCl₂, 1 mM EDTA, pH 7.40), angereichert mit 1 % Rinderserum-Albumin und 0.1% Bacitracin, rezentrifugiert und resuspendiert (1 ml / 1000000 Zellen). Das Homogenat wird bei -80°C eingefroren. Die Membranpräparationen sind bei diesen Bedingungen für mehr als 6 Wochen stabil.

Nach Auftauen wird das Homogenat 1:10 mit Assay-Puffer (50 mM Tris, 150 mM NaCl, 5 mM MgCl₂, 1 mM EDTA, pH 7.40) verdünnt und 30 Sekunden lang mit einem Ultra-Turrax homogenisiert. 230 µl des Homogenats werden für 180 Minuten bei Raumtemperatur mit 50 pM ¹²⁵I-Iodotyrosyl-Caicitonin-Gene-Relsted Peptide (Amersham) und ansteigenden Konzentrationen der Testsubstanzen in einem Gesamtvolumen von 250 µl inkubiert. Die Inkubation wird durch rasche Filtration durch mit Polyethylenimin (0.1 %) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM humanem CGRP-alpha während der Inkubation definiert.

Die Analyse der Konzentrations-Bindungskurven erfolgt mit Hilfe einer Computergestützten nichtlinearen Kurvenanpassung.

Die eingangs erwähnten Verbindungen zeigen in dem beschriebenen Test IC₅₀-Werte ≤ 10000 nM.

### B. CGRP-Antagonismus in SK-N-MC-Zellen

SK-N-MC-Zellen (1 Mio. Zellen) werden zweimal mit 250 µl Inkubationspuffer (Hanks' HEPES, 1 mM 3-Isobutyl-1-methylxanthin, 1% BSA, pH 7.4) gewaschen und bei 37°C für 15 Minuten vorinkubiert. Nach Zugabe von CGRP (10 pl) als Agonist in steigenden Konzentrationen (10⁻¹¹ bis 10⁻⁶ M) bzw. zusätzlich von Substanz in 3 bis 4 verschiedenen Konzentrationen wird nochmals 15 Minuten inkubiert.

Intrazelluläres cAMP wird anschließend durch Zugabe von 20 µl 1M HCl und Zentrifugation (2000 x g, 4°C für 15 Minuten) extrahiert. Die Überstände werden in flüssigem Stickstoff eingefroren und bei -20°C gelagert.

Die cAMP-Gehalte der Proben werden mittels Radioimmunassay (Fa. Amersham) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁵ M.

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv:
Nicht-insulinabhängigen Diabetes mellitus ("NIDDM"), cardiovaskuläre Erkrankungen, Morphintoleranz, clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindemde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet,

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann,

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidaien Antiphlogistica, Corticosteroiden, Calcium-Antagonisten, 5-HT_{1B/1D}-Agonisten oder andere Antimigranemittetn, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/-Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragées, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetylsalicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lomoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PO-typ Blockern, N-typ Blockern, Kaliumkanalörffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptoren Agonisten, wie z.B. CB2-Agonisten, CB1-Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosolformulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel I gemäß der obigen bevorzugten Ausführungsformen.

Besonders bevorzugt ist es, wenn die Verbindungen der Formel I oral verabreicht werden, besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum. Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbsfiverständlich außer den genannten Trägerstoffen auch Zusätze, wie z.B. Natriumcitrat, Calciumcarbonat und Dicalciumphrosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel 1 inhalativ verabreicht werden, besonders bevorzugt ist es, wenn die Verabreichung ein oder zweimal täglich erfolgt, Hierzu müssen die Verbindungen der Formel I in inhalierbaren Darreichungsformen bereitgestellt werden. Als inhalierbare Darreichungsformen kommen Inhalationspulver, treibgashaltige Dosieraerosole oder treibgasfreie inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### Inhalatlonspulver

Sind die Verbindungen der Formel I im Gemisch mit physiologisch unbedenklichen Hilfsstoffen enthalten, können zur Darstellung der erfindungsgemäßen Inhalationspulver die folgenden physiologisch unbedenklichen Hilfsstoffe zur Anwendung gelangen: Monosaccharide (z.B. Glucose oder Arabinose), Disaccharide (z.B. Lactose, Saccharose, Maltose), Oligo- und Polysaccharide (z.B. Dextrane), Polyalkohole (z.B. Sorbit, Mannit, Xylit), Salze (z.B. Natriumchlorid, Calciumcarbonat) oder Mischungen dieser Hilfsstoffe miteinander. Bevorzugt gelangen Mono- oder Disaccharide zur Anwendung, wobei die Verwendung von Lactose oder Glucose, insbesondere, aber nicht ausschließlich in Form ihrer Hydrate, bevorzugt ist. Als besonders bevorzugt im Sinne der Erfindung gelangt Lactose, höchst bevorzugt Lactosemonohydrat als Hilfsstoff zur Anwendung. Verfahren zur Herstellung der erfindungsgemäßen Inhatationspulver durch Mahlen und Mikronisieren sowie durch abschließendes Mischen der Bestandteile sind aus dem Stand der Technik bekannt,

### Treibgashaltlge Inhalationsaerosole

Die im Rahmen der erfindungsgemäßen Verwendung einsetzbaren treibgashaltigen Inhalationsaerosole können I im Treibgas gelöst oder in dispergierter Form enthalten, Die zur Herstellung der Inhalationsaerosole einsetzbaren Treibgase sind aus dem Stand der Technik bekannt. Geeignete Treibgase sind ausgewählt aus der Gruppe bestehend aus Kohlenwasserstoffen wie n-Propan, n-Butan oder Isobutan und Halogenkohlenwasserstoffen wie bevorzugt fluorierten Derivaten des Methans, Ethans, Propans. Butans, Cyclopropans oder Cyclobutans. Die vorstehend genannten Treibgase können dabei allein oder in Mischungen derselben zur Verwendung kommen. Besonders bevorzugte Treibgase sind fluorierte Alkanderivate ausgewählt aus TG134a (1,1,1,2-Tetrafluorethan), TG227 (1,1,1,2,3,3,3-Heptafluorpropan) und Mischungen derselben. Die im Rahmen der erfindungsgemäßen Verwendung einsetzbaren treibgashaltigen Inhalationsaerosole können ferner weitere Bestandteile wie Co-Solventien, Stabilisatoren, oberflächenaktive Mittel (surfactants), Antioxidantien, Schmiermittel sowie Mittel zur Einstellung des pH-Werts enthalten. All diese Bestandteile sind im Stand der Technik bekannt

### Treibgasfreie Inhalationslösungen

Die erfindungsgemäße Verwendung von Verbindungen der Formel I erfolgt bevorzugt zur Herstellung von treibgasfreien Inhalationslösungen und Inhalationssuspensionen. Als Lösungsmittel kommen hierzu wässrige oder alkoholische, bevorzugt ethanolische Lösungen in Betracht. Das Lösungsmittel kann ausschließlich Wasser sein oder es ist ein Gemisch aus Wasser und Ethanol, Die Lösungen oder Suspensionen werden mit geeigneten Säuren auf einen pH-Wert von 2 bis 7, bevorzugt von 2 bis 5 eingestellt. Zur Einstellung dieses pH-Werts können Säuren ausgewählt aus anorganischen oder organischen Säuren Verwendung finden. Beispiele für besonders geeignete anorganische Säuren sind Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure. Maleinsäure. Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit einem der Wirkstoffe ein Säureadditionssalz bilden. Unter den organischen Säuren sind Ascorbinsäure, Fumarsäure und Zitronensäure bevorzugt. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe, Antioxidantien oder Komplexbildner besitzen, wie beispielsweise Zitronensäure oder Ascorbinsäure. Erfindungsgemäß besonders bevorzugt wird Salzsäure zur Einstellung des pH-Werts verwendet.

Den im Rahmen der erfindungsgemäßen Verwendung einsetzbaren treibgasfreien Inhalationslösungen können Cosolventien und/oder weitere Hilfsstoffe zugesetzt werden. Bevorzugte Cosolventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester.
Unter Hilfs- und Zusatzstoffen wird in diesem Zusammenhang jeder pharmakologisch verträgliche Stoff verstanden, der kein Wirkstoff ist, aber zusammen mit dem (den) Wirkstoff(en) in dem pharmakologisch geeigneten Lösungsmittel formuliert werden kann, um die qualitativen Eigenschaften der Wirkstofffarmulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Polysorbate, Polyvinylpyrrolidon, sonstige Stabilisatoren, Komplexbildner, Antioxidantien und/oder Konservierungsstoffe, die die Verwendungsdauer der fertigen Arzneimitfieiformutierung gewährleisten oder verlängern, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid als Isotonantien. Zu den bevorzugten Hilfsstoffen zählen Antioxidantien, wie beispielsweise Ascorbinsäure, sofern nicht bereits für die Einstellung des pH-Werts verwendet, Vitamin A. Vitamin E, Tocopherole und ähnliche im menschlichen Organismus vorkommende Vitamine oder Provitamine. Konservierungsstoffe können eingesetzt werden, um die Formulierung vor Kontamination mit Keimen zu schützen. Als Konservierungsstoffe eignen sich die dem Stand der Technik bekannten, insbesondere Cetylpyridiniumchlorid, Benzalkoniumchlorid oder Benzoesäure bzw. Benzoate wie Natriumbenzoat in der aus dem Stand der Technik bekannten Konzentration.

Für die oben beschriebenen Behandlungsformen werden gebrauchsfertige Packungen eines Medikaments zur Behandlung von Atemwegserkrankungen, beinhaltend eine beigelegte Beschreibung, welche beispielsweise die Worte Atemwegserkrankung, COPD oder Asthma enthalten, ein Pteridin und ein oder mehrere Kombinationspartner ausgewählt aus der oben beschriebenen Gruppe, bereit gestellt.

### EXPERIMENTELLER TEIL

Für die hergestellten Verbindungen liegen in der Regel IR-, ¹H-NMR und/oder Massenspektren vor.
Wenn nicht anders angegeben, werden R_{f}-Werte unter Verwendung von DC-Fertigplatten Kieselgel 60 F254 (E. Merck, Darmstadt, Artikel-Nr. 1,05714) ohne Kammersättigung bestimmt.
Die unter der Bezeichnung Polygram-Alox ermittelten R_{f}-Werte werden unter Verwendung von DC-Fertigfolien Polygram Alox N/UV₂₅₄ (beschichtet mit 0.2 mm Aluminiumoxid) der Firma Macherey-Nagel (Düren, Artikel-Nr. 802 021) erhoben.
Die bei den Fliessmitteln angegebenen Verhältnisse beziehen sich auf Volumeneinheiten der jeweiligen Lösungsmittel, Die angegebenen Volumeneinheiten bei NH₃ beziehen sich auf eine konzentrierte Lösung von NH₃ in Wasser.
Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX*^{™}*, 35-70 µm) verwendet.
Zu chromatographischen Reinigungen wird Aluminiumoxid (Alox) der Firma ICN Biomedicals (Eschwege, Artikelnummer 02090) verwendet. Gemäss Herstellerangaben wird vor Verwendung die benötigte Aktivitätsstufe erzeugt.
Die angegebenen HPLC-Daten werden unter nachstehend angeführten Parametern gemessen:
Methode A:

| Zeit (min) | Volumenprozent Wasser | Volumenprozent Acetonitril |
|---|---|---|
| | (mit 0.1% Ameisensäure) | (mit 0.1 % Ameisensäure) |
| 0 | 95 | 5 |
| 9 | 10 | 90 |
| 10 | 10 | 90 |
| 11 | 95 | 5 |

Analytische Säule: Zorbax-Säule (Agilent Technologies), SB (Stable Bond) C18; 3.5 µm; 4.6 x 75 mm; Säulentemperatur; 30°C; Fluss: 0.8 mL / min; Injektionsvolumen: 5 µL; Detektion bei 254 nm
Methode B:

| Zeit (min) | Volumenprozent Wasser | Volumenprozent Acetonitril |
|---|---|---|
| | (mit 0.1 % Ameisensäure) | (mit 0.1% Ameisensäure) |
| 0 | 95 | 5 |
| 4.5 | 10 | 90 |
| 5 | 10 | 90 |
| 5.5 | 90 | 10 |

Analytische Säule: Zorbax-Säule (Agilent Technologies), SB (Stable Bond) C18; 3.5 µm; 4.6 x 75 mm; Säulentemperatur: 30°C; Fluss: 1.6 mL / min; Injektionsvolumen: 5 µL; Detektion bei 254 nm
Methode C:

| Zeit (min) | Volumenprozent Wasser | Volumenprozent Acetonitril |
|---|---|---|
| | (mit 0.1% Ameisensäure) | (mit 0.1% Ameisensäure) |
| 0 | 95 | 5 |
| 4 | 50 | 50 |
| 4.5 | 10 | 90 |
| 5 | 10 | 90 |
| 6.5 | 90 | 10 |

Analytische Säule: Zorbax-Säule (Agilent Tochnologies), SB (Stable Bond) C18; 3.5 µm; 4.6 x 75 mm, Säulentemperatur: 30°C; Fluss: 1.6 mL / min; Injektionsvolumen: 5 µL; Detektion bei 254 nm
Methode D:

| Zeit (min) | Volumenprozent Wasser | Volumenprozent Acetonitril |
|---|---|---|
| | (mit 0.04% TFA) | (mit 0.04% TFA) |
| 0 | 80 | 20 |
| 15 | 20 | 80 |
| 17 | 20 | 80 |

Analytische Säule: Symmetry C8 Waters - 4.6 X 150 mm; 5 micron, Fluss: 1.3 ml/min, Säulentemperatur 25°C, Detektion bei 254 nm.

Bei präparativen HPLC-Reinigungen werden in der Regel die gleichen Gradienten verwendet, die bei der Erhebung der analytischen HPLC-Daten benutzt wurden.
Die Sammlung der Produkte erfolgt massengesteuert, die Produkt enthaltenden Fraktionen werden vereinigt und gefriergetrocknet.
Falls nähere Angaben zur Konfiguration fehlen, bleibt offen, ob es sich um reine Enantiomere handelt oder ob partielle oder gar völlige Racemisierung eingetreten ist,

In den Versuchsbeschreibungen werden die folgenden Abkürzungen verwendet:
- Cyc: Cyclohexan
- DCM: Dichlormethan
- DIPE: Diisopropylether
- DMF: *N,N*-Dimethylformamid
- EtOAc: Essigsäureethylester
- EtOH: Ethanol
- HATU: O-(7-Azabenztriazol-1-yl)-*N,N,N',N'*-tetramethyturonium-hexafluorophosphat
- AcOH: Essigsäure
- i.vac.: in vacuo (im Vakuum)
- MCPBA: *m*-Chlorperbenzoesäure

- MeOH: Methanol
- NaOAc: Natriumacetat
- PE: Petrolether
- RT: Raumtemperatur
- TBME: *tert*-Butylmethylether
- TBTU: O-(Benzotriazol-1-yl)-*N,N,N',N',*-tetramethyluronium-tetrafluoroborat
- TFA: Trifluoressigsäure
- THF: Tetrahydrofuran

### Amin A1

### [1,4']Bipiperidinyl-4-carbonitril

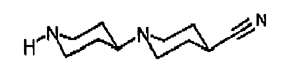

### A1 a) 4-Cyano-[1,4]bipiperidinyl-1'-carbonsäure-tert-butylester

Zu einer auf 0°C gekühlten Suspension von 5.44 g (27.3 mmol) 4-Oxo-piperidin-1-carbonsäure-*tert*-butylester, 3.00 g (27.2 mmol) 4-Cyanopiperidin und 1.6 mL (28.0 mmol) AcOH in 400 mL DCM wurden portionenweise 6.09 g (27.3 mmol) NaBH(OAc)₃ zugegeben und das Reaktionsgemisch über Nacht gerührt, wobei es sich auf RT erwärmte. Man versetzte mit 400 mL Wasser und säuerte mit Zitronensäure an. Die organische Phase wurde abgetrennt, die wässrige Phase mit gesättigter NaHCO₃-Lösung alkalisch gestellt und erschöpfend mit DCM extrahiert. Die vereinigten organischen Phasen wurden i.vac. eingeengt und der Rückstand mit wenig DIPE kristallisiert.
- Ausbeute:: 7.1 g (89% der Theorie)
- ESI-MS:: (M+H)⁺ = 294

### A1b) [1,4]Bipiperidinyl-4-carbonitril

Zu einer Suspension von 7.1 g (24.2 mmol) 4-Cyano-[1,4']bipiperidinyl-1'-carbonsäure-*tert*-butylester in 100 mL DCM wurden 10 mL TFA gegeben und das Reaktionsgemisch über Nacht bei RT gerührt. Man engte i.vac. ein, nahm den Rückstand in wenig EtOH auf und versetzte mit etherischer HCl. Das ausgefallene Salz wurde abfiltriert und getrocknet. Man erhielt das Produkt als Bis-Hydrochlorid-Salz.
- Ausbeute:: 3.6 g (56% der Theorie)
- ESI-MS:: (M+H)⁺ = 194

### Amin A2

### Essigsäure- [1,4']bipiperidinyl-4-yl-ester

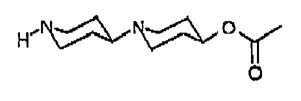

### A2a) 1'-Benzyl-[1,4']bipiperidinyl-4-ol

Zu einer Mischung aus 15.0 mL (79.3 mmol) 1-Benzyl-piperidin-4-on und 8.0 g (79.3 mmol) 4-Hydroxy-piperidin wurden 25.7 mL (87,4 mmol) Titan(IV)-isopropoxid zugetropft und das Reaktionsgemisch 2 h bei RT gerührt. Man verdünnte mit 150 mL EtOH und versetzte portionenweise mit NaBH₃CN. Nach beendeter Zugabe wurde das Reaktionsgemisch 70 h bei RT gerührt. Man versetzte mit 60 ml. Wasser, filtrierte unlösliche Bestandteile über Celite ab und engte das Filtrat i.vac. ein. Weitere Reinigung erfolgte durch Chromatographie (Kieselgel. DCM/MeOH/NH₃ 80:20:1).
- Ausbeute:: 17.1 g (79% der Theorie)
- ESI-MS:: (M+H)⁺ = 275
- R_{f} =: 0.51 (Kieselgel, DCM/MeOH/NH₃ 80:20:1)

### A2b) Essigsäure-1'-benzyl-[1,4']bipiperidinyl-4-yl-ester

Eine Lösung von 1.5 g (5.47 mmol) 1'-Benzyl-[1,4']bipiperidinyl-4-ol in 30 mL Essigsäureanhydrid wurde 1 h auf 50°C (Badtemperatur) erwärmt. Man engte i.vac, ein und reinigte den Rückstand chromatographisch (Kieselgel, EtOAc/MeOH/NH₃ 95:5:0.5).
- Ausbeute:: 1.46 g (84% der Theorie)
- ESI-MS:: (M+H)⁺ = 317
- R_{f} =: 0.43 (Kieselgel, EtOAc/MeOH/NH₃ 90:10:1)

### A2c) Essigsäure-[1,4']biperidinyl-4-yl-ester

Eine Suspension von 1.4 g (4.42 mmol) Essigsäure-1'-benzyl-[1,4']bipiperidinyl-4-yl-ester und 200 mg 10% Pd/C in 20 mL MeOH wurde bei 3 bar Wasserstoffdruck 8 h bei RT und 3 h bei 40°C hydriert. Dann erfolgte die Zugabe von weiteren 200 mg 10% Pd/C und das Reaktionsgemisch wurde erneut 6 h hydriert. Zur Vervollständigung der Reaktion wurde mit 200 mg Pd(OH)₂ versetzt und weitere 12 h hydriert. Der Katalysator wurde abgesaugt und das Filtrat eingeengt. Man erhielt das Produkt als zähflüssiges Öl.
- Ausbeute:: 1,00 g (100% der Theorie)
- ESI-MS:: (M+H)⁺ = 227
Retentionszeit (HPLC-MS): 0.5 min (Methode B)

### Amin A3

### 4-Methoxy-[1,4']bipiperidinyl

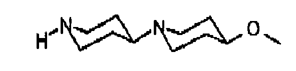

### A3a) 1'-Benzyl-4-methoxy-[1,4']bipiperidinyl

Unter Stickstoffatmosphäre wurde zu einer auf 0°C gekühlten Lösung von 5.00 g (43.4 mmol) 4-Methoxy-piperidin und 9.30 mL (52.1 mmol) 1-Benzyl-piperidin-4-on in 200 mL DCM 2.4 mL (43.4 mmol) AcOH gegeben, das Reaktionsgemisch 2 h bei dieser Temperatur gerührt und dann portionenweise mit 19.3 g (91.2 mmol) NaBH(OAc)₃ versetzt. Nach beendeter Zugabe wurde über Nacht bei RT gerührt, die Reaktionslösung mit 30%-iger K₂CO₃-Lösung alkalisch gestellt und mit 200 mL DCM versetzt. Die organische Phase wurde abgetrennt, mit 15% K₂CO₃-Lösung gewaschen und i.vac. eingeengt. Der ölige Rückstand wurde mit methanolischer HCl (1.25 M) sauer gestellt, i.vac. eingeengt, der Rückstand in EtOH aufgenommen, das ausgefallene Produkt abgesaugt, mit PE verrieben, filtriert und getrocknet. Das Produkt fiel als Bis-Hydrochiorid-Salz an.
- Ausbeute:: 2.1 g (13% der Theorie)

### A3b) 4-Methoxy-[1,4']bipiperidinyl

Eine Suspension von 2.1 (5.87 mmol) 1'-Benzyl-4-methoxy-[1,4']bipiperidinyl und 1.0 g 10% Pd/C in 50 mL MeOH und 20 mL Wasser wurde 8 h bei RT und 3 bar Wasserstoffdruck hydriert. Der Katalysator wurde abgesaugt, das Filtrat i.vac. eingeengt, der Rückstand mit Diethylether verrieben, abgesaugt und getrocknet.
- Ausbeute:: 1.50 g (94% der Theorie)
- ESI-MS:: (M+H)⁺ = 199

### Amin A4

### 1-(1,1-Dioxo-hexahydro-1λ⁶-thiopyran-4-yl)-piperazin

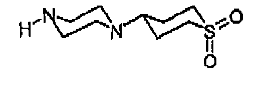

### A4a) 1,1-Dioxo-tetrahydro-1λ⁶-thiopyran-4-on

Eine Lösung von 5.3 g (45.6 mmol) Tetrahydrothiopyran-4-on in 50 mL EtOAc wurde auf 50°C erwärmt. Bei dieser Temperatur wurden 18.6 mL (110 mmol) Peressigsäure-Lösung (39% in AcOH) langsam zugetropft, wobei die Temperatur mittels Eisbades zwischen 50-55°C gehalten wurde. Nach beendeter Zugabe wurde das Reaktionsgemisch auf RT abgekühlt, wobei ein Niederschlag ausfiel. Dieser wurde abgesaugt und getrocknet. Das Produkt wurde ohne Reinigung weiter umgesetzt.
- Ausbeute:: 6.70 g (99% der Theorie, Reinheit 80%)
- EI-MS:: (M)⁺ = 148
- R_{f} =: 0.50 (Kieselgel, EtOAc)

### A4b) 1-Benzyl-4-(1,1-dioxo-hexahydro-1λ⁶-thiopyran-4-yl)-piperazin

Eine Suspension von 5.1 g (27.5 mmol, Reinheit 80%) 1,1-Dioxo-tetrahydro-1λ⁶-thio-pyran-4-on, 7.0 mL (40.3 mmol) 1-Benzyl-piperazin und 30 g Molekularsieb A3 in 140 mL Isopropanol wurde mit einer Spatelspitze *p*-Toluolsulfonsäure versetzt und 7 h unter Rückfluss erhitzt. Vom Molekularsieb wurde abfiltriert, die Reaktionslösung mit 8.0 g (37.7 mmol) NaBH(OAc)₃ versetzt und über Nacht bei RT gerührt. Zur Reaktionslösung wurde mit 1 g NaBH₄ gegeben, 2 h auf 60°C erwärmt, mit Wasser versetzt und anschließend filtriert. Das Filtrat wurde mit 4 M HCl angesäuert, i.vac. vom Isopropanol befreit, mit gesättigter NaHCO₃-Lösung alkalisch gestellt und erschöpfend mit EtOAc extrahiert. Die vereinigten organischen Phasen wurden über Na₂SO₄ getrocknet, filtriert und i.vac, eingeengt. Der Rückstand wurde chromatographisch (Alox, Aktivitätsstufe II-III, EtOAc) gereinigt.
- Ausbeute:: 0.64 g (7% der Theorie)
- ESI-MS:: (M+H)⁺ = 309
- R_{f} =: 0.50 (Polygram-Alox, EtOAc)

### A4c) 1-(1,1-Dioxo-hexahydro-1λ⁸-thiopyran-4-yl)-piperazin

Eine Suspension von 0.57 g (1.85 mmol) 1-Benzyl-4-(1,1-dioxo-hexahydro-1λ⁶-thiopyran-4-yl)-piperazin und 300 mg 10% Pd/C in 30 mL MeOH wurde bei 50°C und 50 psi Wasserstoffdruck 10 h hydriert. Der Katalysator wurde abgesaugt und das Filtrat i.vac. eingeengt. Das Produkt wurde ohne Reinigung weiter umgesetzt.
- Ausbeute:: 0.38 g (94% der Theorie)
- ESI-MS:: (M+H)⁺ = 219

### Amin A5

### 1-Methyl-4-(4-methyl-piparidin-4-yl)-piparazin

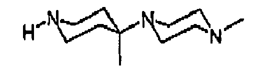

### A5a) 1-Benzyl-4-(4-methyl-piperazin-1-yl)-piperidin-4-carbonitril

Eine Lösung von 10.0 g (45.3 mmol) 1-Benzyl-4-hydrooy-piperidin-4-carbonitril und 6.0 mL (53.6 mmol) *N*-Methyl-piperazin in 50 mL MeOH wurde 6 h unter Rückfluss erhitzt. Man engte i,vac. auf ca. 25 mL ein, wobei ein Niederschlag ausfiel. Man versetzte mit 50 mL Eiswasser, filtrierte den Niederschlag ab und trocknete diesen bei 40°C.
- Ausbeute:: 11.5 g (85% der Theorie)
- ESI-MS;: (M+H)⁺ = 299
- R_{f} =: 0.58 (Polygram-Alox, PE/EtOAc 1:1)

### A5b) 1-(1-Benzyl-4-methyl-piperidin-4-yl)-4-methyl-piperazin

Zu einer Lösung von 3.0 g (10.1 mmol) 1-Benzyl-4-(4-methyl-piporazin-1-yl)-piperidin-4-carbonitril in 50 mL trockenem THF wurden bei RT 10 mL Methylmagnesiumchlorid-Lösung (30 mmol, 3 M in THF) zugegeben und das Reaktionsgemisch 2 h gerührt, Man versetzte mit gesättigter NH₄Cl-Lösung, rührte 10 min nach, extrahierte erschöpfend mit Diethylether und trocknete die vereinigten organischen Phasen über Na₂SO₄. Nach Entfernen des Trocken- und Lösungsmittels wurde der Rückstand chromatographisch (Alox, Aktivitätsstufe II-III, Gradient DCM/MeOH 100:1 zu 50;1) gereinigt.
- Ausbeute:: 1.01 g (33% der Theorie)

- ESI-MS:: (M+H)⁺ = 288

### A5c) 1-Methyl-4-(4-methyl-piperidin-4-yl)-piperazin

Eine Suspension von 0.99 g (3.27 mmol) 1-(1-Benzyl-4-methyl-piperidin-a-yl)-4-methyl-piperazin und 200 mg 10% Pd/C in 25 mL MeOH wurde bei 50°C und 50 psi Wasserstoffdruck 2 h hydriert. Zur Vervollständigung der Reaktion wurde erneut mit 200 mg Katalysator versetzt und das Reaktionsgemisch weitere 22 h bei 60°C und 50 psi Wasserstoffdruck hydriert. Der Katalysator wurde abfiltriert und das Filtrat zur Trockene eingeengt. Das Produkt wurde ohne Reinigung weiter umgesetzt.
- Ausbeute:: 0.56 g (87% der Theorie)
- ESI-MS:: (M+H)⁺ = 198

### Amin A6

### 4-(4-Methyl-piperidin-4-yl}-morpholin

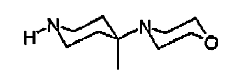

### A6a) 1-Benzyl-4-morpholin-4-yl-piperidin-4-carbonitril

Eine Lösung von 5.25 g (23.8 mmol) 1-Benzyl-4-hydroxy-piperidin-4-carbonitril und 2.2 mL (25.2 mmol) Morpholin in 30 mL MeOH wurde 6 h unter Rückfluss erhitzt. Man engte i,vac. zur Trockene ein, wobei das Produkt in Form von Kristallen anfiel.
- Ausbeute:: 6.9g(100% der Theorie)
- ESI-MS:: (M+H)⁺ = 286

### A6b) 4-(1-Benzyl-4-methyl-piperidin-4-yl)-morpholin

Hergestellt analog Beispiel A5b aus 6.88 g (23.6 mmol) 1-Benzyl-4-morpholin-4-yl-piperidin-4-carbonitril und 25 mL Methylmagnesiumchlorid-Lösung (75 mmol, 3 M in THF).
- Ausbeute:: 3.0 g (44% der Theorie)
- ESI-MS:: (M+H)⁺ = 275
- R_{f}=: 0.6 (Polygram-Alox, DCM/MeOH 50:1)

### A6c) 4-(4-Methyl-piperidin-4-yl)-morpholin

Eine Suspension von 3.0 g (10.4 mmol) 4-(1-Benzyl-4-methyl-piperidin-4-yl)-morpholin und 150 mg 10% Pd/C in 50 mL MeOH wurde bei 50°C und 50 psi Wasserstoffdruck 2 h hydriert. Man versetzte mit 1 mL konzentrierter HCl und hydrierte weitere 18 h bei 50°C und 50 psi Wasserstoffdruck. Der Katalysator wurde abfiltriert und das Filtrat zur Trockene eingeengt. Das Produkt, das als Hydrochlorid-Salz anfiel, wurde ohne Reinigung weiter umgesetzt.
- Ausbeute:: 2.4 g (94% der Theorie)
- ESI-MS:: (M+H)⁺ = 185

### Amin 7

### 3-[4,4']Bipiperidinyl-1-yl-propionsäureethylester

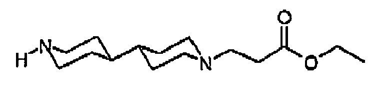

### A7a) 1'-(2-Ethoxycarbonyl-ethyl)-[4,4']bipiperidinyl-1-carbonsäure-tert-butylester

Zu einer Lösung von 10.0 g (37.3 mmol) [4,4']Bipiperidinyl-1-carbonsäure-*tert*-butylester in 100 mL EtOH wurden 4.4 mL (40.6 mmol) Acrylsäureethylester gegeben und das Reaktionsgemisch 2 h unter Rückfluss erhitzt. Zur Vervollständigung der Reaktion wurden weitere 1 mL (9.2 mmol) Acrylsäureethylester zugegeben, 1 h unter Rückfluss erhitzt und über Nacht bei RT belassen. Man entfernte i.vac. das Lösungsmittel und setzte das Rohprodukt ohne Reinigung weiter um.
- Ausbeute:: 14.0 g (100% der Theorie)
- ESI-MS:: (M+H)⁺ = 369

### A7b) 3-[4,4']Bipiperidinyl-1-yl-propionsäureethylester

Zu einer Lösung von 14.0 g des Rohproduktes aus Beispiel A7a in 250 mL DCM wurden 28 mL TFA zugetropft und das Reaktionsgemisch 4 h bei RT gerührt. Man engte i.vac. ein, nahm den Rückstand in 200 mL DCM auf und gab diese Lösung portionenweise zu einer Lösung von 20 g Na₂CO₃ in 120 mL Wasser. Die organische Phase wurde abgetrennt, die wässrige Phase noch zweimal mit DCM extrahiert und die vereinigten organischen Phasen über Na₂SO₄ getrocknet. Nach Entfernen des Trocken- und Lösungsmittels wurde der Rückstand getrocknet und ohne weitere Reinigung umgesetzt.
- Ausbeute:: 8.8 g (88% der Theorie)

- ESI-MS:: (M+H)⁺ = 269

### Amin A8

### 3-[1,4']Bipiperidinyl-4-yl-propionsäure-ethylester

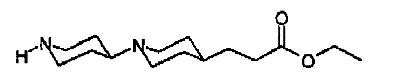

### A8a) 4-(2-Methoxycarbonyl-ethyl)-[1,4']bipiperidinyl-1'-carbonsäure-tert-butylester

Eine Lösung von 4.00 g (19.3 mmol) 3-Piperidin-4-yl-propionsäuremethylester (eingesetzt als Hydrochiond-Salz) und 3.85 g (19.3 mmol) 4-Oxo-piperidin-1-carbonsäure-*tert*-butylester in 50 mL THF wurde mit AcOH auf pH 5 gebracht und das Reaktionsgemisch 1 h bei RT gerührt. Man versetzte unter Kühlung portionenweise mit 5.15 g (24.3 mmol) NaBH(OAc)₃ und rührte die Reaktionslösung über Nacht bei RT. Man versetzte tropfenweise mit 90 mL 30% K₂CO₃-Lösung, extrahierte erschöpfend mit EtOAc und trocknete die vereinigten organischen Phasen über Na₂SO₄. Nach Entfernen des Trocken- und Lösungsmittels wurde das Produkt ohne Reinigung weiter umgesetzt.
- Ausbeute:: 5.4 g (79% der Theorie)
- ESI-MS:: (M+H)⁺= 355
- R_{f} =: 0.63 (Kieselgel, DCM/MeOH/NH₃ 80:20:2)

### A8b) 3-[1,4']Bipiperidinyl-4-yl-propionsäure-ethylester

Eine Lösung von 5.4 g (15.2 mmol)-4-(2-Methoxycarbonyl-ethyl)-[1,4']bipiperidinyl-1'-carbonsäure-*tert*-butylester in 150 mL ethanolischer HCl (1.25 M) wurde über Nacht bei RT gerührt. Das Lösungsmittel wurde größtenteils i.vac. entfernt, der entstandene Niederschlag abgesaugt und getrocknet. Das Produkt, das als Bis-Hydrochlorid-Salz anfiel, wurde ohne Reinigung weiter umgesetzt.
- Ausbeute:: 2.3 g (44% der Theorie)
- ESI-MS:: (M+H)⁺ = 269
- Retentionszeit (HPLC-MS):: 1.2 min (Methode B)

### Amin A9

### 4-(4-Piperazin-1-yl-piperidin-1-yl)-but-ansäureathylester

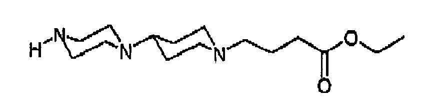

### A9a) 4-[4-(4-Benzyl-piperazin-1-yl)-piperidin-1-yl]-butansäureethylester

Eine Lösung von 3.11 g (12.0 mmol) 1-Benzyl-4-piperidin-4-yl-piperazin und 7.5 mL (12.0 mmol, 15% in Wasser) 4-Oxo-butansäure in 70 mL THF wurde mit AcOH auf pH 5 eingestellt und 1 h bei RT gerührt. Unter Eiskühlung wurde portionenweise mit 5.35 g (24.0 mmol) NaBH(OAC)₃ versetzt und anschließend über Nacht bei RT gerührt. Zum Reaktionsgemisch wurden 80 mL 30% K₂CO₃-Lösung innerhalb von 15 min zugetropft und dieses nach beendeter Zugabe zweimal mit EtOAc gewaschen. Die wässrige Phase wurde i.vac. zur Hälfte eingeengt, mit 1 M KHSO₄-Lösung neutralisiert, der entstandene Niederschlag abgesaugt, das Filtrat erneut mit EtOAc gewaschen und die wässrige Phase i.vac. eingeengt. Man versetzte mit 150 mL ethanolischer HCl (1.25 M) und rührt über Nacht bei RT. Man engte i.vac. ein, nahm den Rückstand in wenig 15% K₂CO₃-Lösung auf, extrahierte erschöpfend mit EtOAc und trocknete die vereinigten organischen Phasen Ober Na₂SO₄, Nach Entfernen des Trocken- und Lösungsmittels wurde der Rückstand ohne Reinigung weiter umgesetzt.
- Ausbeute:: 2.9 g (65% der Theorie)
- ESI-MS:: (M+H)⁺ = 374

### A9b) 4-(4-Pierazin-1-yl-piperidin-1-yl)-butansäureethylester

Eine Suspension von 2.9 g (7.76 mmol) 4-[4-(4-Benzyl-piperazin-1-yl)-piperidin-1-yl]-butansäureethylester und 300 mg 10% Pd/C in 60 mL MeOH wurde bei RT und 50 psi Wasserstoffdruck 24 h hydriert, Man filtrierte den Katalysator ab, engte i.vac. ein, nahm den Rückstand in wenig DIPE und Isopropanol auf und versetzte mit 4 M HCl in 1,4-Dioxan. Das ausgefallene Bis-Hydrochlorid-Salz wurde abgetrennt und getrocknet.
- Ausbeute:: 2.5 g (90% der Theorie)
- ESI-MS:: (M+H)⁺ = 284
- Retentionszeit (HPLC-MS):: 0.7 min (Methode B)

### Amin A10

### 3-(4-Piperazin-1-yl-pipendin-1-yl)-proplonsäureethylester

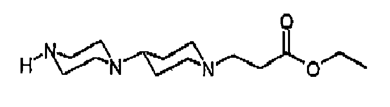

### A10a) 3-[4-(4-Benzyl-piperazin-1-yl)-piperidin-1-yl]-propionsaureethylester

Zu einer Lösung von 11.7 g (44.9 mmol) 1-Benzyl-4-piperidin-4-yl-piperazin in 120 mL trockenem EtOH wurden 5.5 mL (50.8 mmol) Acrylsäureethylester gegeben und das Reaktionsgemisch 1 h unter Rückfluss erhitzt und dann über Nacht bei RT gerührt. Man entfernte das Lösungsmittel i.vac. und trocknete den Rückstand 1 h im Ölpumpenvakuum. Das Rohprodukt wurde ohne Reinigung weiter umgesetzt.
- Ausbeute:: 16.5 g (99% der Theorie)
- ESI-MS;: (M+H)⁺ = 360

### A10b) 3-(4-Piperazin-1-yl-piperidin-1-yl)-propionsäureethylester

Eine Suspension von 16.5 g des Rohproduktes aus Beispiel A10a und 1.6 g 10 % Pd/C in 200 mL EtOH und wurde 4 h bei 50°C und 50 psi Wasserstoffdruck hydriert. Der Katalysator wurde abgesaugt, das Filtrat auf ca. 120 mL eingeengt und mit 72 mL ethanolischer HCl (1.3 M) versetzt. Der ausgefallene Niederschlag wurde abgesaugt und i.vac. getrocknet.
Das Produkt wurde als Bis-Hydrochlorid Salz erhalten.
- Ausbeute:: 12.6 g (83% der Theorie)
- ESI-MS:: (M+H)⁺ = 270

### Amin A11

### 3-(4-Piperidin-4-yl-piperazin-1-yl)-propionsäureethylester

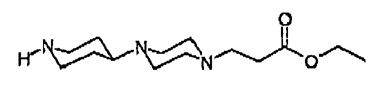

### A11a) 3-[4-(1-Benzyl-piperidin-4-yl)-piperazin-1-yl]-propionsäureethylester

Zu einer Lösung von 11.0 g (33.2 mmol) 1-(1-Benzyl-piperidin-4-yl)-piperazin (eingesetzt als Bis-Hydrochlorid Salz) in 40 mL EtOH wurden 12.5 mL (73.0 mmol) Ethyldiisopropylamin und 5.0 mL (46.1 mmol) Acrylsäureethylester gegeben und das Reaktionsgemisch 3 h auf 90°C (Badtemperatur) erhitzt, Nach dem Abkühlen wurde mit Wasser versetzt, erschöpfend mit EtOAc extrahiert und die vereinigten organischen Phasen über Na₂SO₄ getrocknet. Nach Entfernen des Trocken- und Lösungsmittels wurde der Rückstand chromatographisch (Kieselgel, DCM/EtOH/NH₃ 100:10:1) gereinigt.
- Ausbeute:: 6,8 g (56% der Theorie)
- ESI-MS:: (M+H)⁺ = 360
- R_{f} =: 0.64 (Kieselgel, DCM/MeOH/NH₃ 90:9:1)

### A11b) 3-(4-Piperidin-4-yl-piperazin-1-yl)-propionsäureethylester

Eine Suspension von 5.13 g (14.3 mmol) 3-[4-(1-Benzyl-piperidin-4-yl)-piperazin-1-yl]-propionsäureethylester und 1.0 g 10% Pd/C in 100 mL EtOH wurde 2 h bei 50°C und 50 psi Wasserstoffdruck hydriert. Der Katalysator wurde abfiltriert und das Filtrat zur Trockene eingeengt. Das ölige Produkt wurde ohne Reinigung weiter umgesetzt.
- Ausbeute:: 3.6 g (93% der Theorie)
- ESI-MS:: (M+H)⁺ = 270

### Amin A12

### ([1,4']Bipiperidinyl-4-yloxy)-essigsäureethylester

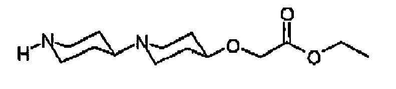

### A12a) 4-tert-Butoxvcarbonvimathoxy-[1,4']bipiperidinyl-1-carbonsäure-benzylester

Zu einer Lösung von 2.58 g (11.1 mmol)) 4-Oxo-pipendin-1-carbonsäure-*tert*-butylester und 2.80 g (12.4 mmol) (Piperidin-4-yloxy)-essigsäure-*tert*-butylester in 30 mL THF wurden portionenweise 2.90 g (13.3 mmol) NaBH(OAc)₃ gegeben und das Reaktionsgemisch Ober Nacht bei RT gerührt. Man versetzte mit 50 mL 1 M NaOH, rührte 1 h bei RT, extrahierte erschöpfend mit EtOAc und trocknete die vereinigten organischen Phasen über Na₂SO₄. Nach Entfernen des Trocken- und Lösungsmittels wurde der Rückstand chromatographisch (Alox, Aktivitätsstufe II-III, DCM/MeOH 100:1) gereinigt.
- Ausbeute:: 3.1 g (65% der Theorie)
- ESI-MS:: (M+H)⁺ = 433

### A12b) ([1.4]Bipiperidinyl-4-yloxy)-essigsaure-tert-butylester

Eine Suspension von 3.08 g (7.12 mmol) 4-tert-Butoxycarbonylmethoxy-[1,4]bipiperidiriyl-1'-carbonsäure-benzylester und 300 mg 10% Pd/C in 60 mL MeOH wurde bei 50°C und 50 psi Wasserstoffdruck 2 h hydriert. Der Katalysator wurde abgesaugt und das Filtrat zur Trockene eingeengt. Das verbleibende Öl wurde ohne Reinigung weiter umgesetzt.
- Ausbeute:: 2.15 g (99% der Theorie)
- ESI-MS:: (M+H)⁺ = 299

### A12c) ([1,4']Bipiperidinyl-4-yloxy)-essigsäureethylester

Eine Lösung von 100 mg (0.33 mmol) ([1,4']Bipiperidinyl-4-yloxy)-essigsäure-*tert*-butylester in 1 mL EtOH wurde in der Hitze mit 2 mL ethanolischer HCl versetzt, weitere 2 h unter Rückfluss erhitzt und über Nacht bei RT belassen. Der entstandene Niederschlag wurde abgesaugt und getrocknet.
- Ausbeute:: 71 mg (63% der Theorie)
- ESI-MS:: (M+H)⁺ = 271

### Beispiel 1

### 4-(2-Oxo-1,2,4,5-tetrahydro-1,3-benzdiezepin-3-yl)-pipeddin-1-carbonsäure-(R)-1-heptylcarbamoyl-2-(4-hydroxy-3,5-dirytethyl-phenyl)-ethytester

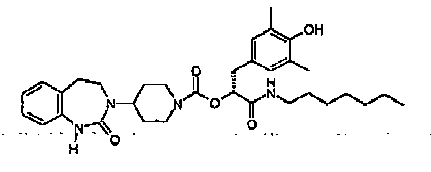

### 1a) 2-Benzyloxy-5-brom-1,3-dimethylbenzol

Zu einer Lösung von 50.0 g (249 mmol) 2,6-Dimethyl-4-bromphenol in 500 mL DMF wurden 39.9 g (286 mmol) K₂CO₃ gegeben und 20 min nachgerührt. Dann wurden 34.0 mL (286 mmol) Benzylchlorid langsam zugetropft und das Reaktionsgemisch 3 h bei 100°C Badtemperatur gerührt. Nach beendeter Umsetzung wurde auf 500 mL Wasser gegossen und mit EtOAc erschöpfend extrahiert. Die organischen Phasen wurden vereint, über Na₂SO₄ getrocknet und i. vac. eingeengt.
- Ausbeute:: quantitativ
- GC-MS:: (M⁺) = 290/292 (Br)

- R_{f} =: 0.87 (Kieselgel, Cyc/EtOAc 3:1)

### 1b) 2-Acetylamino-3-(4-benzyloxy-3,5-dimethyl-phenyl)-acrylsäuremethylester

Unter Stickstoffatmosphäre wurde eine Mischung aus 40.0 g (137 mmol) 2-Benzyloxy-5-brom-1,3-dimethylbenzol und 24.1 g (165 mmol) 2-Acetylamino-acrylsäuremethylester in 420 mL Triethylamin und 200 mL Acetonitril mit 3.5 g (11.2 mmol) Tri-o-tolyl-phosphan und 2.5 g (11.1 mmol) Pd(OAc)₂ versetzt und 18 h bei 80°C gerührt. Der Niederschlag wurde abgesaugt, das Filtrat i. vac. eingeengt und mit 800 mL DCM und 800 mL Wasser versetzt. Die organische Phase wurde abgetrennt, über Na₂SO₄ abgesaugt, das Lösungsmittel i.vac. entfernt, der Rückstand mit EtOAc verrührt, abgesaugt und i.vac getrocknet.
- Ausbeute:: 31.1 g (64% der Theorie)
- ESI-MS:: (M+H)⁺ = 354
- Retentionszeit (HPLC-MS);: 8.6 min (Methode A)

### 1c) 3-(4-Benzyloxy-3,5-dimethyl-phenyl)-2-oxo-propionsäure

31.1 g (88.1 mmol) 2-Acetylamino-3-(4-benzyroxy-3,5-dimethyl-phenyl)-acrylsäure-methylester in 150 mL 1,4-Dioxan wurden mit 125 mL 4 M HCl versetzt, 7 h unter Rückfluss und über Nacht bei RT gerührt, Der Niederschlag wurde abgesaugt, mit Wasser gewaschen und bei 45°C im Vakuumtmckenschrank getrocknet.
- Ausbeute:: 14.3 g (54 % der Theorie)
- EI-MS:: (M)⁺ = 298
- Retentionszeit (HPLC-MS):: 9.0 min (Methode A)

### 1d) (R)-3-(4-Benzoyl-3,5-dimethyl-phenyl)-2-hydroxy-propionsäure

Unter einer Stickstoffatmosphäre wurde eine Lösung von 14.3 g (47.8 mmol) 3-(4-Benzyl-oxy-3,5-dimethyl-phenyl)-2-oxo-propionsäure und 8.3 mL (59.8 mmol) Triethylamin in 170 mL THF bei -35°C mit einer Lösung von 22.1 (69.0 mmol) (1*R*)-*B*-Chlordiisopino-campheylboran in 70 mL THF innerhalb von 30 min versetzt. Nach beendeter Zugabe wurde das Kältebad entfernt und die Reaktionslösung über Nacht bei RT gerührt. Das Reaktionsgemisch wurde bei 0°C mit 70 mL 1 M NaOH alkalisch gestellt, mit 100 mL TBME versetzt, 15 min nachgerührt und die Phasen getrennt. Die organische Phase wurde mit 50 mL Wasser und dreimal mit je 50 mL 1 M NaOH gewaschen. Die vereinigten wässrigen Phasen wurden mit halbkonzentrierter HCl sauer gestellt, mit EtOAc erschöpfend extrahiert und die vereinigten organischen Phasen über Na₂SO₄ getrocknet. Nach Entfernen des Trocken- und Lösungsmittels wurde der Rückstand ohne Reinigung weiter umgesetzt.
- Ausbeute:: 14.0 g (98% der Theorie)
- ESI-MS:: (M-H)⁻ = 299
- Retentionszeit (HPLC-MS):: 7.9 min (Methode A)

### 1e) (R)-3-(4-Benzyloxy-3,5-dimethyl-phenyl)-2-hydroxy-propionsäuremethylester

Zu einer auf 0°C gekühlten Lösung von 14.0 g (23.3 mmol) (R)-3-(4-Benzoyl-3,5-dimethylphenyl)-2-hydroxy-propionsäure in 150 mL MeOH wurden tropfenweise 2.0 mL (27.4 mmol) SOCl₂ zugegeben und das Reaktionsgemisch 1 h bei RT nachgerührt. Die Reaktionslösung wurde i. vac. eingeengt und der Rückstand chromatographisch (Kieselgel, Cyc/EtOAc 3:1) gereinigt
- Ausbeute:: 5.7 g (78% der Theorie)
- ESI-MS:: (M+NH₄)⁺ = 332
- Retentionszeit (HPLC-MS):: 9.1 min (Methode A)

### 1f) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxycarbonyl-ethylester

Unter Stickstoffatmosphäre wurde zu einer Lösung von 1.17 g (9.58 mmol) 4-Dimethylaminopyridin in 50 mL Pyridin 1.93 g (9.58 mmol) Chlorameisensäure-4-nitraphenylester gegeben, 1.5 h bei RT gerührt, mit 3,0 g (9,58 mmol) (R)-3-(4-Benzyloxy-3,5-dimethylphenyl)-2-hydroxy-propionsäuremethylester versetzt und 20 min bei RT gerührt. Anschließend wurden 2.35 g (9.58 mmol) 3-Piperidin-4-yl-1,3,4,5-tetrahydro-1,3-benzdi-azepin-2-on zugegeben und 20 h bei RT gerührt. Das Reaktionsgemisch wurde i. vac. eingeengt, der Rückstand in EtOAc aufgenommen, die organische Phase mit 10% KHSO₄ und gesättigter NaHCO₃-Lösung gewaschen und über Na₂SO₄ getrocknet. Nach Entfernen des Trocken- und Lösungsmittels wurde der Rückstand chromatographisch (Kieselgel, Gradient Cyc/EtOAc 1:1 zu 1:2) gereinigt.
- Ausbeute:: 3.21 g (57% der Theorie)
- ESI-MS:: (M+H)⁺ = 586
- Retentionszeit (HPLG-MS):: 10.4 min (Methode A)

### 1g) 4-(2-Oxo-1,2,4,5-tetrahydro-1,3-benzdiazepin-3-yl)-piperidin-1-carbonsaure-(R)-2-(4-benzyloxy-3,5-dimethyl-phenyl)-1-carboxy-ethylester

Eine Lösung von 3.21 g (5.48 mmol) 4-(2-Oxo-1,2,4,5-tetrahydro-1,3-benzdiazepin-3-yl)-piperidin-1-carbonsäure-(R)-2-(4-benzyloxy-3,5-dimethyl-phenyl)-1-methoxy-carbonyl-ethylester in 80 mL THF wurde mit einer Lösung von 200 mg (8.35 mmol) LiOH in 40 mL Wasser versetzt und 1 h bei RT gerührt. Das Reaktionsgemisch wurde i. vac. eingeengt, der Rückstand in 100 mL Wasser aufgenommen, mit 2 M HCl sauer gestellt, der Niederschlag abgesaugt und im Vakuumtrockenschrank bei 40°C getrocknet.
- Ausbeute:: quantitativ
- ESI-MS:: (M+H)⁺ = 572
- Retentionszeit (HPLC-MS):: 9.2 min (Methode A)

### 1h) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethylester

3.72 g (6.51 mmol) 4-(2-Oxo-1,2,4,5-tetrahydro-1,3-benzdiazepin-3-yl)-piparidin-1-carbonsäure-(*R*)-2-(4-benzyloxy-3,5-dimethyl-phenyl)-1-carboxy-ethylester in 50 mL DCM wurden mit 300 mg 10% Pd/C versetzt und bei RT und 3 bar Wasserstoff bis zum Reaktionsstillstand geschüttelt, Der Katalysator wurde abgesaugt und das Lösungsmittel i. vac. eingeengt. Der Rückstand wurde mit DIPE verrieben und abgesaugt.
- Ausbeute:: 2.41 g (77% der Theorie)
- ESI-MS:: (M+H)⁺ = 482
- Retentionszeit (HPLC-MS):: 7,0 min (Methode A)

### 1i) 4-(2-Oxo-1,2,4,5-tetrahydro-1,3-benzdiazepin-3-yl)-piperidin-1-carbonsäure-(R)-1-heptylcarbamoyl-2-(4-hydroxy-3,5-dimethyl-phenyl)-ethylester

Eine Lösung von 100 mg (0.21 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethyl-ester, 73 mg (0.23 mmol) TBTU und 36 µL (0,18 mmol) Triethylamin in 1 mL DMF wurde 1 h bei RT gerührt. Dann erfolgte die Zugabe von 26 mg (0.23 mmol) *n*-Heptylamin und das Reaktionsgemisch wurde 5 h bei RT gerührt. Die Reaktionslösung wurde Ober einen Spritzenfilter filtriert und ohne weitere Aufarbeitung direkt via HPLC gereinigt. Die das Produkt enthaltenden Fraktionen wurden vereinigt und lyophilisiert.
- Ausbeute:: 48 mg (40 % der Theorie)
- ESI-MS:: (M-H)⁻ = 577
- Retentionszeit (NPLC-MS):: 4.6 min (Methode B)

Analog wurden folgende Verbindungen aus jeweils 100 mg (Beispiele 1.1 bis 1.5) bzw. aus jeweils 80 mg (Beispiel 1,6 bis 1.8) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester und der entsprechenden Menge an Amin hergestellt:

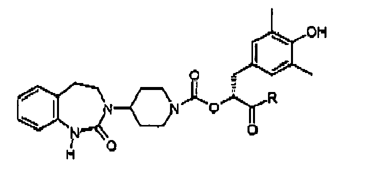

| **Beispiel** | **R** | **Ausbeute (%)** | **Massenspektrum** | **Retentionszeit HPLC (Methode)** |
|---|---|---|---|---|
| 1.1 | 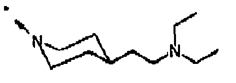 | 27 | 648 | 3.1 min |
| | | | [M+H]⁺ | (B) |
| 1.2 | 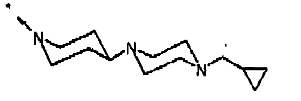 | 13 | 687 | 3.0 min |
| | | | [M+H]⁺ | (B) |
| | | | | |
| 1.3 | 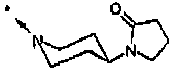 | 52 | 631 | 3.6 min |
| | | | [M+H]⁺ | (B) |
| 1.4 | 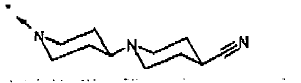 | 19 | 657 | 3.1 min |
| | | | [M+H]⁺ | (B) |
| | | | | |
| 1.5 | 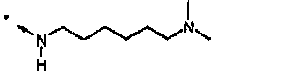 | 46 | 608 | 3.1 min |
| | | | [M+H]⁺ | (B) |
| 1.6 | 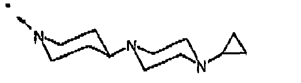 | 44 | 673 | 2,6 min |
| | | | [M+H]⁺ | (B) |
| | | | | |
| 1.7 | 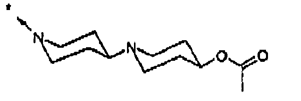 | 42 | 690 | 2,7 min |
| | | | [M+H]⁺ | (B) |
| 1.8 | 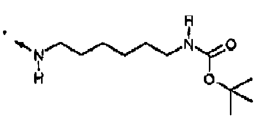 | 59 | 680 | 4.4 min |
| | | | [M+H]⁺ | (B) |

### Beispiel 1.9

### 4-(2-Oxo-1,2,4,5-tetrahydro-1,3-benzdiazepin-3-yl)-piperidin-1-carbonsäure-(R)-1-(6-amino-hexylearbamoyl)-2-(4-hydroxy-3,5-dimethyl-phenyl)-ethylester

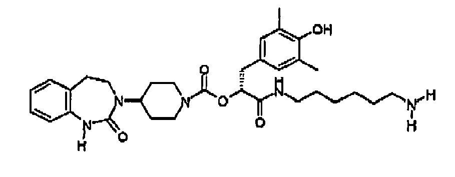

Eine Lösung von 55 mg (0.08 mmol) 4-(2-Oxo-1,2,4,5-tetrahydro-1,3-benzdiazepin-3-yl)-piperidin-1-carbonsäure-(*R*)-1-(6-*tert*-butoxycarbonylamino-hexylcarbamoyl)-2-(4-hydroxy-3,5-dimethyl-phenyl)-ethylester (Beispiel 1.8) in 1 mL 1 M HCl wurde bei RT über Nacht gerührt. Die Reaktionslösung wurde lyophilisiert und der Rückstand via HPLC gereinigt. Die das Produkt enthaltenden Fraktionen wurden vereinigt und erneut lyophilisiert.
- Ausbeute:: 4 mg (8 % der Theorie)
- ESI-MS:: (M+H)⁺ = 580
- Retentionszeit (HPLC-MS):: 2.6 min (Methode B)

### Beispiel 1.10

### 4-(2-Oxo-1,2,4,5-tetrahydro-1,3-benzodiazepin-3-yl)-piperidin-1-carbonsäure-(R)-1-(4-hydroxy-3,5-dimethyl-benzyl)2-oxo-2-(3,4,5,6-tetrahydro-2H-4,4'-bipyridinyl-1-yl)-ethylester

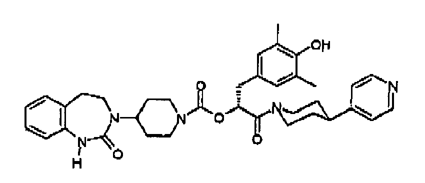

Zu einer Lösung von 182 mg (0.38 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethyl-ester, 133 mg (0.42 mmol) TBTU und 66 µL (0.47 mmol) Triethylamin in 10 mL THF und 2 mL DMF wurden bei RT 67 mg (0.42 mmol) 1,2,3,4,5,6-Mexahydro-[4,4']bipyridinyl gegeben und das Reaktionsgemisch 2 h gerührt, Man versetzte mit 20 mL EtOAc, wusch die organische Phase mit 20 mL halbgesättigter NaHCO₃-Lösung und trocknete diese über Na₂SO₄. Nach Entfernen des Trocken- und Lösungsmittels wurde der Rückstand via HPLC gereinigt. Die das Produkt enthaltenden Fraktionen wurden vereinigt, eingeengt, der Rückstand mit halbgesättigter NaHCO₃-Lösung alkalisch gestellt, mit 50 mL EtOAc extrahiert und die organische Phase über Na₂SO₄ getrocknet. Nach Entfernen des Trocken- und Lösungsmittels wurde der Rückstand mit DIPE verrührt, abgesaugt und getrocknet.
- Ausbeute:: 145 mg (61 % der Theorie)
- ESI-MS:: (M+H)⁺ = 626
- Retentionszeit (HPLC-MS):: 3.1 min (Methode B)

### Beispiel 1.11

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-piperazin-1-yl-ethylester

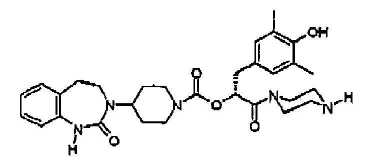

### 1.11a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-(4-benzyl-piperazin-1-yl)-2-oxo-ethylester

Eine Lösung von 1.54 g (2.69 mmol) 4-(2-Oxo-1,2,4,5-tetrahydro-1,3-benzdiazepin-3-yl)-piporidin-1-carbonsäure-(*R*)-2-(4-benzyloxy-3,5-dimethyl-phenyl)-1-carboxy-ethylester (Beispiel 1g), 952 mg (2,96 mmol) TBTU und 0.47 mL (3.37 mmol) Triethylamin in 10 mL THF und 2 mL DMF wurde bei RT 1 h gerührt. Dann erfolgte die Zugabe von 0.52 mL (2.96 mmol) 1-Benzyl-piperazin und das Reaktionsgemisch wurde weitere 14 h bei RT gerührt. Man versetzte mit 30 mL EtOAc, wusch die organische Phase mit halbgesättigter NaHCO₃-Lösung und trocknete diese über Na₂SO₄. Nach Entfernen des Trocken- und Lösungsmittels wurde der Rückstand chromatographisch (Kieselgel. EtOAc/Cyc 95:5) gereinigt, die das Produkt enthaltenden Fraktionen vereinigt, eingeengt, der Rückstand mit DIPE verrieben, abgesaugt und getrocknet.
- Ausbeute:: 1.62 g (82 % der Theorie)
- ESI-M8:: (M+H)⁺ = 730
- Retentionszeit (HPLC-MS):: 4.3 min (Methode B)

### 1.11b) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-piperazin-1-yl-ethylester

1.62 g (2.22 mmol) 4-(2-Oxo-1,2,4,5-tetrahydro-1,3-benzdiazepin-3-yl)-piperidin-1-carbonsäure-(*R*)-1-(4-benzyloxy-3,5-dimathyl-benzyl)-2-(4-benzyl-piperazin-1-yl)-2-oxo-ethyl-ester in 40 mL MeOH wurden mit 150 mg 10% Pd/C versetzt und bei RT und 3 bar Wasserstoff bis zum Reaktionsstilistand hydriert. Der Katalysator wurde abgesaugt und das Lösungsmittel i. vac. eingeengt. Der Rückstand wurde chromatographisch (Kieselgel, EtOAc/MeOH/NH₃ 85:13.5;1,5) gereinigt.
- Ausbeute:: 1.03 g (85% der Theorie)
- ESI-MS:: (M+H)⁺ = 550
- Retentionszeit (HPLC-MS):: 2.7 min (Methode B)

### Beispiel 1.12

### 4-(2-Oxo-1,2,4,5-tetrahydro-1,3-benzdiazepin-3-yl)-piperidin-1-carbonsäure-(R)-2-(4-cyclopropylmethyl-piperazin-1-yl)-1-(4-hydroxy-3,5-dimethyl-benzyl)-2-oxo-ethylester

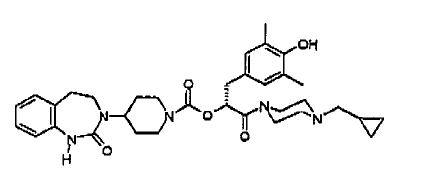

Eine Lösung von 100 mg (0.18 mmol) 4-(2-Oxo-1,2,4,5-tetrahydro-1,3-benzdiazepin-3-yl)-piperidin-1-carbonsäure-(*R*)-1-(4-hydroxy-3,5-dimethyl-benzyl)-2-oxo-2-piperazin-1-yl-ethylester. 20 µL (0.37 mmol) AcOH und 41,6 µL (0.55 mmol) Cyclopropancarbaldehyd in 2 mL THF/MeOH (2:1) wurde über Nacht bei RT gerührt. Nach Abkühlen auf 0°C wurde mit 24 mg (0.36 mmol) NaBH₃CN versetzt, weitere 4 h bei dieser Temperatur und über Nacht bei RT gerührt. Man entfernte die Lösungsmittel, nahm den Rückstand in 1 mL DMF auf und reinigte via HPLC. Die das Produkt enthaltenden Fraktionen wurden vereinigt und lyophilisiert,
- Ausbeute:: 45 mg (41 % der Theorie)
- ESI-MS:: (M+H)⁺ = 604
- Retentionszeit (HPLC-MS):: 3.1 min (Methode B)

Analog wurden folgende Verbindungen aus jeweils 100 mg 4-(2-Oxo-1,2,4.5-tetrahydro-1,3-benzdiazepin-3-yl)-piperidin-1-carbonsäure-(*R*)-1-(4-hydroxy-3,5-dimathyl-benzyl)-2-oxo-2-piperazin-1-yl-ethylester und der entsprechenden Menge an Aldehyd bzw. Keton hergestellt:

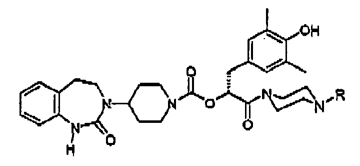

| Beispiel | R | Ausbeute (%) | Massenspektrum | Retentionszeit HPLC (Methode) |
|---|---|---|---|---|
| 1.13 | 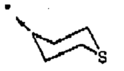 | 27 | 650 | 3.2 min |
| | | | [M+H]⁺ | (B) |
| 1.14 | 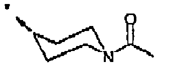 | 58 | 675 | 2.9 min |
| | | | [M+H]⁺ | (B) |
| 1.15 | 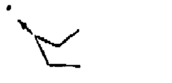 | 17 | 620 | 3.3 min |
| | | | [M+H]⁺ | (B) |
| 1.16 | 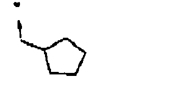 | 57 | 632 | 3.3 min |
| | | | [M+H]⁺ | (B) |
| | | | | |
| 1.17 | 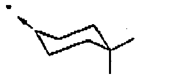 | 46 | 660 | 3,6 min |
| | | | [M+H]⁺ | (B) |

### Beispiel 1.18

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(1'-oxy-3,4,5,6-tetrahydro-2H-4,4'-bipyridinyl-1-yl)-ethylester

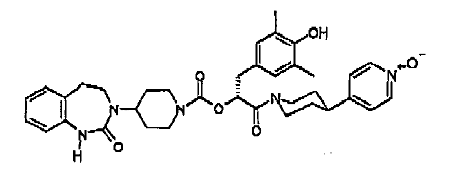

Zu einer auf 0°C gekühlten Lösung von 253 mg (0.40 mmol) 4-(2-Oxc-1,2,4,5-tetrahydro-1,3-benzodiazepin-3-yl)-piperidin-1-carbonsäure-(*R*)-1-(4-hydroxy-3,5-dimethyl-benzyl)-2-oxo-2-(3,4,5,6-tetrahydro-2*H*-4,4'-bipyridinyl-1-yl)-ethylester (Beispiel 1.10) in 10 mL Chloroform wurden 77 mg (0.46 mmol) MCPBA zugegeben und das Reaktionsgemisch 2 h gerührt, wobei dieses sich dabei auf RT erwärmte. Man engte i.vac. ein, nahm den Rückstand in wenig DMF auf und reinigte via HPLC. Die das Produkt enthaltenden Fraktionen wurden vereinigt und lyophilisiert.
- Ausbeute:: 107 mg (41% der Theorie)
- ESI-MS;: (M+H)⁺ = 642

- Retentionszeit (HPLC-MS):: 3.4 min (Methode B)

### Beispiel 1.19

### 4-(2-Oxo-1,2,4,5-tetrahydro-1,3-benzdiazepin-3-yl)-piperidin-1-carbonsaure-(R)-1-(4-hydroxy-3,5-dimethyl-benzyl)-2-(1'-methyl-1'-oxy-4,4-bipiperidinyl-1-yl)-2-oxo-ethylester

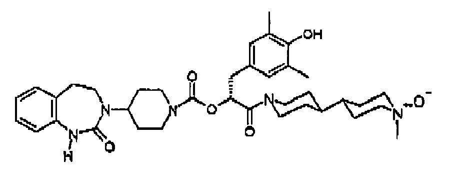

### 1.19a) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(1'-methyl-4,4'-bipiperidinyl-1-yl)-2-oxo-ethylester

Hergestellt analog Beispiel 1i aus 150 mg (0.31 mmol) 4-(2-Oxo-1,2,4,5-tetrahydro-1,3-benzdiazepin-3-yl)-piperidin-1-carbonsäure-(R)-2-(4-hydroxy-3,5-dimethyl-phenyl)-1-carboxy-ethyl-ester und 62 mg (0.34 mmol) 1-Methyl-[4,4']bipiperidinyl.
- Ausbeute:: 42 mg (21 % der Theorie)
- ESI-MS:: (M+H)⁺ = 646
- Retentionszeit (HPLC-MS):: 3.1 min (Methode B)

### 1.19b) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(1'-methyl-1'-oxy-4,4'-bipiperidinyl-1-yl)-2-oxo-ethylester

Hergestellt analog Beispiel 1.18 aus 40 mg (0.06 mmol) 4-(2-axo-1,2,4,5-tetrahydro-1,3-benzdiazepin-3-yl)-piperidin-1-carbonsäure-(*R*)-1-(4-hydroxy-3,5-dimethyl-benzyl)-2-(1'-methyl-4,4'-bipiperidinyl-1-yl)-2-oxo-ethylester und 17 mg (0.07 mmol) MCPBA.
- Ausbeute;: 17 mg (41 % der Theorie)
- ESI-MS:: (M+H)⁺ = 662
- Retentionszeit (HPLC-MS):: 3.2 min (Methode B)

### Beispiel 1.20

### 4-(2-Oxo-1,2,4,5-tetrahydro-1,3-benzdiazepin-3-yl)-pipehdin-1-carbonsäure-(R)-1-(4-hydroxy-3,5-dimethyl-benzyl)-2-Oxo-2-[4-(4-oxy-morpholin-4-yl)-piperidin-1-yl]-ethylester

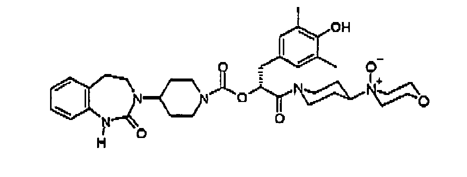

### 1.20a) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-morpholin-4-yl-piperidin-1-yl)-2-oxo-ethylester

Hergestellt analog Beispiel 1i aus 70 mg (0.15 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester und 25 mg (0.15 mmol) 4-Piperidin-4-yl-morpholin.
- Ausbeute:: 35 mg (38 % der Theorie)
- ESI-MS:: (M+H)⁺ = 634
- Retentionszeit (HPLC-MS):: 5.6 min (Methode A)

### 1.20b) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-[(4-(4-morpholin-4-yl)-piperidin-1-yl]-ethylester

Hergestellt analog Beispiel 1.18 aus 80 mg (0.13 mmol) 4-(2-Oxo-1,2,4,5-tetrahydro-1,3-benzdiazepin-3-yl)-piperidin-1-carbonsäure-(*R*)-1-(4-hydroxy-3,5-dimethy)-benzyl)-2-(4-morpholin-4-yl-piperidin-1-yl)-2-oxo-ethylester und 24 mg (0.14 mmol) MCPBA.
- Ausbeute:: 36 mg (44 % der Theorie)
- ESI-MS:: (M+H)⁺ = 650
- Retentionszeit (HPLG-MS):: 4.1 min (Methode C)

### Beispiel 1.21

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-methoxy-1,4'-bipiperidinyl-1'-yl)-2-oxo-ethylester

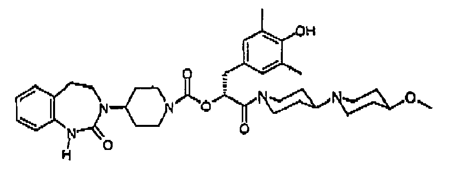

Hergestellt analog Beispiel 1i aus 80 mg (0.17 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethytester, 77 µL (0.55 mmol) Triethylamin und 46 mg (0.17 mmol) 4-Methoxy-[1,4']bipiperidinyl (eingesetzt als Bis-Hydrochlorid-Salz).
- Ausbeute:: 74 mg (67 % der Theorie)
- ESI-MS:: (M+H)⁺ = 662
- Retentionszeit (HPLC-MS):: 6.1 min (Methode A)

### Beispiel 1.22

### 4-(2-Oxo-1,2,4,5-tetrahydro-1,3-benzodiazepin-3-yl)-piperidin-1-carbonsäure-(R)-1-(4-hydroxy-3,5-dimethyl-benzyl)-2-oxo-2-[4-(1-oxo-1λ⁴-thiomorpholin-4-yl)-piperidin-1-yl]-ethylester

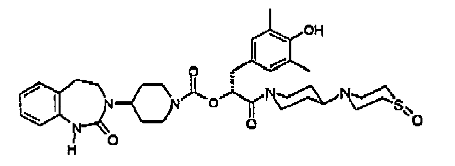

Hergestellt analog Beispiel 1.21 aus 80 mg (0.17 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester. 77 µL (0.55 mmol) Triethylamin und 47 mg (0.17 mmol) 4-Piperidin-4-yl-thiomorpholin-1-oxid (eingesetzt als Bis-Hydrochlorid-Salz).
- Ausbeute:: 45 mg (41 % der Theorie)
- ESI-MS:: (M+H)⁺ = 666
- Retentionszeit (HPLC-MS):: 5.7 min (Methode A)

### Beispiel 1.23

### 4-(2-Oxo-1,2,4,5-tetrahydro-1,3-benzdiazepin-3-yl)-piperidin-1-carbonsäure-(R)-2-[4-(1,1-dioxo-hexahydro-1λ⁶-thiopyran-4-yl)-piperazin-1-yl]-1-(4-hydroxy-3,5-dimethyl-benzyl)-2-oxo-ethylester

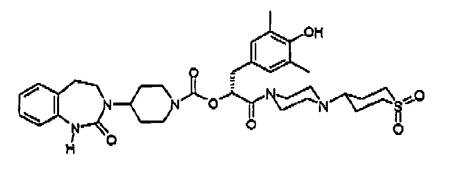

### 1.23a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-[4-(1,1-dioxo-hexahydro-1λ⁶-thiopyran-4-yl)-piperazin-1-yl]-2-oxo-ethylester

Hergestellt analog Beispiel 1i aus 150 mg (0.26 mmol) 4-(2-Oxo-1,2,4,5-tetrahydro-1,3-benzdiazepin-3-yl)-piperidin-1-carbonsäure-(*R*)-2-(4-benzyloxy-3,6-dimethyl-phenyl)-1-carboxy-ethylester (Beispiel 1g) und 57 mg (0.26 mmol) 1-(1,1-Dioxo-hexahydro-1λ⁶-thiopyran-4-yl)-piperazin.
- Ausbeute;: 49 mg (24% der Theorie)
- ESI-MS:: (M+H)⁺ = 772
- Retentionszeit (HPLC-MS):: 3.9 min (Methode B)

### 1.23b) 4-(2-Oxo-1,2,4,5-tetrahydro-1,3-benzdiazepin-3-yl)-piperidin-1-carbonsäure-(R)-2-[4-(1,1-dioxo-hexahydro-1λ⁶-thiopyran-4-yl)-piperazin-1-yl]-1-(4-hydroxy-3,5-dimethyl-benzyl)-2-oxo-ethylester

Eine Lösung von 45 mg (0.06 mmol) 4-(2-Oxo-1,2,4,5-tetrahydro-1.3-benzdiazepin-3-yl)-piperidin-1-carbonsäure-(*R*)-1-(4-benzyloxy-3.5-dimethyl-benzyl)-2-[4-(1,1-dioxo-hexa-hydro-1λ⁶-thiopyran-4-yl)-piperazin-1-yl]-2-oxo-ethylester in 10 mL MeOH wurde mit 10 mg 10% Pd/C versetzt und bei RT und 3 bar Wasserstoffdruck bis zum Reaktionsstülstand hydriert, Der Katalysatorwurde abgesaugt und der Rückstand via HPLC gereinigt. Die das Produkt enthaltenden Fraktionen wurden vereinigt und lyophilisiert.
- Ausbeute:: 23 mg (57% der Theorie)
- ESI-MS:: (M+H)⁺ = 682
- Retentionszeit (HPLC-MS):: 3,1 min (Methode B)

### Beispiel 1.24

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-[4-methyl-4-(4-methyl-piperazin-1-yl)-piperidin-1-yl]-2-oxo-ethylester

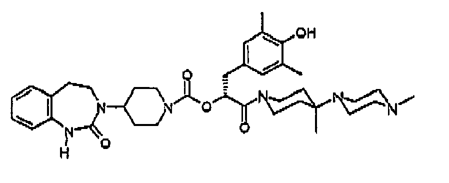

Hergestellt analog Beispiel 1i aus 80 mg (0.17 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester (Beispiel 1h) und 36 mg (0.18 mmol) 1-Methyl-4-(4-methyl-piperidin-4-yl)-piperazin.
- Ausbeute:: 36 mg (32% der Theorie)
- ESI-MS:: (M+H)⁺ = 661
- Retentionszeit (HPLC-MS):: 2.5 min (Methode B)

### Beispiel 1.25

### 4-(2-Oxo-1,2,4,5-tetrahydro-1,3-benzdiazepin-3-yl)-piperidin-1-carbonsäurt-(R)-1-(4-hydroxy-3,5-dimethyl-benzyl)-2-(4-methyl-4-morpholin-4-yl-piperidin-1-yl)-2-oxo-ethylester

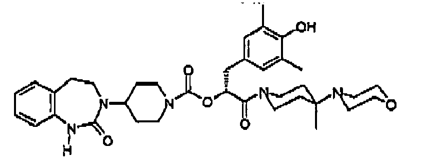

Zu einer Lösung von 90 mg (0,19 mmol) 4-(2-Oxo-1,2,4,5-tetrahydro-1,3-benzdiazepin-3-yl)-piperidin-1-carbonsäure-(*R*)-2-(4-hydroxy-3,5-dimethyl-phenyl)-1-carboxy-ethylester (Beispiel 1h), 70 mg (0.22 mmol) TBTU und 70 µL (0.50 mmol) Triethylamin in 1 mL DMF wurden bei RT 55 mg (0.22 mmol) 4-(4-Methyl-piperidin-4-yl)-morpholin (eingesetzt als Hydrochlorid-Salz, Reinheit 90%) gegeben und das Reaktionsgemisch 20 h gerührt. Die Reaktionslösung wurde auf gesättigte NaHCO₃-Lösung gegossen, der entstandene Niederschlag abfiltriert und getrocknet. Weitere Reinigung erfolgte via HPLC, die das Produkt enthaltenden Fraktionen wurden vereinigt und lyophilisiert.
- Ausbeute:: 32 mg (26% der Theorie)
- ESI-MS:: (M+H)⁺ = 648
- Retentionszeit (HPLC-MS):: 3.0 min (Methode B)

### Beispiel 1.26

### 4-(2-Oxo-1,2,4,5-tetrahydro-1,3-benzdiazepin-3-yl)-piperidin-1-carbonsäure-(R)-1-(4-formyloxy-3,5-dimethyl-benzyl)-2-(4-morpholin-4-yl-piperidin-1-yl)-2-oxo-ethylester

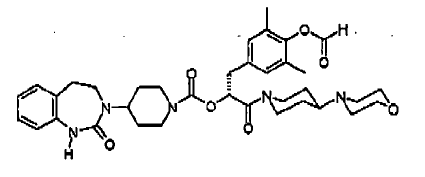

Eine Lösung aus 100 µL Essigsäureanhydrid und 1 mL Ameisensäure in 3 mL DCM wurde 2 h bei RT gerührt (Bildung des gemischten Anhydrides), Dann erfolgte die Zugabe von 100 mg (0.16 mmol) 4-(2-Oxo-1,2,4,5-tetrahydro-1,3-benzdiazepin-3-yl)-piperidin-1-carbonsäure-(*R*)-1-(4-hydroxy-3,5-dimethyl-benzyl)-2-(4-morpholin-4-yl-piperidin-1-yl)-2-oxo-ethylester (Beispiel 1.20a) und das Reaktionsgemisch wurde 2 h bei RT gerührt. Die Reaktionslösung wurde eingeengt und der Rückstand erneut zu einer Lösung des gemischten Anhydrides gegeben und über Nacht bei RT gerührt. Man engte i.vac. ein und reinigte den Rückstand via HPLC. Die das Produkt enthaltenden Fraktionen wurden vereinigt und lyophilisiert.
- Ausbeute;: 77 mg (69% der Theorie)
- ESI-MS:: (M+H)⁺ = 662
- Retentionszeit (HPLC-MS):: 3.1 min (Methode B)

### Beispiel 2

### 4-(5-Oxo-3-phenyl-4,5-dihydro-1,2,4-triazol-1-yl)-piperidin-1-carbonsäure-(R)-1-(4-hydroxy-3,5-dimethyl-benzyl)-2-[4-(4-methyl-plperazin-1-yl)-pipendin-1-yl]-2-oxo-ethylester

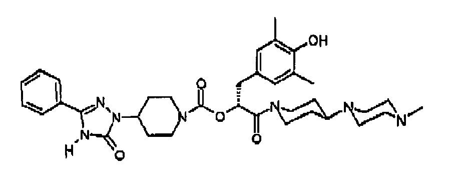

### 2a) 4-(5-Oxo-3-phenyl-4,5-dihydro-1,2,4-triazol-1-yl)-piperidin-1-carbonsäure-(R)-2-(4-benzyloxy-3,5-dimethyl-phenyl)-1-methoxycarbonyl-ethylester

Hergestellt analog Beispiel 1f aus 5.0 g (15.9 mmol) (*R*)-3-(4-Benzyloxy-3,5-dimethyl-phenyl)-2-hydroxy-propionsäuremethylester (Beispiel 1 e) und 5.98 g (15.9 mmol) 3-5-Phenyl-2-piperidin-4-yl-2,4-dihydro-1,2,4-triazol-3-on (Reinheit 65%).
- Ausbeute:: 4.96 g (53% der Theorie)
- ESI-MS:: (M+H)⁺ = 585
- Retentionszeit (HPLC-MS):: 5,0 min (Methode B)

### 2b) 4-(5-Oxo-3-phenyl-4,5-dihydro-1,2,4-triazol-1-yl)-piperidin-1-carbonsäure-(R)-2-(4-benzyloxy-3,5-dimethyl-phenyl)-1-carboxy-ethylester

Zu einer Lösung von 4.96 g (8.48 mmol) 4-(5-Oxo-3-phenyl-4,5-dihydro-1,2,4-triazol-1-yl)-piperidin-1-carbonsäure-(*R*)-2-(4-benzyloxy-3,5-dimethyl-phenyl)-1-methoxy-carbonyl-ethylester in 50 mL THF wurde eine Lösung von 310 mg (12,93 mmol) LiOH in 30 mL Wasser gegeben und das Reaktionsgemisch 7 h bei RT gerührt. Die Lösung wurde über Nacht bei -18°C gelagert und nach Erwärmen auf RT zur Vervollständigung der Reaktion erneut mit 310 mg LiOH versetzt. Nach 1 h wurde die Reaktionslösung i.vac. eingeengt, der Rückstand in 150 mL Wasser aufgenommen und mit 1 M HCl sauer gestellt, Der Niederschlag wurde abfiltriert und bei 40°C getrocknet.
- Ausbeute:: 4.75 g (98% der Theorie)
- ESI-MS:: (M+H)⁺ = 571
- Retentionszeit (HPLC-MS):: 4.3 min (Methode B)

### 2c) 4-(5-Oxo-3-phenyl-4,5-dihydro-1,2,4-triazol-1-yl)-Piperidin-1-carbonsäure-(R),-1-carboxy-2-(4-hydroxy-3,5-dimethyl-phenyl)-ethylester

Eine Lösung von 2.50 g (4.38 mmol) 4-(5-Oxo-3-phenyl-4,5-dihydro-1,2,4-triazo!-1-yl)-piperidin-1-carbonsäure-(*R*)-2-(4-benzyloxy-3,5-dimethyl-phenyl)-1-carboxy-ethylester in 50 mL DCM wurde mit 250 mg 10% Pd/C versetzt und bei RT und 3 bar Wasserstoffdruck 4.5 h hydriert. Zur Vervollständigung der Reaktion wurde erneut mit 250 mg Katalysator versetzt, 12 h bei 40°C hydriert, mit 25 mL THF und 250 mg Katalysator versetzt und weitere 12 h bei 40°C hydriert. Der Katalysator wurde abgesaugt und das Filtrat i.vac. eingeengt. Der Rückstand wurde mit DIPE verrührt, abgesaugt und getrocknet.
- Ausbeute:: 1.87 g (89% der Theorie)
- ESI-MS:: (M-H)⁻ = 479
- Retentionszeit (HPLC-MS):: 3.5 min (Methode B)

### 2d) 4-(5-Oxo-3-phenyl-4,5-dihydro-1,2,4-triazol-1-yl)-piperidin-1-carbonsäure-(R)-1-(4-hydroxy-3,5-dimethyl-benzyl)-2-[4-(4-methyl-piyerazin-1-yl)-piperidin-1-yl]-2-oxo-ethylester

Eine Lösung von 100 mg (0.21 mmol) 4-(5-Oxo-3-phenyl-4,5-dihydro-1,2,4-triazol-1-yl)-piperidin-1-carbonsäure-(*R*)-1-carboxy-2-(4-hydroxy-3,5-dimethyl-phenyl)-ethylester, 74 mg (0.23 mmol) TBTU und 35 µL (0.26 mmol) Triethylamin in 1 mL DMF wurde bei RT 1 h gerührt. Dann erfolgte die Zugabe von 39 mg (0.21 mmol) 1-Methyl-4-piperidin-4-yl-piperazin und das Reaktionsgemisch wurde weitere 5 h bei RT gerührt. Die Reaktionslösung wurde ohne weitere Aufarbeitung via HPLC gereinigt, Die das Produkt enthaltenden Fraktionen wurden vereinigt und lyophilisiert.
- Ausbeute:: 40 mg (30 % der Theorie)
- ESI-MS:: (M+H)⁺ = 646
- Retentionszeit (HPLC-MS):: 2.7 min (Methode B)

Analog wurden folgende Verbindungen aus jeweils 100 mg (Beispiele 2.1 bis 2.7) bzw. aus 150 mg (Beispiel 2.8) 4-(5-Oxo-3-phenyl-4,5-dihydra-1,2,4-triazol-1-yl)-piperidin-1-carbonsäure-(*R*)-1-carboxy-2-(4-hydroxy-3,5-dimethyl-phenyl)-ethylester und der entsprechenden Menge an Amin hergestellt:

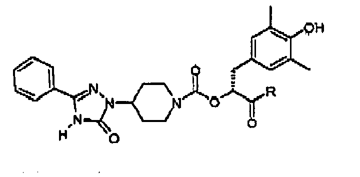

| **Beispiel** | **R** | **Ausbeute (%)** | **Massenspektrum** | **Retentionszeit HPLC (Methode)** |
|---|---|---|---|---|
| **2.1** | 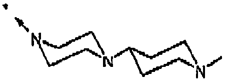 | 22 | 646 | 2.5 min |
| | | | [M+H]⁺ | (B) |
| | | | | |
| **2.2** | 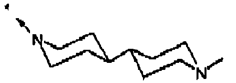 | 28 | 645 | 3.0 min |
| | | | [M+H]⁺ | (B) |
| | | | | |
| **2.3** | 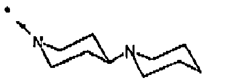 | 29 | 631 | 3.0 min |
| | | | [M+H]⁺ | (B) |
| | | | | |
| **2.4** | 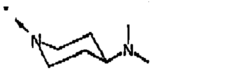 | 25 | 591 | 2,8 min |
| | | | [M+H]⁺ | (B) |
| **2.5** | 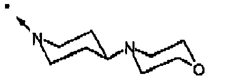 | 28 | 633 | 2.9 min |
| | | | [M+H]⁺ | (B) |
| | | | | |
| **2.6** | 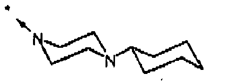 | 24 | 631 | 3.1 min |
| | | | [M+H]⁺ | (B) |
| | | | | |
| **2.7** | 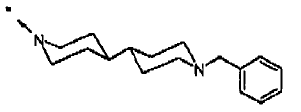 | 23 | 721 | 3.4 min |
| | | | [M+H]⁺ | (B) |

Analog wurden folgende Verbindungen aus jeweils 250 mg 4-(5-Oxo-3-phenyl-4,5-dihydro-1,2,4-triazol-1-yl)-piperidin-1-carbonsäure-(*R*)-2-(4-benzyloxy-3,5-dimethylphenyl)-1-carbvxy-ethylester (Beispiel 2b) und der entsprechenden Menge an Amin hergestellt;

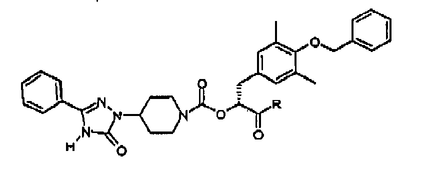

| **Beispiel** | **R** | **Ausbeute (%)** | **Massenspektrum** | **Retentionszeit HPLC (Methode)** |
|---|---|---|---|---|
| **2.8** | 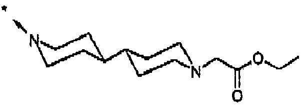 | 17. | 807 | 3.2min |
| | | | [M+H]⁺ | (B) |
| **2.9** | 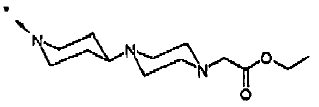 | 13 | 808 | 3.1 min |
| | | | [M+H]⁺ | (B) |

### Beispiel 2.10

### 4-(5-Oxo-3-phenyl-4,5-dihydro-1,2,4-triazol-1-yl)-pipendin-1-carbonsäure-(R)-2-(4,4'-bipiperidinyl-1-yl)-1-(4-hydroxy-3,5-dimethyl-benzyl)-2-oxo-ethylester

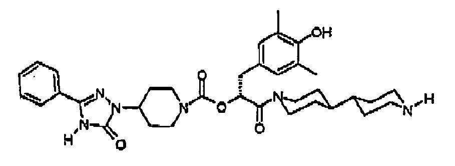

Eine Lösung von 51 mg (0.07 mmol) 4-(5-Oxo-3-phenyl-4,5-dihydro-1,2,4-triazol-1-yl)-piperidin-1-carbonsäure-(*R*)-2-(1'-benzyl-4,4'-pipiperidinyl-1-yl)-1-(4-hydroxy-3,5-dimethylbenzyl)-2-oxo-ethylester (Beispiel 2.7) in 10 mL MeOH wurde mit 5 mg 10% Pd/C versetzt und bei RT und 3 bar Wasserstoffdruck bis zum Reaktionsstillstand hydriert. Der Katalysator wurde abgesaugt und der Rückstand via HPLC gereinigt. Die das Produkt enthaltenden Fraktionen wurden vereinigt und lyophilisiert.
- Ausbeute:: 15 mg (45 % der Theorie)
- ESI-MS:: (M-H)⁻ = 629
- Retentionszeit (HPLC-MS):: 2.5 min (Methode B)

### Beispiel 2.11

### 4-(5-Oxo-3-phenyl-4,5-dihydro-1,2,4-triazol-1-yl)-piperidin-1-carbonsäure-(R)-2-(1'-ethoxycarbonylmethyl-4,4'-bipiperidinyl-1-yl)-1-(4-hydroxy-3,5-dimethyl-benzyl)-2-oxo-ethylester

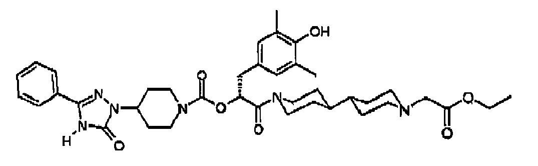

Eine Lösung von 58 mg (0.07 mmol) 4-(5-Oxo-3-phenyl-4,5-dihydro-1,2,4-triazol-1-yl)-piperidine-1-carbonsäure-(*R*)-1-(4-benzyloxy-3,5-dimethyl-benzyl)-2-(1'-ethoxy-carbonyl-methyl-4,4'-bipiperidinyl-1-yl)-2-Oxo-ethylester (Beispiel 2.8) in 5 mL EtOH wurde mit 10 mg 10% Pd/C versetzt und bei RT und 3 bar Wasserstoffdruck bis zum Reaktionsstillstand hydriert. Der Katalysator wurde abgesaugt und der Rückstand via HPLC gereinigt. Die das Produkt enthaltenden Fraktionen wurden vereinigt und lyophilisiert.
- Ausbeute:: 23 mg (45 % der Theorie)
- ESI-MS:: (M+H)⁺ = 717
- Retentionszeit (HPLC-MS):: 3.1 min (Methode B)

### Beispiel 2.12

### 4-(5-Oxo-3-phenyl-4,5-dihydro-1,2,4-triazol-1-yl)-piperidin-1-carbonsäure-(R)-2-(1'-carboxymethyl-4,4'-bipiperidinyl-1-yl)-1-(4-hydroxy-3,5-dimethyl-benzyl)-2-oxo-ethylester

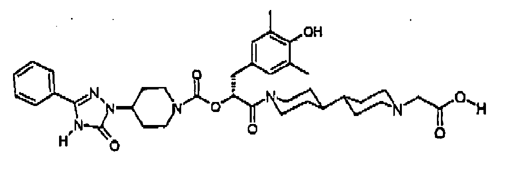

Zu einer Lösung von 21 mg (0.03 mmol) 4-(5-Oxo-3-phenyl-4,5-dihydro-1,2,a-triazol-1-yl)-piperidin-1-carbonsäure-(*R*)-2-(1'-ethoxycarbonylmethyl-4,4'-bipiperidinyl-1-yl)-1-(4-hydroxy-3,5-dimethyl-benzyl)-2-oxo-ethylester in 3 mL THF wurde eine Lösung von 1.2 mg (0.05 mmol) LiOH in 2 mL Wasser gegeben und die Reaktionsmischung 1 h bei RT gerührt. Man engte i.vac. das THF ein, stellte den Rückstand mit 1 M HCl sauer und engte erneut i.vac. ein. Der Rückstand wurde via HPLC gereinigt, die das Produkt enthaltenden Fraktionen wurden vereinigt und lyophilisiert.
- Ausbeute:: 12 mg (58% der Theorie)
- ESI-MS:: (M+H)⁺ = 689
- Retentionszeit (HPLC-MS):: 2,9 min (Methode B)

### Beispiel 2.13

### 4-(5-Oxo-3-phonyl-4,5-dihydro-1,2,4-triazol-1-yl)-piperidin-1-earbonsäure-(R)-2-[4-(4-ethoxycarbonylmethyl-piperazin-1-yl)-pipeddin-1-yl]-1-(4-hydroxy-3,5-dimethyl-benzyl)-2-oxo-ethylester

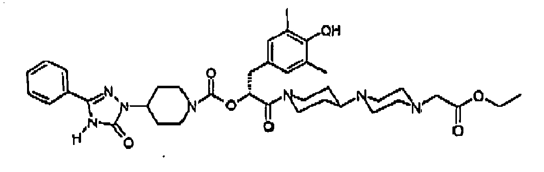

Hergestellt analog Beispiel 2.11 aus 47 mg (0,06 mmol) 4-(5-Oxo-3-phenyl-4,5-dihydro-1,2,4-triazol-1-yl)-piperidin-1-carbonsäure-(*R*)-1-(4-benzyloxy-3,5-dimethyl-benzyl)-2-[4-(4-ethoxycarbonylmethyl-piperazin-1-yl)-piperidin-1-yl]-2-oxo-ethylester (Beispiel 2.9).
- Ausbeute:: 18 mg (43% der Theorie)
- ESI-MS:: (M+H)⁺ = 718
- Retentionszeit (HPLC-MS):: 3.0 min (Methode B)

### Beispiel 2.14

### 4-(5-Oxo-3-phenyl-4,5-dihydro-1,2,4-tdazol-1-yl)-piperidin-1-carbonsäure-(R)-2-[4-(4-carboxymethyl-piperazin-1-yl)-piperidin-1-yl]-1-(4-hydroxy-3,5-dimethyl-benzyl)-2-oxo-ethylester

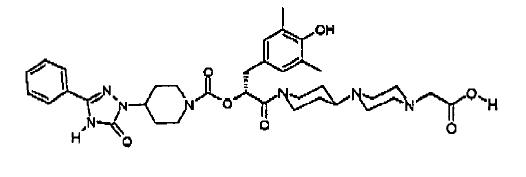

Hergestellt analog Beispiel 2.12 aus 38 mg (0.05 mmol) 4-(5-Oxo-3-phenyl-4,5-dihydro-1,2,4-tdazol-1-yl)-piperidin-1-carbonsäure-(*R*)-2-[4-(4-ethoxycarbonylmethyl-piperazin-1-yl)-piperidin-1-yl]-1-(4-hydroxy-3,5-dimethyl-benzyl)-2-oxo-ethylester und 2.1 mg (0.09 mmol) LiOH.
- Ausbeute:: 20 mg (55% der Theorie)
- ESI-MS:: (M+H)⁺ = 690
- Retentionszeit (HPLC-MS):: 2,7 min (Methode B)

### Beispiel 3

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-{1'-[2-(2-oxo-pyrrolidin-1-yl)-ethoxycarbonyl-methyl]-4,4'-bipiperidinyl-1-yl}-ethylester

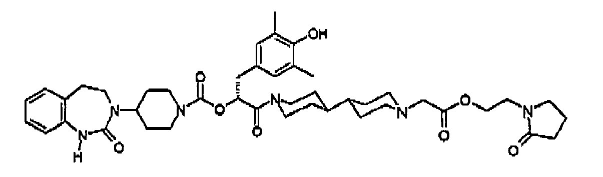

### 3a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-(1'-ethoxycarbonylmethyl-4,4'-bipiperidinyl-1-yl)-2-oxo-ethylester

Zu einer Lösung von 2.00 g (3.50 mmol) 4-(2-Oxo-1,2,4,5-tetrahydro-1,3-benzodiazepin-3-yl)-piperidin-1-carbonsäure-(*R*)-2-(4-benzyloxy-3,5-dimethyl-phenyl)-1-carboxy-ethyl-ester (Beispiel 1g). 1.25 g (3.90 mmol) TBTU und 0.67 mL (3.90 mmol) Ethyldiisopropylamin in 20 mL TFIF und 5 mL DMF wurden bei RT 0,99 g (3.90 mmol) [4,4']Bipiperidinyl-1-yl-essigsäureethylester gegeben und das Reaktionsgemisch über Nacht gerührt.
Man versetzte mit 15% K₂CO₃-Lösung, extrahierte erschöpfend mit EtOAc, wusch die vereinigten organischen Phasen mit gesättigter NaCl-Lösung und trocknete über Na₂SO₄. Nach Entfernen des Trocken- und Lösungsmittels erhielt man das Produkt, welches ohne Reinigung weiter umgesetzt wurde.
- Ausbeute:: 2.80 g (99 % der Theorie)
- ESI-MS:: (M+H)⁺ = 809
- Retentionszeit (HPLC-MS),: 4,1 min (Methode B)

### 3b) 4-(2-Oxo-1,2,4,5-tetrahydro-1,3-benzdiazepin-3-yl)-piperidin-1-carbonsäure-(R)-2-(1'-carboxymethyl-4,4'-bipiperidinyl-1-yl)-1-(4-hydroxy-3,5-dimethyl-benzyl)-2-oxo-ethylester

2.80 g (3.47 mmol) 4-(2-Oxo-1,2,4,5-tetrahydro-1,3-benzdiazepin-3-yl)-piperidin-1-carbonsäure-(*R*)-1-(4-benzyloxy-3,5-dimethyl-benzyl)-2-(1'-ethoxycarbonylmethyl-4,4'-bipiperidinyl-1-yl)-2-oxo-ethylester in 30 mL MeOH wurden mit 300 mg 10% Pd/C versetzt und bei RT und 3 bar Wasserstoff bis zum Reaktionsstillstand hydriert (24h). Der Katalysator wurde abgesaugt und das Lösungsmittel i. vac. eingeengt. Der Rückstand wurde in 30 mL THF gelöst, mit einer Lösung von 127 mg (5.33 mmol) LiOH in 10 mL Wasser versetzt und das Reaktionsgemisch über Nacht bei RT gerührt. Man engte i.vac. ein und reinigte den Rückstand chromatographisch (Kieselgel, Gradient DCM zu DCM/MeOH/NH₃ 70:30:3). Die das Produkt enthaltenden Fraktionen wurden vereinigt, i.vac. eingeengt, der Rückstand mit DIPE verrieben, abgesaugt und getrocknet.
- Ausbeute:: 1.80 g (75% der Theorie)
- ESI-MS:: (M+H)⁺ = 690
- R_{f}=: 0.16 (Kieselgel, DCM/MeOH/Cyc/NH₃ 70:15:15:2)

### 3c) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-{1'-[2-(2-oxo-pymolidin-1-yl)-ethoxycarbonyl-methyl]-4,4'-bipiperidinyl-1-yl}-ethylester

Eine Lösung von 80 mg (0.12 mmol) 4-(2-Oxo-1,2,4,5-tetrahydro-1,3-benzdiazepin-3-yl)-piperidin-1-carbonsäure-(*R*)-2-(1'-carbonxymethyl-4,4'-biperidinyl)-1-(4-hydroxy-3,5-dimethyl-benzyl)-2-oxo-ethylester, 45 mg (0.14 mmol) TBTU und 32 µL (0.23 mmol) Triethylamin in 1,2 mL DMF wurde 1 h bei RT gerührt. Dann erfolgte die Zugabe von 26 mg (0.23 mmol) 1-(2-Hydroxy-ethyl)-pyrrotidin-2-on und das Reaktionsgemisch wurde über Nacht bei RT gerührt. Die Reaktionslösung wurde mit 5 Tropfen Ameisensäure versetzt, über einen Spritzenfilter filtriert und ohne weitere Aufarbeitung direkt via HPLC gereinigt Die das Produkt enthaltenden Fraktionen wurden vereinigt, mit 30 mL EtOAc extrahiert, die organische Phase mit 5% NaHCO₃-Lösung alkalisch gestellt, abgetrennt und Ober Na₂SO₄ getrocknet Nach Entfernen des Trocken- und Lösungsmittels wurde der Rückstand mit DIPE verrieben, abgesaugt und getrocknet.
- Ausbeute:: 26 mg (28 % der Theorie)
- ESI-MS:: (M+H)⁺ = 801

- Retentionszeit (HPLC-MS):: 3.1 min (Methode B)

### Beispiel 3.1

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-[1'-(2-methoxy-ethoxycarbonylmethyl)-4,4'-bipiperidinyl-1-yl]-2-oxo-ethylester

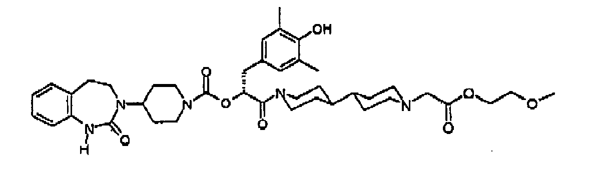

Hergestellt analog Beispiel 3c aus 80 mg (0.12 mmol) 4-(2-Oxo-1,2,4,5-tetrahydro-1,3-benzdiazepin-3-yl)-piperidin-1-carbonsäure-(*R*)-2-(1'-carboxymethy)-4.4'-bipiperidinyl-1-yl)-1-(4-hydroxy-3,5-dimethyl-benzyl)-2-oxo-ethylester und 18 µL (0.23 mmol) 2-Methoxyethanol,
- Ausbeute:: 33 mg (38 % der Theorie)
- ESI-MS:: (M+H)⁺ = 748
- Retentionszeit (HPLC-MS):: 3.2 min (Methode B)

### Beispiel 3.2

### 4-(2-Oxo-1,2,4,5-tetrahydro-1,3-benzdiazepin-3-yl)-piperidin-1-carbonsäure-(R)-2-(1'-hexyloxycarbonylmethyl-4,4'-bipiperidinyl-1-yl)-1-(4-hydroxy-3,5-dimethyl-benzyl)-2-oxo-ethylester

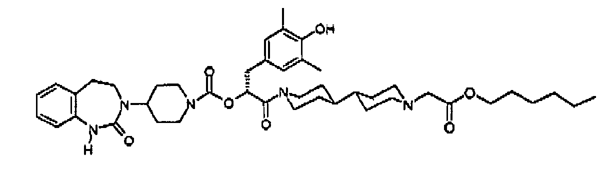

Eine Lösung von 80 mg (0.12 mmol) 4-(2-Oxo-1,2,4,5-tetrahydro-1,3-benzdiazepin-3-yl)-piperidin-1-carbonsäure-(*R*)-2-(1'-carboxymethyl-4,4'-bipiperidinyl-1-yl)-1-(4-hydroxy-3,5-dimethyl-benzyl)-2-oxo-ethylester. 45 mg (0.14 mmol) TBTU und 32 µL (0.23 mmol) Triethylamin in 1.2 mL DMF wurde 1 h bei RT gerührt. Dann erfolgte die Zugabe von 29 µL (0,23 mmol) 1-Hexanol und das Reaktionsgemisch wurde 4 Tage bei RT gerührt. Man engte i.vac. ein, nahm den Rückstand in 30 mL EtOAc auf, wusch die organische Phase mit 20 mL 5% NaHCOs-Lösung und trocknete diese über Na₂SO₄. Nach Entfernen des Trocken- und Lösungsmittels wurde der Rückstand chromatographisch (Kieselgel, Gradient DCM/Isopropanol 95:5 zu DCM/Isopropanal 90:10) gereinigt. Die das Produkt enthaltenden Fraktionen wurden i.vac. eingeengt, der Rückstand mit DIPE verrieben, abgesaugt und getrocknet.
- Ausbeute:: 24 mg (27 % der Theorie)
- ESI-MS:: (M+H)⁺ = 774
- Retentionszeit (HPLC-MS):: 4.0 min (Methode B)

### Beispiel 3.3

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-{1'-[2-(2-oxo-piperidin-1-yl)-ethoxycarbonyl-methyl]-4,4'-bipiperidinyl-1-yl}-ethylester

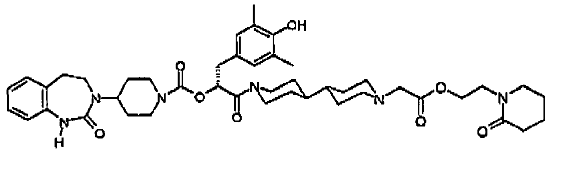

Hergestellt analog Beispiel 3c aus 80 mg (0.12 mmol) 4-(2-Oxo-1,2,4,5-tetrahydro-1,3-benzdiazepin-3-yl)-piperidin-1-carbonsäure-(*R*)-2-(1'-carboxymethyl-4,4'-bipiperidinyl-1-yl)-1-(4-hydroxy-3,5-dimethyl-benzyl)-2-oxo-ethylester und 33 mg (0.23 mmol) 1-(2-Hydroxyethyl)-piperidin-2-on. Nach Entfernen des Trocken- und Lösungsmittels wurde der Rückstand in DCM aufgenommen und erneut chromatographisch (Kieselgel, DCM/EtOH/NH₃ 95:5:0,5) gereinigt. Die das Produkt enthaltenden Fraktionen wurden vereinigt, i.vac. eingeengt, mit DIPE verrieben, abgesaugt und getrocknet.
- Ausbeute;: 23 mg (24 % der Theorie)
- ESI-MS:: (M+H)⁺ = 815
- Retentionszeit (HPLC-MS):: 3.2 min (Methode B)

### Beispiel 3.4

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-[1'-(2-morpholin-4-yl-ethoxycarbonylmethyl)-4,4'-bipiperidinyl-1-yl]-2-oxo-ethylester

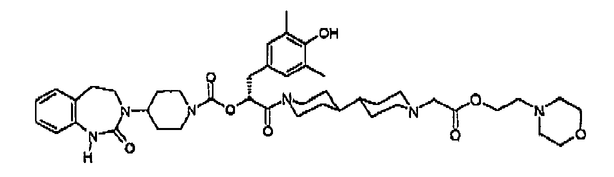

Hergestellt analog Beispiel 3c aus 80 mg (0.12 mmol) 4-(2-Oxo-1,2.4,5-tetrahydro-1,3-benzdiazepin-3-yl)-piper-idin-1-carbonsäure-(*R*)-2-(1'-carboxymethyl-4,4'-blpiperidinyl-1-yl)-1-(4-hydroxy-3,5-dimethyl-benzyl)-2-oxo-ethylester und 17 µL (0.14 mmol) 2-Morpholin-4-yl-ethanol. Nach Reinigung via HPLC wurde das erhaltene Produkt in 10 mL DCM gelöst und mit 5 mL 5% NaHCO₃-Lösung ausgerührt. Die organische Phase wurde abgetrennt und über Na₂SO₄ getrocknet. Nach Entfernen des Trocken- und Lösungsmittel wurde der Rückstand mit Diethylether verrieben, abgesaugt und getrocknet.
- Ausbeute:: 32 mg (34 % der Theorie)
- ESI-MS:: (M+H)⁺ = 803
- Retentionszeit (HPLC-MS):: 2.7 min (Methode B)

### Beispiel 3.5

### 4-(2-Oxo-1,2,4,5-tetrahydro-1,3-benzdiazepin-3-yl)-piperidin-1-carbonsäure-(R)-2-(1'-dimethylcarbamoylmethoxycarbonylmethyl-4,4'-bipiperidinyl-1-yl)-1-(4-hydroxy-3,5-dimethyl-benzyl)-2-oxo-ethylester

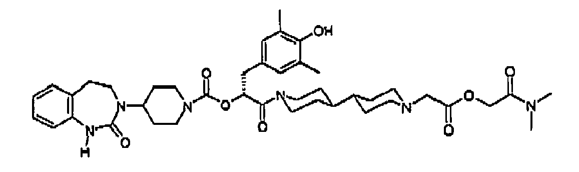

Hergestellt analog Beispiel 3c aus 100 mg (0.15 mmol) 4-(2-Oxo-1,2,4,5-tetrahydro-1,3-benzdiazepin-3-yl)-piperidin-1-carbonsäure-(*R*)-2-(1'-carboxymethyl-4,4'-bipiperidinyl-1-yl)-1-(4-hydroxy-3,5-dimethyl-benzyl)-2-oxo-ethylester und 16 mg (0.16 mmol) 2-Hydroxy-*N,N*-dimethyl-acetamid. Nach Reinigung via HPLC wurden die das Produkt enthaltenden Fraktionen vereinigt und lyophilisiert,
- Ausbeute:: 45 mg (40 % der Theorie)
- ESI-MS:: (M+H)⁺ = 775
- Retentionszeit (HPLC-MS):: 3.1 min (Methode B)

### Beispiel 4

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-{1-[2-(2-oxo-pyrrolidin-1-yl)-ethoxycarbonyl-methyl]-piperidin-4-yl}-piperazin-1-yl)-ethylester

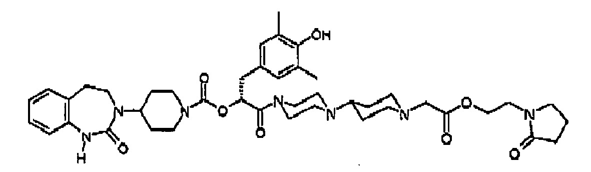

### 4a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-[4-(1-ethoxycarbonylmethyl-pineridin-4-yl)-piperazin-1-yl]-2-oxo-ethylester

Eine Lösung von 2.80 g (4.90 mmol) 4-(2-Oxo-1,2,4,5-tetrahydro-1,3-benzodiazepin-3-yl)-piperidin-1-carbonsäure-(*R*)-2-(4-benzyloxy-3,5-dimethyl-phenyl)-1-carboxy-ethylester (Beispiel 1g), 1.80 g (5.61 mmol) TBTU und 3.10 mL (22.08 mmol) Triethylamin in 30 mL DMF wurde 1 h bei RT gerührt. Dann erfolgte die Zugabe von 2.00 g (5.48 mmol) (4-Piperazin-1-yl-piperidin-1-yl)-essigsäureethylester. Das Reaktionsgemisch wurde 1 h bei RT gerührt, auf 150 mL 15% K₂CO₃-Lösung gegossen und mit 200 mL EtOAc extrahiert, Die organische Phase wurde mit 150 mL 10% Zitronensäure-Lösung extrahiert, die wässrige Phase mit 15% K₂CO₃-Lösung alkalisch gestellt und mit 200 mL EtOAc extrahiert. Die organische Phase wurde i.vac. eingeengt und der Rückstand chromatographisch (Kieselgel. EtOH) gereinigt.
- Ausbeute:: 2.00 g (51 % der Theorie)
- ESI-MS:: (M+H)⁺= 809
- Retentionszeit (HPLC-MS):: 3.9 min (Methode B)

### 4b) 4-(2-Oxo-1,2,4,5-tetrahydro-1,3-benzdiazepin-3-yl)-piperidin-1-carbonsäure-(R)-2-[4-(1-ethoxycarbonylmethyl-piperidin-4-yl)-piperazin-1-yl]-(4-hydroxy-3,6-dimethyl-benzyl)-2-oxo-ethylester

2.00 g (2.47 mmol) 4-(2-Oxo-1,2,4,5-tetrahydro-1,3-benzdiazepin-3-yl)-piperidin-1-carbonsäure-(*R*)-1-(4-benzyloxy-3,5-dimethyl-benzyl)-2-[4-(1-ethoxycarbonylmethyl-piperidin-4-yl)-piperazin-1-yl]-2-oxo-ethylester in 25 mL EtOH wurden mit 200 mg 10% Pd/C versetzt und bei RT und 3 bar Wasserstoff bis zum Reaktionsstillstand hydriert, Der Katalysator wurde abgesaugt und das Lösungsmittel i. vac. eingeengt. Der Rückstand wurde chromatographisch ohne Reinigung weiter umgesetzt.
- Ausbeute:: 1.77 g (100% der Theorie)
- ESI-MS:: (M+M)⁺ = 719
- Retentionszeit (HPLC-MS):: 2.6 min (Methode B)

### 4c) 4-(2-Oxo-1,2,4,5-tetrahydro-1,3-benzdiazepin-3-yl)-piperidin-1-carbonsäure-(R)-2-[4-(1-Carboxymethyl-piperidin-4-yl)-piperazin-1-yl]-1-(4-hydroxy-3,5-dimethylbenzyl)-2-oxo-ethylester

Zu einer Lösung von 1.77 g (2.46 mmol) 4-(2-Oxo-1,2,4,5-teirahydro-1,3-benzdiazepin-3-yl)-piperidin-1-carbonsäure-(*R*)-2-[4-(1-ethoxycarbonylmethyl-pipendin-4-yl)-piperazin-1-yl]-1-(4-hydroxy-3,5-dimethyl-benzyl)-2-oxo-ethylester in 20 mL THF wurde bei RT eine Lösung von 96 mg (4.00 mmol) LiOH in 5 mL Wasser gegeben und die Reaktionslösung über Nacht bei RT gerührt. Man engte i.vac. ein, nahm den Rückstand in 10 mL Wasser auf und wusch die wässrige Phase mit 10 mL EtOAc. Die wässrige Phase wurde mit 4 mL 1 M HCl versetzt und diese erneut mit 10 mL EtOAc gewaschen. Die wässrige Phase wurde i.vac. eingeengt und getrocknet. Das Produkt, das LiCl enthielt, wurde ohne Reingung weiter umgesetzt.
- Ausbeute:: 1.70 g (100% der Theorie)
- ESI-MS:: (M+H)⁺ = 691
- Retentionszeit (HPLC-MS):: 2.7 min (Methode B)

### 4d) 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-oxo-2-(4-{1-[2-(2-oxo-pyrrolidin-1-yl)-ethoxycarbonyl-methyl]-piperdin-4-yl}-piperazin-1-yl)-ethylester

Zu einer Lösung von 100 mg (0.15 mmol) 4-(2-Oxo-1,2,4,5-tetrahydro-1,3-benzdiazepin-3-yl)-piperidin-1-carbonsäure-(*R*)-2-[4-(1-carboxymethyl-pipeddin-4-yl)-piperazin-1-yl]-1-(4-hydroxy-3,5-dimethyl-benzyl)-2-oxo-ethylester, 18 µL (0.16 mmol) 1-(2-Hydroxy-ethyl)-pyrrolidin-2-on und 50 µL (0.36 mmol) Triethylamin in 1,5 mL DMF wurden bei RT 58 mg (0.18 mmol) TBTU gegeben und das Reaktionsgemisch 2 h bei RT gerührt. Man versetzte mit 10 mL halbgesättigter NaHCO₃-Lösung, filtrierte den entstandenen Niederschlag ab, löste diesen in 1.5 mL DMF und reinige via HPLC. Die das Produkt enthaltenden Fraktionen wurden vereinigt und lyophilisiert.
- Ausbeute:: 13 mg (11 % der Theorie)
- ESI-MS:: (M+H)⁺ = 802
- Retentionszeit (HPLC-MS):: 2.9 min (Methode B)

Analog wurden folgende Verbindungen aus jeweils 100 mg 4-(2-Oxo-1,2,4,5-tetrahydro-1,3-benzdiazepin-3-yl)-piperidin-1-carbonsäure-(*R*)-2-[4-(1-carboxymethyl-piperidin-4-yl)-piperazin-1-yl]-1-(4-hydroxy-3,5-dimethyl-benzyl)-2-oxo-ethylester und der entsprechenden Menge der Alkoholkomponente hergestellt:

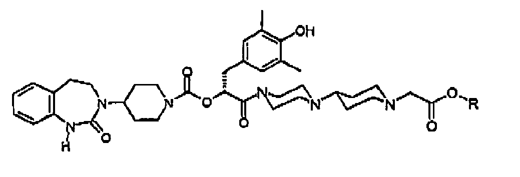

| **Beispiel** | **R** | **Ausbeute (%)** | **Massenspektrum** | **Retentionszeit HPLC (Methode)** |
|---|---|---|---|---|
| **4.1** | 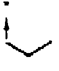 | 17 | 733 | 3.0 min |
| | | | [M+H]⁺ | (B) |
| **4.2** | 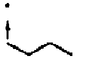 | 38 | 747 | 3,3 min |
| | | | [M+H]⁺ | (B) |
| **4.3** | 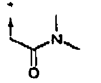 | 11 | 776 | 2.9 min |
| | | | [M+H]⁺ | (B) |

### Beispiel 4.4

### 4-(2-Oxo-1,2,4,5-tetrahydro-1,3-benzdiazepin-3-yl)-piperidin-1-carbonsäure-(R)-1-(4-hydroxy-3,5-dimethyl-benzyl)-2-(4-{1-[2-(2-methoxy-ethoxy)-ethoxycarbonylmethyl]-piperidin-4-yl}-piperazin-1-yl)-2-oxo-ethylester

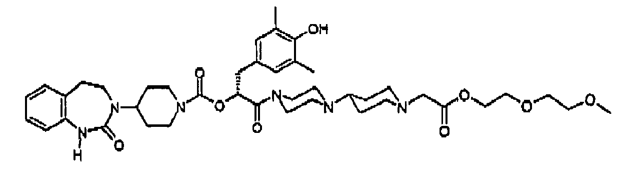

Hergestellt analog zu Beispiel 4d aus 70 mg (0.10 mmol) 4-(2-Oxo-1,2,4,5-tetrahydro-1,3-benzdiazepin-3-yl)-pipendin-1-carbonsäure-(*R*)-2-[4-(1-carboxymethyl-pipendin-4-yl)-piperazin-1-yl]-1-(4-hydroxy-3,5-dimethyl-benzyl)-2-oxo-ethylester und 13 µL (0.11 mmol) 2-(2-Methoxy-ethoxy)-ethanol. Die Reaktionslösung wurde ohne weitere Aufarbeitung via HPLC gereinigt; die das Produkt enthaltenden Fraktionen vereinigt und lyophilisiert.
- Ausbeute:: 54 mg (68 % der Theorie)
- ESI-MS;: (M+H)⁺ = 793
- Retentionszeit (HPLC-MS);: 2.8 min (Methode B)

### Beispiel 4.5

### 4-(2-Oxo-1,2,4,5-tetrahydro-1,3-benzdiazepin-3-yl)-piperidin-1-carbonsäure-(R)-2-[4-(1-hexyloxycarbonylmethyl-piparidin-4-yl)-pipardin-1-yl]-1-(4-hydroxy-3,5-dimethyl-benzyl)-2-oxo-ethylester

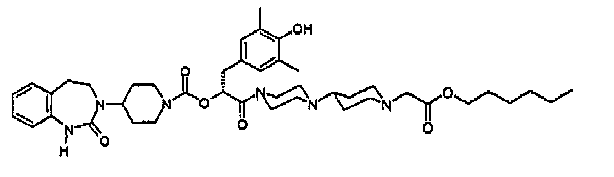

Hergestellt analog zu Beispiel 4d aus 934 mg (1.35 mmol) 4-(2-Oxo-1,2,4,5-tetrahydro-1,3-benzdiazepin-3-yl)-piperidin-1-carbonsäure-(*R*)-2-[4-(1-carboxymehtyl-piperidin-4-yl)-piperazin-1-yl]-1-(4-hydroxy-3,5-dimethyl-benzyl)-2-oxo-ethylester und 190 µL (0.11 mmol) 1-Hexanol. Die Reaktionslösung wurde mit 30 mL halbgesättigter NaHCO₃-Lösung versetzt, mit 50 mL EtOAc extrahiert und die organische Phase Ober Na₂SO₄ getrocknet. Nach Entfernen des Trocken- und Lösungsmittels wurde der Rückstand chromatographisch (Kieselgel, EtOAc/MeOH/NH₃ 90:10:1) gereinigt.
- Ausbeute:: 199 mg (19 % der Theorie)
- ESI-MS:: (M+H)⁺ = 775
- Retentionszeit (HPLC-MS):: 3.6 min (Methode B)

### Beispiel 5

### 4-(2-Oxo-1,2,4,5-tetrahydro-1,3-benzdiazepin-3-yl)-piperidin-1-carbonsäure-(R)-2-[1'-(2-dimethylcarbamoylmethoxycarbonyl-ethyl)-4,4'-bipiperidinyl-1-yl]-1-(4-hydroxy-3,5-dimethyl-benzyl)-2-oxo-ethylester

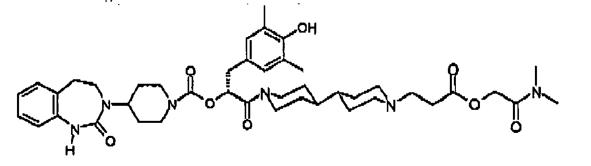

### 5a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-[1'-(2-ethoxycarbonyl-ethyl)-4,4'-bipiperidinyl-1-yl]-2-oxo-ethylester

Hergestellt analog zu Beispiel 3a aus 2.00 g (3,50 mmol) 4-(2-Oxo-1,2,4,5-tetrahydro-1,3-benzodiazepin-3-yl)-piperidin-1-carbonsäure-(*R*)-2-(4-benzyloxy-3,5-dimethyl-phenyl)-1-carboxy-ethylester (Beispiel 1g) und 1.05 g (3.90 mmol) 3-[4,4']Bipiperidinyl-1-yl-propionsäureethylester.
- Ausbeute:: 2.90 g (100 % der Theorie)
- ESI-MS:: (M+H)⁺= 823
- Retentionszeit (HPLC-MS):: 4.0 min (Methode B)

### 5b) 4-(2-Oxo-1,2,4,5-tetrahydro-1,3-benzdiazepin-3-yl)-piperidin-1-carbonsäure-(R)-2-[1'-(2-carboxy-ethyl)-4,4'-bipiperidinyl-1-yl]-1-(4-hydroxy-3,5-dimethyl-benzyl)-2-oxo-ethylester

Hergestellt analog zu Beispiel 3b aus 2.90 g (3.35 mmol) 4-(2-Oxo-1,2,4,5-tetrahydro-1,3-benzdiazepin-3-yl)-piperidin-1-carbonsäure-(*R*)-1-(4-benzyloxy-3,5-dimethyl-benzyl)-2-[1' (2-ethoxycarbonyl-ethyl)-4,4'-bipiporidinyl-1-yl]-2-oxo-ethylester, 300 mg 10% Pd/C (Hydrierung) und 128 mg LiOH (Esterhydrolyse).
- Ausbeute:: 1.70 g (71 % der Theorie)
- ESI-MS:: (M+H)⁺ = 704
- R_{f} =: 0.20 (Kieselgel, DCM/MeOH/Cyc/NH₃ 70:15:15:2)

### 5c) 4-(2-Oxo-1,2,4,5-tetrahydro-1,3-benzdiazepin-3-yl)-piperidin-1-carbonsäure-(R)-2-[1'-(2-dimethylcarbamoylmethoxycarbonyl-ethyl)-4,4'-bipiperidinyl-1-yl]-1-(4-hydroxy-3,5-dimethyl-benzyl)-2-oxo-ethylester

Hergestellt analog zu Beispiel 1i aus 100 mg (0.14 mmol) 4-(2-Oxo-1,2,4,5-tetrahydro-1,3-benzdiazepin-3-yl)-piporidin-1-carbonsäure-(*R*)-2-[1'-(2-carboxy-ethyl)-4,4'-bipiperidinyl-1-yl]-1-(4-hydroxy-3,5-dimethyl-benzyl)-2-oxo-ethylester und 16 mg (0.16 mmol) 2-Hydroxy-*N,N*-dimethyl-acetamid.
- Ausbeute:: 35 mg (31 % der Theorie)
- ESI-MS:: (M+H)⁺ = 789
- Retentionszeit (HPLC-MS):: 3.6 min (Methode B)

### Beispiel 6

### 1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-ethylester

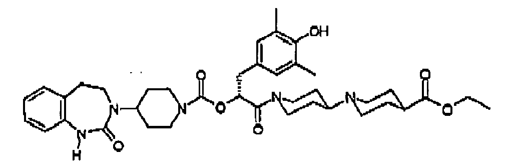

### 6a) 1'-{(R)-3-(4-Benzyloxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-ethylester

Eine Lösung von 400 mg (0.70 mmol) 4-(2-Oxo-1,2,4,5-tetrahydro-1,3-benzdiazepin-3-yl)-pipendin-1-carbonsäure-(*R*)-2-(4-benzyloxy-3,5-dimethyl-phenyl)-1-carboxy-ethylester (Beispiel 1g), 250 mg (0.78 mmol) TBTU und 0.12 mL (3.73 mmol) Triethylamin in 20 mL THF wurden bei RT 1 h gerührt. Dann erfolgte die Zugabe von 0.4 mL (2.87 mmol) Triethylamin und 270 mg (0.86 mmol) [1,4']Bipiperidinyl-4-carbonsäureethylester (eingesetzt als Bis-Hydrochlorid-Salz). Das Reaktionsgemisch wurde 24 h bei RT gerührt, mit EtOAc verdünnt, mit gesättigter NaHCO₃-Lösung gewaschen und die organische Phase Ober Na₂SO₄ getrocknet. Nach Entfernen des Trocken- und Lösungsmittels wurde der Rückstand chromatographisch (Alox Aktivitätsstufe II-III, Gradient DCM/EtOH 40;1 zu DCM/EtOH 30:1) gereinigt.
- Ausbeute:: 440 mg (79 % der Theorie)
- ESI-MS:: (M+H)⁺ = 794
- Retentionszeit (HPLC-MS):: 4.1 min (Methode B)

### 6b) 1'{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-ethylester

80 mg (0.10 mmol) 1'-{(*R*)-3-(4-Benzyloxy-3,5-dimethyl-phenyl)-2-[4-(2-oxo-1,2,4,5-tetra-hydro-1,3-benzdiazepin-3-yl)-piperidin-1-carbonyloxy]-propionyl}-1,4'-bipiperidinyl-4-carbonsäure-ethylester in 10 mL EtOH wurden mit 30 mg 10% Pd/C versetzt und bei 50°C und 50 psi Wasserstoff 1 h hydriert. Der Katalysator wurde abgesaugt und das Lösungsmittel i. vac. eingeengt. Der Rückstand wurde mit Diethylether verrieben, abgesaugt und getrocknet.
- Ausbeute:: 37 mg (52% der Theorie)
- ESI-MS:: (M+H)⁺ = 704
- Retentionszeit (HPLC-MS):: 3.2 min (Methode B)

### Beispiel 6.1

### 1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure

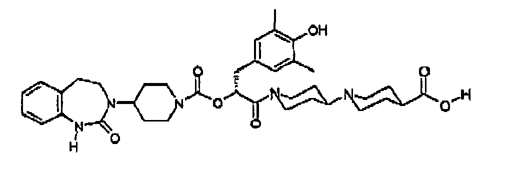

### 6.1a) 1'-{(R)-3-(4-Benzyloxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure

Zu einer Lösung von 350 mg (0.44 mmol) 1'-{(*R*)-3-(4-Benzyloxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-ethylester (Beispiel 6a) in 15 mL THF wurde eine Lösung von 20 mg (0.84 mmol) LiOH in 3 mL Wasser gegeben und das Reaktionsgemisch 3 h bei RT gerührt. Man verdünnte mit wenig Wasser, entfernte das THF i.vac. und versetzte die wässrige Lösung unter Eiskühlung mit 4 M HCl bis zur sauren Reaktion. Man extrahierte erschöpfend mit DCM und trockete über Na₂SO₄, Nach Entfernen des Trocken- und Lösungsmittels wurde der Rückstand mit Diethylether verrieben, abgesaugt und getrocknet, wobei das Produkt als Hydrochlorid-Salz anfiel.
- Ausbeute:: 310 mg (88% der Theorie)
- ESI-MS:: (M+H)⁺ = 766

### 6.1b) 1'-{(R)-3-4-Hydroxy-3,5-dimethyl-phenyl)-2-[4-(2-oxo-1,2,4,6-tetrahydro-1,3-benzdiazepin-3-yl)-piperidin-1-carbonyloxy]-propionyl}-1,4'-bipiperidinyl-4-carbonsäure

100 mg (0.13 mmol) 1'-{(*R*)-3-(4-Benzyloxy-3,5-dimethyl-phenyl)-2-[4-(2-oxo-1,2,4,6-tetrahydro-1,3-benzdiazepin-3-yl)-piperidin-1-carbonyloxy]-propionyl}-1,4'-bipiperidinyl-4-carbonsäure in 10 mL MeOH wurden mit 30 mg 10% Pd/C versetzt und bei 50°C und 3 bar Wasserstoff 2 h hydriert. Der Katalysator wurde abgesaugt und das Lösungsmittel i. vac. eingeengt. Der Rückstand wurde mit Diethylether verrieben, abgesaugt und getrocknet.
- Ausbeute:: 64 mg (72% der Theorie)
- ESI-MS;: (M+H)⁺ = 676
- Retentionszeit (HPLC-MS):: 3.0 min (Methode B)

### Beispiel 6.2

### 1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-dimethylcarbamoylmethylester

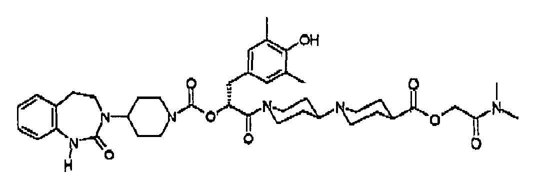

### 6.2a) 1'-{(R)-3-(4-Benzyloxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-dimethylcarbamoylmethylester

Zu einer Lösung von 200 mg (0.25 mmol) 1'-{(*R*)-3-(4-Benzyloxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure (Beispiel 6.1a), 90 mg (0.28 mmol) TBTU und 80 µL (3.73 mmol) Triethylamin in 1.8 mL DMF wurden bei RT 30 mg (0.29 mmol) 2-Hydroxy-*N,N-*dimethyl-acetamid gegeben und diese 20 h bei RT gerührt. Das Reaktionsgemisch wurde auf gesättigte NaHCO₃-Lösung gegossen, der ausgefallenen Niederschlag abgesaugt und getrocknet. Weitere Reinigung erfolgte via HPLC, die das Produkt enthaltenden Fraktionen wurden vereinigt und lyophilisiert.
- Ausbeute:: 100 mg (47% der Theorie)
- ESI-MS:: (M+H)⁺ = 851
- Retentionszeit (HPLC-MS);: 3.9 min (Methode B)

### 6.2b) 1'-{(R)-3-(4-Hydroxy-3,5-dimethyl-phenyl)-2-[4-(2-oxo-1,2,4,5-tetrahydro-1,3-benzdiazepin-3-yl)-piperidin-1-carbonyloxy]-propionyl}-1,4'-bipiperidinyl-4-carbonsäure-dimethylcarbamoylmethylester

95 mg (0.11 mmol) 1'-{(*R*)-3-(4-Benzyloxy-3,5-dimethyl-phenyl)-2-[4-(2-oxo-1,2,4,5-tetra-hydro-1,3-benzdiazepin-3-yl)-piperidin-1-carbonyloxy]-propionyl}-1,4'-bipiperidinyl-4-carbonsäure-dimethylcarbamoylmethylester in 10 mL THF wurden mit 50 mg 10% Pd/C versetzt und bei 50°C und 50 psi Wasserstoff 6 h hydriert. Der Katalysator wurde abgesaugt und das Lösungsmittel i. vac. eingeengt. Der Rückstand wurde mit Diethylether verrieben, abgesaugt und getrocknet.
- Ausbeute:: 52 mg (61 % der Theorie)
- ESI-MS:: (M+H)* = 761
- Retentionszeit (HPLC-MS):: 3.1 min (Methode B)

### Beispiel 7

### 4-(2-Oxo-1,2,4,5-tetrahydro-1,3-benzdiazepin-3-yl)-piperidin-1-carbonsäure-(R)-2-(4-ethoxycarbonylmethoxy-1,4'-bipiperidinyl-1'-yl)-1-(4-hydroxy-3,5-dimethyl-benzyl)-2-oxo-ethylester

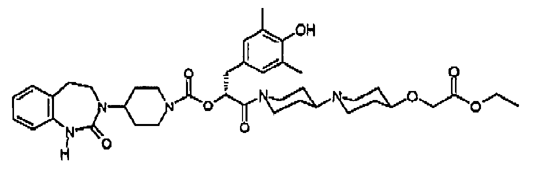

### 7a) 4-(2-Oxo-1,2,4,5-tetrahydro-1,3-benzdiazepin-3-yl)-piperidin-1-carbonsäure-(R)-1-(4-benzyloxy-3,5-dimethyl-benzyl)-2-(4-ethoxycarbonylmethoxy-1,4-bipiperidinyl-1'-yl)-2-oxo-ethylester

Zu einer Lösung von 330 mg (0.58 mmol) 4-(2-Oxo-1,2,4,5-tetrahydro-1,3-benzdiazepin-3-yl)-piperidin-1-carbonsäure-(*R*)-2-(4-benzyloxy-3,5-dimethyl-phenyl)-1-carboxy-ethyl-ester (Beispiel 1g). 200 mg (0.62 mmol)TBTU und 0.30 mL (2.16 mmol) Triethylamin in 3 mL DMF wurden bei RT 220 mg (0.64 mmol) ([1,4']Bipiperidinyl-4-yloxy)-essigsäureethylester (eingesetzt als Bis-Hydrochlorid-Salz) gegeben und diese 4 h gerührt. Das Reaktionsgemisch wurde unter Eiskühlung mit gesättigter NaHCO₃-Lösung versetzt und der Niederschlag abgesaugt. Dieser wurde in wenig DCM und EtOH gelöst und via HPLC gereinigt; die das Produkt enthaltenden Fraktionen wurden vereinigt und lyophilisiert.
- Ausbeute:: 340 mg (72 % der Theorie)
- ESI-MS:: (M+H)⁺ = 824
- Retentionszeit (HPLGMS):: 4.2 min (Methode B)

### 7b) 4-(2-Oxo-1,2,4,5-tetrahydro-1,3-benzdiazepin-3-yl)-piperidin-1-carbonsäure-(R)-2-(4-ethoxycarbonylmethoxy-1,4'-bipiperidinyl-1'-yl)-1-(4-hydroxy-3,5-dimethyl-) benzyl)-2-oxo-ethylester

340 mg (0.41 mmol) 4-(2-Oxo-1,2,4,5-tetrahydro-1,3-benzdiazepin-3-yl)-piperidin-1-carbonsäure-(*R*)-1-(4-benzyloxy-3,5-dimethyl-benzyl)-2-(4-ethoxycarbonylmethoxy-1,4'-bipiperidinyl-1'-yl)-2-oxo-ethylester in 20 mL EtOH wurden mit 100 mg 10% Pd/C versetzt und bei 50°C und 50 psi Wasserstoff 2 h hydriert. Der Katalysator wurde abgesaugt und das Lösungsmittel i. vac. eingeengt.
- Ausbeute:: 270 mg (89% der Theorie)
- ESI-MS:: (M+H)⁺ = 734
- Retentionszeit (HPLGMS):: 3.3 min (Methode B)

### Beispiel 7.1

### 4-(2-Oxo-1,2,4,5-tetrahydro-1,3-benzdiazepin-3-yl)-piperidin-1-carborlsäun-(R)-2-(4-carboxymethoxy-1,4'-bipiperidinyl-1'-yl)-1-(4-hydroxy-3,5-dimethyl-benzyl)-2-oxo-ethylester

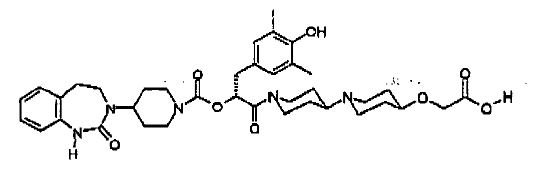

Zu einer Lösung von 180 mg (0.25 mmol) 4-(2-Oxo-1,2,4,5-tetrahydro-1,3-benzdiazepin-3-yl)-piperidin-1-carbonsäure-(*R*)-2-(4-ethoxycarbonylmethoxy-1,4'-bipipoddinyl-1'-yl)-1-(4-hydroxy-3,5-dimethyl-benzyl)-2-oxo-ethylester in 15 mL THF wurde eine Lösung von 15 mg (0.63 mmol) LiOH in 5 mL Wasser gegeben und das Reaktionsgemisch 4 h bei RT gerührt. Man versetzte mit 0,66 mL 1 M HCl und i.vac. eingeengt. Der Rückstand wurde in wenig DCM/MeOH (7:3) aufgenommen und über Kieselgel filtriert. Das Filtrat wurde i.vac. eingeengt, der Rückstand mit Diethylether verrieben, abgesaugt und getrocknet.
- Ausbeute:: 173 mg (100% der Theorie)
- ESI-MS:: (M+H)⁺ = 706
- Retentionszeit (HPLC-MS):: 2.9 min (Methode B)

### Beispiel 8

### 4-(2-Oxo-1,2,4,5-tetrahydro-1,3-benzdiazepin-3-yl)-piperidin-1-carbonsäure-(R)-2-[4-(2-ethoxycarbonyl-ethyl)-1,4'-bipiperidinyl-1'-yl]-1-(4-hydroxy-3,5-dimethyl-benzyl)-2-oxo-ethylester

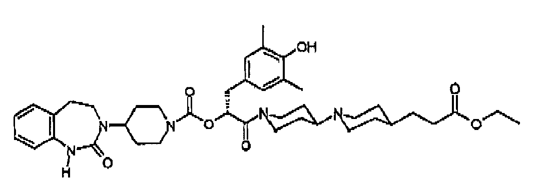

Zu einer Lösung von 500 mg (1.04 mmol) 4-(2-Oxo-1,2,4,5-tetraliydro-1,3-benzdiazepin-3-yl)-piperidin-1-carbonsäure-(*R*)-2-(4-hydroxy-3,5-dimethyl-phanyl)-1-carboxy-ethylester (Beispiel 1h), 385 mg (1.20 mmol) TBTU und 0.48 mL (3.40 mmol) Triethylamin in 10 mL DMF wurden bei RT 410 mg (1.20 mmol) 3-[1,4']Bipiperidinyl-4-yl-propionsäureethylester (eingesetzt als Bis-Hydrochlorid-Salz) gegeben und diese 2 h gerührt. Das Reaktionsgemisch wurde ohne weitere Aufarbeitung via HPLC gereinigt. Die das Produkt enthaltenden Fraktionen wurden vereinigt, i.vac eingeengt und der Rückstand mit gesättigter NaHCO₃-Lösung gerührt. Der entstandene Niederschlag wurde abfiltriert und getrocknet.
- Ausbeute:: 310 mg (41 % der Theorie)
- ESI-MS:: (M+H)⁺ = 732
- Retentionszeit (HPLC-MS):: 2.4 min (Methode B)

### Beispiel 8.1

### 4-(2-Oxo-1,2,4,5-tetrahydro-1,3-benzdiazepin-3-yl)-piperidin-1-carbonsäure-(R)-2-[4-(2-carboxy-ethyl)-1,4'-bipiperidinyl)-1'-yl]-1-(4-hydroxy-3,6-dimethyl-benzyl)-2-oxo-ethylester

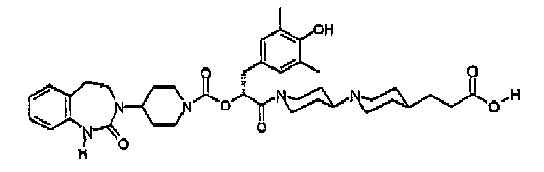

Zu einer Lösung von 50 mg (0.07 mmol) 4-(2-Oxo-1,2,4,5-tetrahydro-1,3-berlzdiazepin-3-yl)-piperidin-1-carbonsäure-(*R*)-2-[4-(2-ethoxycarbonyl-ethyl)-1,4'-bipipehdiriyl-1'-yl]-1-(4-hydroxy-3,5-dimethyl-benzyl)-2-oxo-ethylester in 3 mL THF wurde eine Lösung von 2.6 mg (0.11 mmol) LiOH in 1 mL Wasser gegeben und das Reaktionsgemisch 4 h bei RT gerührt. Man entfernte das THF im Stickstoffstrom und reinigte das Rohprodukt via HPLC. Die das Produkt enthaltenden Fraktionen wurden vereinigt und lyophilisiert.
- Ausbeute:: 32 mg (67 % der Theorie)
- ESI-MS:: (M+H)⁺ = 704
- Retentionszeit (HPLC-MS):: 3.1 min (Methode B)

### Beispiel 9

### 4-(2-Oxo-1,2,4,5-tetrahydro-1,3-benzdiazepin-3-yl)-piperidin-1-carbonsäure-(R)-2-{4-[1-(3-ethoxycarbonyl-propyl)-piperidin-4-yl]-piperazin-1-yl}-1-(4-hydroxy-3,5-dimethyl-benzyl)-2-oxo-ethylester

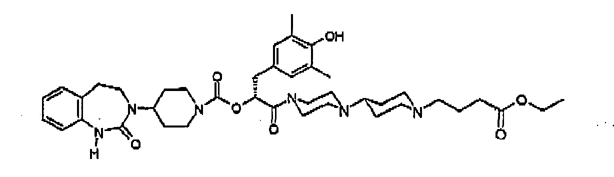

Hergestellt analog Beispiel 8 aus 500 mg (1.04 mmol) 4-(2-Oxo-1,2,4,6-tetrahydro-1,3-benzdiazepin-3-yl)-piperidin-1-carbonsäure-(R)-2-(4-hydroxy-3,5-dimethyl-phenyl)-1-carboxy-ethylester (Beispiel 1h) und 385 mg (1.20 mmol) 4-(4-Piperazin-1-yl-piperidin-1-yl)-butansäureethylester.
- Ausbeute:: 85 mg (11 % der Theorie)
- ESI-MS:: (M+H)⁺ = 747
- Retentionszeit (HPLC-MS):: 2.8 min (Methode B)

### Beispiel 9.1

### 4-(2-Oxo-1,2,4,5-tetrahydro-1,3-benzdiazepin-3-yl)-piperidin-1-carbonsäure-(R)-2-{4-[1-(3-carboxy-propyl)-piperidin-4-yl]-piperazin-1-yl}-1-(4-hydroxy-3,5-dimethyl-benzyl)-2-oxo-ethylester

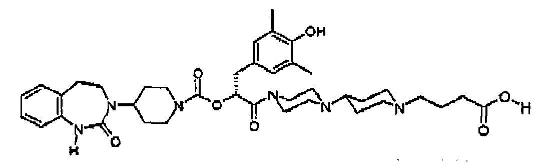

Hergestellt analog Beispiel 8.1 aus 50 mg (0.07 mmol) 4-(2-Oxo-1,2,4,5-tetrahydro-1,3-benzdiazepin-3 yl)-piperidin-1-carbonsäure-(*R*)-2-{4-[1-(3-ethoxycarbonyl-propyl)-piperidin-4-yl]-piperazin-1-yl}-1-(4-hydroxy-3,5-dirnethyl-benzyl)-2-oxo-ethylester und 2.6 mg (0.11 mmol) LiOH.
- Ausbeute:: 24 mg (50 % der Theorie)
- ESI-MS:: (M+H)⁺ = 719
- Retentionszeit (HPLC-MS):: 2.7 min (Methode B)

### Beispiel 10

### 4-(2-Oxo-1,2,4,5-tetrahydro-1,3-benzdiazepin-3-yl)-pipendin-1-carbonsäure-(R)-1-(4-hydroxy-3-methoxy-5-methyl-bene)-2-[4-(4-methyl-piperazin-1-yl)-piparidin-1-yl]-2-Oxo-ethylester

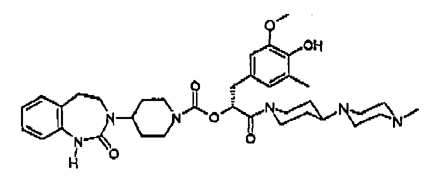

### 10a) 4-Brom-2-methoxy-6-methyl-phenol

Zu einer Lösung von 42.3 g (0.31 mol) 2-Methoxy-6-methyl-phenol in 450 mL AcOH wurde innerhalb von 5.5 h eine Lösung von 56.2 g (0.32 mol) *N*-Bromsuccinimid in 1700 mL AcOH zugetropft und das Gemisch 16 h bei RT gerührt. Das Reaktionsgemisch wurde i.vac. eingeengt und der Rückstand in DCM aufgenommen. Die organische Phase wurde mit 5% NaHCOs- und gesättigter NaCl-Lösung gewaschen, über Na₂SO₄ getrocknet und i.vac. eingeengt. Das rote Öl wurde ohne weitere Reinigung in den nachfolgenden Reaktionsschritt eingesetzt.
- Ausbeute:: 65.9 g (66% d. Theorie)
- R_{f} =: 0.32 (Kieselgel, Hexan/EtOAc 4:1)
- Retentionszeit (HPLC-MS):: 11.1 min (Methode D)

### 10b) 2-Benzyloxy-5-brom-1-methoxy-3-methyl-benzol

Zu einer Lösung von 65.9 g (0.26 mol) 4-Brom-2-methoxy-6-methyl-phenol in 330 mL DMF wurden bei RT 45,7 g (0.33 mol) K₂CO₃ und eine Lösung von 40.3 mL (0.33 mol) Benzylbromid zugegeben und das Gemisch 18 h bei RT gerührt. Das Gemisch wurde filtriert, i.vac. eingeengt und der Rückstand in Diethylether aufgenommen. Die organische Phase wurde mit Wasser, 5% Na₂CO₃- und NaCl-Lösung gewaschen, über Na₂SO₄ getrocknet und i.vac. eingeengt. Das Rohprodukt wurde ohne weitere Reinigung in den nachfolgenden Reaktionsschritt eingesetzt.
- Ausbeute:: 92,2 g (81% d. Theorie)
- R_{f} =: 0.56 (Kieselgel, Hexan/EtOAc 4:1)
- Retentionszeit (HPLC-MS):: 16.3 min (Methode D)

### 10c) 4-Benzyloxy-3-methoxy-5-methyl-benzaldehyd

Zu einer Lösung von 61.2 g (119.5 mmol) 2-Benzyloxy-5-brom-1-methoxy-3-methylbenzol in 240 mL THF wurden bei -75°C 96 mL (240 mmol) n-Butyllithium (2.5 M in Hexan) zugetropft und das Gemisch 15 min bei -75 °C gerührt. Eine Lösung von 31 mL (402 mmol) DMF in 30 mL THF wurde zugetropft, das Gemisch auf 0°C erwärmt und weitere 2 h gerührt. Die Reaktion wurde mit gesättigter NH₄Cl-Lösung versetzt, mit 150 mL Wasser verdünnt und die Phasen getrennt. Die wässrige Phase wurde erschöpfend mit Diethylether extrahiert. Die vereinigten organischen Phasen wurden mit gesättigter NaCl-Lösung gewaschen, über Na₂SO₄ getrocknet und i.vac. eingeengt. Säufenchromatographie (Kieselgel, Hexan/EtOAc 85:15) ergab das Produkt als gelbes Öl.
- Ausbeute:: 27.1 g (88% d. Theorie)
- R_{f} =: 0.32 (Kieselgel, Hexan/EtOAc 4:1)
- Retentionszeit (HPLC-MS):: 13.3 min (Methode D)

### 10d) 2-Acetylamino-3-(4-benzyloxy-3-methoxy-5-methyl-phenyl)-acrylsäure

Eine Suspension von 27.0 g (105.4 mmol) 4-Benzyloxy-3-methoxy-5-methyl-benzaldehyd, 18.5 g (158.0 mmol) N-Acetylglycin und 12.96 g (158.0 mmol) NaOAc in 120 mL Essigsäureanhydrid wurde unter Stickstoff 3.5 h auf 115°C erwärmt. Bei 100 °C wurden langsam 60 mL Wasser zugetropft und das Gemisch 1 h gerührt. Das Reaktionsgemisch wurde auf RT gekühlt, in Wasser gegossen und die wässrige Phase mit EtOAc erschöpfend extrahiert. Die vereinigten organischen Phasen wurden mit gesättigter NaCl-Lösung gewaschen, über Na₂SO₄ getrocknet und i.vac. eingeengt. Der Rückstand wurde mit Isopropanol verrieben, der erhaltene Feststoff mit Isopropanol, Diethylether und wenig Aceton gewaschen und i.vac. bei 45°C getrocknet.
- Ausbeute:: 21.2 (57% d. Theorie)
- R_{f} =: 0.24 (Kieselgel, Hexan/EtOAc 4:1)
- Retentionszeit (HPLC-MS):: 9.4 min (Methode D)

### 10e) 3-(4-Benzyloxy-3-methoxy-5-methyl-phenyl)-2-oxo-propionsäure

Das Produkt wurde analog zu Beispiel 1c ausgehend von 20.0 g (56.3 mmol) 2-Acetylamino-3-(4-benzyloxy-3-methoxy-5-mothyl-phenyl)-acrylsäure erhalten, Das Rohprodukt wurde ohne weitere Reinigung in den nachfolgenden Reaktionsschritt eingesetzt.
- Ausbeute:: 15.6 g (53% d. Theorie)
- Retentionszeit (HPLC-MS):: 11.9 min (Methode D)

### 10f) (R)-3-(4-Benzyloxy-3-methoxy-5-methyl-phenyl)-2-hydroxy-propionsäure

Das Produkt wurde analog zu Beispiel 1d ausgehend von 16.0 g (50.90 mmol) 3-(4-Benzyloxy-3-methoxy-5-methyl-phenyl)-2-oxo-pmpionsäure hergestellt.
- Ausbeute:: 7.63 g (47% d. Theorie)
- Retentionszeit (HPLC-MS):: 9.8 min (Methode D)

### 10g) (R)-3-(4-Benzyloxy-3-methoxy-5-methyl-Phenyl)-2-hydroxy-Propionsäure-methylester

Das Produkt wurde analog zu Beispiel 1e ausgehend von 7,6 g (24.02 mmol) (R)-3-(4-Benzyloxy-3-methoxy-5-methyl-phenyl)-2-hydroxy-propionsäure hergestellt.
- Ausbeute:: 6.84 g (86% d. Theorie)
- Retentionszeit (MPLG-MS):: 11.7 min (Methode D)

### 10h) 4-(2-Oxo-1,2,4,5-tetrehydro-1,3-benzdiazepin-3-yl)-piperidin-1-carbonsäure-(R)-2-(4-benzyloxy-3-methoxy-5-mothyl-Phenyl)-1-methoxycarbonyl-ethylester

Das Produkt wurde analog zu Beispiel 1f ausgehend von 6.8 g (20.6 mmol) (R)-3-(4-Benzyloxy-3-methoxy-5-methyl-phenyl)-2-hydroxy-propionsäure-methyl-ester in Acetonitril hergestellt,
- Ausbeute:: 8.16 g (66% d. Theorie)
- ESI-MS:: (M+H)⁺ = 602
- Retentionszeit (HPLC-MS):: 14.1 min (Methode D)

### 10i) 4-(2-Oxo-1,2,4,5-tetrahydro-1,3-benzdiazepin-3-yl)-piperidin-1-carbonsäure-(R)-2-(4-benzyloxy-3-methoxy-5-methyl-phenyl)-1-carboxy-ethylester

Das Produkt wurde analog zu Beispiel 1g ausgehend von 8.16 g (13.65 mmol) 4-(2-Oxo-1,2,4,5-tetrahydro-1,3-benzdiazepin-3-yl)-piperidin-1-carbonsäure-(*R*)-2-(4-benzyloxy-3-methoxy-5-methyl-phenyl)-1-methoxycarbonyl-ethylesfier hergestellt.
- Ausbeute:: 7.83 g (98% d. Theorie)
- ESI-MS:: (M+H)⁺ = 588
- Retentionszeit (HPLC-MS):: 12,2 min (Methode D)

### 10j) 4-(2-Oxo-1,2,4,5-tetrahydro-1,3-benzdiazepin-3-yl)-piperidin-1-carbonsäure-(R)-1-carboxy-2-(4-hydroxy-3-methoxy-5-methyl-phenyl)-ethylester

Das Produkt wurde analog zu Beispiel 1 h ausgehend von 7.80 g (13.27 mmol) 4-(2-Oxo-1,2,4,5-tetrahydro-1,3-benzdiazepin-3-yl)-piperidin-1-carbonsäure-(*R*)-2-(4-benzyloxy-3-methoxy-5-methyl-phenyl)-1-carboxy-ethylester hergestellt.
- Ausbeute:: 5.33 g (80% d. Theorie)
- ESI-MS:: (M+H)⁺ = 498
- Retentionszeit (HPLC-MS):: 8.4 min (Methode D)

### 10k) 4-(2-Oxo-1,2,4,5-tetrahydro-1,3-benzdiazepin-3-yl)-piperidin-1-carbonsäure-(R)-1-(4-hydroxy-3-methoxy-5-methyl-benzyl)-2-[4-(4-methyl-piperazin-1-yl)-piperidin-1-yl]-2-oxo-ethylester

Zu einer Lösung von 100 mg (0.20 mmol) 4-(2-Oxo-1,2,4,5-tetrahydro-1,3-benzdiazepin-3-yl)-piperidin-1-Carbonsäure-(*R*)-1-carboxy-2-(4-hydroxy-3-methoxy-5-mathyl-phenyl)-ethylester, 37.8 µL (0.22 mmol) Ethyldiisopropylamin und 44 mg (0.24 mmol) 1-Mothyl-4-(piperidin-4-yl)-piperazin in 5 mL DMF wurde bei RT unter Stickstoff 84 mg (0.22 mmol) HATU zugegeben und das Gemisch 3 h gerührt. Das Reaktionsgemisch wurde bei 50°C i.vac. eingeengt und das Rohprodukt mittels präparativer HPLC-MS gereinigt.
- Ausbeute:: 89 mg (57% d. Theorie)
- ESI-MS:: (M+H)⁺ = 663
- Retentionszeit (HPLC-MS):: 5.6 min (Methode D)

Analog wurden folgende Verbindungen aus jeweils 100 mg (Beispiele 10.1 und 10.2) bzw. aus 150 mg (Beispiele 10.3 bis 10.5) 4-(2-Oxo-1,2,4,5-tetrahydro-1,3-benzdiazepin-3-yl)-piperidin-1-carbonsäure-(*R*)-1-carboxy-2-(4-hydroxy-3-methoxy-5-methyl-phenyl)-ethylester und der entsprechenden Menge an Amin hergestellt:

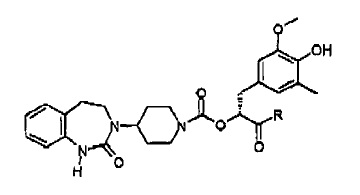

| **Beispiel** | **R** | **Ausbeute (%)** | **Massenspektrum** | **Retentionszeit HPLC (Methode)** |
|---|---|---|---|---|
| **10.1** | 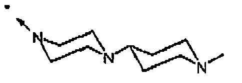 | 60 | 663 | 3.1 min |
| | | | [M+H]⁺ | (D) |
| **10.2** | 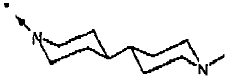 | 71 | 662 | 3.6 min |
| | | | [M+H]⁺ | (D) |
| **10.3** | 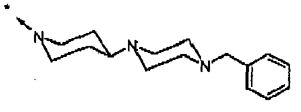 | quant. | 739 | 3.8 min |
| | | | [M+H]⁺ | (D) |
| **10.4** | 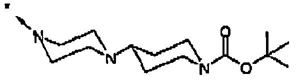 | quant. | 749 | 4.0 min |
| | | | [M+H]⁺ | (D) |
| **10.5** | 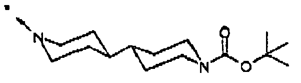 | quant. | 748 | 6.3 min |
| | | | [M+H]⁺ | (D) |

### Beispiel 10.6

### 4-(2-IOxo-1,2,4,5-tetrahydro-1,3-benzdiazepin-3-yl)-piperidin-1-carbonsäure-(R)-1-(4-hydroxy-3-methoxy-5-methyl-benzyl)-2-oxo-2-(4-piperidin-4-yl-piperazin-1-yl)-ethylester

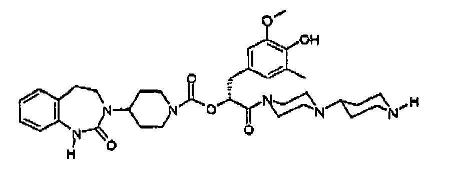

Eine Lösung von 220 mg (0.29 mmol) 4-(2-0xo-1,2,4,5-tetrahydro-1,3-benzdiazepin-3-yl)-piperidin-1-carbonsäure-(*R*)-2-[4-(1-tert-butoxycarbonyl-piperidin-4-yl)-piperazin-1-yl]-1-(4-hydroxy-3-methoxy-5-methyl-benzyl)-2-oxa-ethyl-ester (Beispiel 10.4) in 3 mL Ameisensäure wurde 4 h bei RT gerührt und anschließend bei 40°C i.vac, eingeengt. Das Rohprodukt wurde mittel präparativer HPLC-MS gereinigt.
- Ausbeute:: 13 mg (6% d. Theorie)
- ESI-MS:: (M+H)⁺ = 649
- Retentionszeit (HPLC-MS):: 3.8 min (Methode D)

### Beispiel 10.7

### 4-(2-Oxo-1,2,4,5-tetrahydro-1,3-benzdiazepin-3-yl)-piperidin-1-carbonsäure-(R)-2-4,4'-bipipendinyl-1-yl-1-(4-hydroxy-3-methoxy-5'-methyl-benzyl)-2-oxo-ethylester

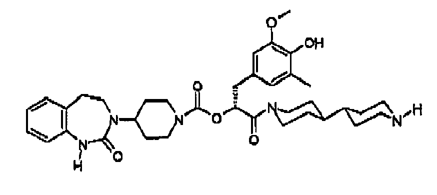

Das Produkt wurde analog zu Beispiel 10.6 ausgehend von 210 mg (0.28 mmol) 1'-{(*R*)-3-(4-Hydroxy-3-methoxy-5-methyl-phenyl)-2-[4-(2-oxo-1,2,4,5-tetrahydro-1,3-benzdiazepin-3-yl)-piparidin-1-carbonyloxy]-propionyl}-4,4'-bipiperidinyl-1-carbonsäure-tert-butylester (Beispiel 10.5) hergestellt.
- Ausbeute:: 164 mg (77% d. Theorie)
- ESI-MS:: (M+H)⁺ = 648
- Retentionszeit (HPLC-MS):: 3.6 min (Methode D)

### Beispiel 10.8

### 4-(2-Oxo-1,2,4,5-tetrahydro-1,3-benzodiazepin-3-yl)-piperidin-1-carbonsäure-(R)-1-(4-hydroxy-3-methoxy-5-methyl-benzyl)-2-oxo-2-(4-piperazin-1-yl-piperidin-1-yl)-ethylester

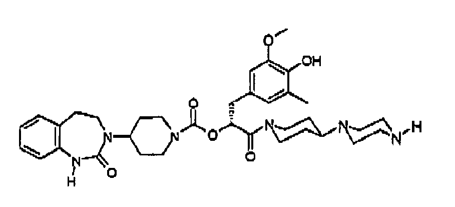

Eine Suspension von 222 mg (0.30 mmol) 4-(2-Oxo-1,2,4,5-tetrahydro-1,3-benzdiazepin-3-yl)-piperidin-1-carbonsäure-(*R*)-2-[4-(4-benzyl-piperazin-1-yl)-piperidin-1-yl]-1-(4-hydroxy-3-methoxy-5-methyl-benzyl)-2-oxo-ethylester (Beispiel 10.3) und 60 mg 10% Pd/C in 10 mL EtOH und 6 mL MeOH wurde bei 60 psi und RT 72 h hydriert. Der Katalysator wurde abfiltriert und das Rohprodukt mittels HPLC-MS gereinigt.
- Ausbeute:: 64 mg (28% d. Theorie)
- ESI-MS:: (M+H)⁺ = 649
- Retentionszeit (HPLC-MS);: 3.2 min (Methode D)

### Beispiel 11

### 4-(2-Oxo-1,2,4,5-tetrahydro-1,3-benzdiazepin-3-yl)-piperidin-1-carbonsäur-(R)-1-(3-ethyl-4-hydraxy-5-methyl-benzyl)-2-[4-(4-methyl-piperazin-1-yl)-piperidin-1-y1]-2-oxo-ethylester

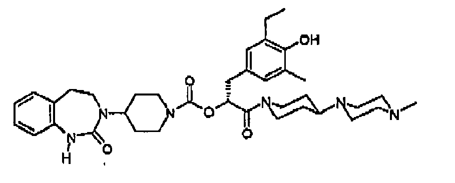

### 11a) 2-Ethyl-6-methyl-Phenol

Zu einer Lösung von 30 g (222 mmol) 2-Ethyl-6-methyl-anilin in 135 mL EtOH wurden bei 0°C 19.4 mL (0.23 mmol) konzentrierte HCl und eine Lösung von 16.1 g (0.23 mmol) Natriumnitrit in Wasser (ca. 70 mL) zugegeben und 15 min gerührt. Dieses Gemisch wurde bei 45°C zu einer Lösung 10.5 mL konzentrierter H₂SO₄ in 300 mL Wasser zugegeben und am Ende der Zugabe auf 70°C erhitzt. Die wässrige Phase wurde auf RT abgekühlt und mit EtOAc erschöpfend extrahiert. Die kombinierten organischen Phasen wurden mit 1 M NaOH-Lösung extrahiert. Die wässrige Phase wurde mit DCM gewaschen, mit 4 N HCl-Lösung auf pH 1 angesäuert und mit DCM extrahiert. Die organische Phase wurde mit gesättigter NaCl-Lösung gewaschen, über Na₂SO₄ getrocknet und i.vac. eingeengt. Das Rohprodukt wurde ohne weitere Reinigung in den nachfolgendenReaktionsschritt eingesetzt.
- Ausbeute:: 12.0 g (40% d. Theorie)

### 11b) 4-Brom-2-ethyl-6-methyl-phenol

Zu einer Lösung von 33.6 g (247 mmol) 2-Ethyl-6-methyl-phenol in 350 mL Chloroform wurde bei RT eine Lösung von 12.7 mL (247 mmol) Brom in 10 mL Chloroform zugetropft und das Gemisch 2 h gerührt. Das Reaktionsgemisch wurde mit einer wässrigen NaHSO₃-Lösung versetzt und 20 min gerührt, Die Phasen wurden getrennt und die organische Phase mit gesättigter NaCl-Lösung gewaschen, über Na₂SO₄ getrocknet und i.vac. eingeengt. Säulenchromatographie (Kieselgel, Cyc/EtOAc 9:1) ergab das Produkt.
- Ausbeute:: 39.8 g (75% d. Theorie)
- ESI-MS:: (M+H)⁺ = 214/216 (Br)
- Retentionszeit (HPLC-MS):: 6.3 min (Methode D)

### 11c) 2-Benzyloxy-5-brom-1-ethyl-3-methyl-benzol

Eine Suspension von 39.8 g (185 mmol) 4-Brom-2-ethyl-6-methyl-phenol. 63.9 g (0.46 mmol) K₂CO₃ und 22.0 mL (185 mmol) Benzylbromid in 450 mL Acetonitril wurde 3 h unter Rückfluss erhitzt, auf RT abgekühlt und i.vac. eingeengt. Der Rückstand wurde mit EtOAc versetzt, die organische Phase mit Wasser und gesättigter NaCl-Lösung gewaschen, über Na₂SO₄ getrocknet und i.vac. eingeengt.
- Ausbeute:: 54.5 g (96% d. Theorie)
- ESI-MS:: (M+H)⁺ = 304/306 (Br)
- Retentionszeit (HPLC-MS):: 9.4 min (Methode D)

### 11d) (Z,E)-2-Acetylamlno-3-(4-benzyloxy-3-ethyl-5-methyl-phenyl)-acrylsäure-methylester

Hergestellt analog Beispiel 1b aus 50.4 g (165,1 mmol) 2-Benzy)oxy-5-brom-1-ethyl-3-methyl-benzot und 28.9 g (198.2 mmol) 2-Acetylamino-acrylsäuremethylester.
- Ausbeute:: 41.0 g (68% d. Theorie)
- ESI-MS;: (M+H)⁺ = 368
- Retentionszeit (HPLC-MS):: 4.5 min (Methode B)

### 11e) 3-(4-Benzyloxy-3-ethyl-5-methyl-phenyl)-2-oxo-propionsäure

Zu einer Lösung von 41.0 g (112 mmol) (Z.E)-2-Acetylamino-3-(4-benzyloxy-3-ethyl-5-mothyl-phenyl)-acrylsäuremethylester in 300 mL 1,4-Dioxan wurden 200 mL 4 M HCl gegeben und die Reaktionslösung 7 h auf 130°C (Badtemperatur) erhitzt. Die organische Phase wurde heiß abgetrennt, i.vac. eingeengt und der erhaltene Rückstand aus Toluol umkristallisiert.
- Ausbeute:: 9.6 g (28% d. Theorie)
- ESI-MS:: (M+H)⁺ = 312
- Retentionszeit (HPLC-MS):: 4.1 min (Methode B)

### 11f) (R)-3-(4-Benzyloxy-3-ethyl-5-methyl-phenyl)-2-hydroxy-propionsäure

Unter einer Argonatmosphäre wurde eine Lösung von 9.59 g (30.7 mmol) 3-(4-Benzyloxy-3-ethyl-5-methyl-phenyl)-2-oxo-propionsäure in 25 mL THF mit 4.26 mL (31.0 mmol) Triethylamin versetzt, 5 min nachgerührt und auf -30°C (interne Temperatur) abgekühlt. Eine Lösung von 19.7 g (61,0 mmol) (1R)-B-Chlordiisopinocampheylboran in 35 mL wurde zugetropft und die Reaktionslösung nach beendeter Zugabe 30 min ohne Kühlung nachgerührt. Man versetzte mit 15 mL 4 N NaOH (Temperaturanstieg auf 20°C), rührt 5 min nach, kühlt auf 0°C ab, versetzt mit 50 mL MTBE und rührt 20 min nach. Die organische Phase wurde abgetrennt und über Na₂SO₄ getrocknet. Nach Entfernen des Trocken- und Lösungsmittels wurde der Rückstand ohne Reinigung weiter umgesetzt.
- Ausbeute;: 10.3 g (100% d. Theorie)
- ESI-MS:: (M-H)⁻ = 313
- Retentionszeit (HPLC-MS):: 4.2 min (Methode B)

### 11g) (R)-3-(4-Benzyloxy-3-ethyl-5-methyl-phenyl)-2-hydroxy-propionsäuremethylester

Hergestellt analog Beispiel 1 e aus 10.3 g (30.7 mmol) (*R*)-3-(4-Benzyloxy-3-ethyl-5-methyl-phenyl)-2-hydroxy-propionsäure und 4.71 mL (64.5 mmol) Thionylchlorid. Das erhaltene Rohprodukt wurde ohne Reinigung weiter umgesetzt.

### 11h) 4-(2-Oxo-1,2,4,5-tetrahydro-1,3-benzdiazepin-3-yl)-piperidin-1-carbonsäure-(R)-2-(4-benzyloxy-3-ethyl-5-methyl-phenyl)-1-methoxycarbonyl-ethylester

Zu einer auf 60°C (Badtemperatur) erwärmten Lösung von 75 mL Pyridin wurden innerhalb 10 min eine Lösung von 7,12 g (34.3 mmol) 4-Nitrophenyl-chloroforrnat in 30 mL THF zugegeben, 10 min nachgerührt und dann eine Lösung von 10.0 g des Rohproduktes aus Beispiel 11g in 50 mL Pyridin zugetropft. Man rührte 1 h nach, versetzte mit 6.72 g (27.4 mmol) 3-Pipendtn-4-yl-1.3.4.5-tetrahydro-1,3-benzdiazepin-2-on und erhöhte die Badtemperatur auf 100°C (2 h). Der entstandene Niederschlag wurde filtriert, das Filtrat i.vac. eingeengt, der Rückstand mit 150 mL EtOAc versetzt, die organische Phase zweimal mit je 50 mL 1 M KHSO₄-Lösung und zehnmal mit je 50 mL 15% K₂CO₃-Lösung gewaschen und über Na₂SO₄ getrocknet, Nach Entfernen des Trocken- und Lösungsmittels wurde der Rückstand chromatographisch (Kieselgel, EtOAc/Cyc 2:1) gereinigt.
- Ausbeute:: 2.28 g (14% d, Theorie)
- ESI-MS:: (M+H)⁺ = 600
- Retentionszeit (HPLC-MS):: 5.4 min (Methode B)

### 11i) 4-(2-Oxo-1,2,4,5-tetrahydro-1,3-benzdiazepin-3-yl)-piperidin-1-carbonsäure-(R)-2-(4-benzyloxy-3-ethyl-5-methyl-phenyl)-1-carboxyethylester

Zu einer Lösung von 800 mg (1.33 mmol) 4-(2-Oxo-1,2,4,5-tetrahydro-1,3-benzdiazepin-3-yl)-piperidin-1-carbonsäure-(*R*)-2-(4-benzyloxy-3-ethyl-5-methyl-phenyl)-1-methoxycarbonyl-ethylesfier in 15 mL THF wurde eine Lösung von 50 mg (2.09 mmol) LiOH in 5 mL Wasser gegeben und das Reaktionsgemisch 1 h bei RT gerührt. Man engte i.vac ein, nahm den Rückstand in 50 mL Wasser auf und versetzte mit 2 M HCl bis zur sauren Reaktion. Der ausgefallene Niederschlag wurde abfiltriert, mit Wasser gewaschen und getrocknet. Weitere Reinigung erfolgte durch Auskochen mit 150 mL Wasser, Filtration und erneutem Trocknen.
- Ausbeute:: quantitativ
- ESI-MS:: (M+H)⁺ = 586
- Retentionszeit (HPLC-MS):: 4.8 min (Methode B)

### 11k) 4-(2-Oxo-1,2,4,5-tetrahydro-1,3-benzdiazepin-3-yl)-piperidin-1-carbonsäure-(R)-1-carboxy-2-(3-ethyl-4-hydroxy-5-methyl-phenyl)-ethylester

810 mg (1.38 mmol) 4-(2-Oxo-1,2,4,5-tetrahydro-1,3-benzdiazepin-3-yl)-piperidin-1-carbonsäure-(*R*)-2-(4-benzyloxy-3-ethyl-5-methyl-phenyl)-1-carboxy-ethylester in 25 mL MeOH wurden mit 80 mg 10% Pd/C versetzt und bei RT und 3 bar Wasserstoff bis zum Reaktionsstillstand hydriert. Der Katalysator wurde abgesaugt und das Lösungsmittel i.vac. eingeengt. Der Rückstand wurde mit DIPE verrieben, abgesaugt und getrocknet.
- Ausbeute:: 639 mg (93% der Theorie)
- ESI-MS:: (M+H)⁺ = 496
- Retentionszeit (HPLC-MS):: 3.7 min (Methode B)

### 11l) 4-(2-Oxo-1,2,4,5-tetrahydro-1,3-benzdiazepin-3-yl)-piperidin-1-carbonsäure-(R)-1-(3-ethyl-4-hydroxy-5-methyl-benzyl)-2-[4-(4-methyl-piperazin-1-yl)-piperidin-1-yl]-2-oxo-ethylester

Zu einer Lösung von 80 mg (0.16 mmol) 4-(2-Oxo-1,2,4,5-tetrahydro-1,3-benzdiazepin-3-yl)-piperidin-1-carbonsäure-(*R*)-1-carboxy-2-(3-ethyl-4-hydroxy-5-methyl-phenyl)-ethyl-ester, 57 mg (0.18 mmol) TBTU und 28 µL (0.20 mmol) Triethylamin in 1 mL DMF wurden bei RT 33 mg (0.18 mmol) 1-Methyl-4-piperidin-4-yl-piperazin gegeben und das Reaktionsgemisch 2 h gerührt. Die Reaktionslösung wurde ohne weitere Aufarbeitung via HPLC gereinigt, die das Produkt enthaltenden Fraktionen wurden vereinigt und lyophilisiert.
- Ausbeute:: 68 mg (64% der Theorie)
- ESI-MS:: (M+H)⁺ = 661
- Retentionszeit (HPLGMS):: 3.0 min (Methode B)

Analog wurden folgende Verbindungen aus 80 mg (Beispiel 11.1) bzw. aus 150 mg (Beispiel 11.2) 4-(2-Oxo-1,2,4,5-tetranydro-1,3-benzdiazepin-3-yl)-piperidin-1-carbonsäure-(*R*)-1-carboxy-2-(3-ethyl-4-hydroxy-5-methyl-phenyl)-ethylester und der entsprechenden Menge an Amin hergestellt:

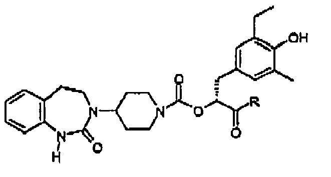

| **Beispiel** | **R** | **Ausbeute (%)** | **Massenspektrum** | **Retentionszeit HPLC (Methode)** |
|---|---|---|---|---|
| **11.1** | 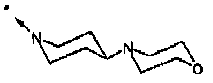 | 73 | 648 | 3.2 min |
| | | | [M+H]⁺ | (B) |
| **11.2** | | 48 | 733 | 3.0 min |
| | 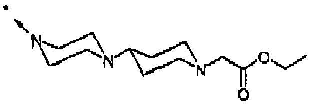 | | [M+H]⁺ | (B) |

### Beispiel 11.3

### 4-(2-Oxo-1,2,4,5-tetrahydro-1,3-benzdiazepin-3-yl)-pipeddin-1-carbonsäure-(R)-2-[4-(1-carboxymethyl-piperidin-4-yl)-piperazin-1-yl]-1-(3-ethyl-4-hydroxy-5-methyl-benzyl)-2-oxo-ethylester

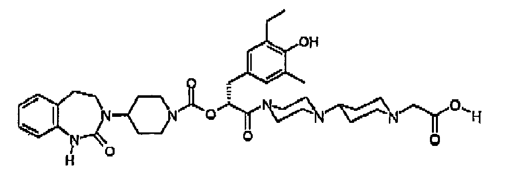

Zu einer Lösung von 73 mg (0.10 mmol) 4-(2-Oxo-1,2,4,5-tetrahydro-1,3-benzdiezepin-3-yl)-piperidin-1-carbonsäure-(*R*)-2-[4-(1-ethoxycarbonylmethyl-piperidin-4-yl)-piperazin-1-yl]-1-(3-ethyl-4-hydroxy-6-methy)-benzyl)-2-oxo-ethylester (Beispiel 11.2) in 5 mL THF wurde eine Lösung von 3.7 mg (0.15 mmol) LiOH in 3 mL Wasser gegeben und das Reaktionsgemisch 2 h bei RT gerührt. Man entfernte i.vac. das THF, versetzte mit 1 M HCl bis zur sauren Reaktion und engt erneut i.vac. ein. Der Rückstand wurde in DMF aufgenommen und via HPLC gereinigt; die das Produkt enthaltenden Fraktionen wurden vereinigt und lyophilisiert.
- Ausbeute:: 39 mg (56% der Theorie)
- ESI-MS:: (M+H)⁺ = 705
- Retentionszeit (HPLC-MS):: 2.9 min (Methode B)

Die nachfolgenden Beispiele beschreiben die Herstellung pharmazeutischer Anwendungsformen, die als Wirkstoff eine beliebige Verbindung der allgemeinen Formel I enthalten:

### Beispiel I

Kapseln zur Pulverinhalation mit 1 mg Wirkstoff

| | |
|---|---|
| Zusammensetzung: | |
| 1 Kapsel zur Pulverinhalation enthält: | |
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen, Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

| | |
|---|---|
| Zusammensetzung: | |
| 1 Hub enthält: | |
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren;

Der Wirkstoff und Benzalkoniumchlorid werden in Wasser gelöst und in Respimat^{®}-Kartuschen abgefüllt.

### Beispiel III

Inhalationslösung für Vemebler mit 1 mg Wirkstoff

| | |
|---|---|
| Zusammensetzung: | |
| 1 Fläschchen enthält; | |
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

| | |
|---|---|
| Zusammensetzung: | |
| 1 Hub enthält: | |
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

| | |
|---|---|
| Zusammensetzung: | |
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

| | |
|---|---|
| Zusammensetzung: | |
| Wirksubstanz | 5 mg |
| Glucose | 250 mg |
| Human-Ssrum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

| | |
|---|---|
| Zusammensetzung: | |
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

Lyophilisat mit 10 mg Wirksubstanz

| | |
|---|---|
| Zusammensetzung: | |
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen,

| Lösungsmittel für Lyophilisat: | |
|---|---|
| Polysorbat 80 = Tween 80 | 20 mg |
| Mannit | 200 mg |
| Wasser für Injektionszwecke ad | 10 ml |

### Herstellung;

Polysorbat 80 und Mannit in Wasser für Injektionszwecke (Wfl) auflösen; in Ampullen abfüllen.

### Beispiel IX

Tabletten mit 20 mg Wirksubstanz

| | |
|---|---|
| Zusammensetzung: | |
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg,

### Beispiel X

Kapseln mit 20 mg Wirksubstanz

| | |
|---|---|
| Zusammensetzung: | |
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel Xi

Zäpfchen mit 50 mg Wirksubstanz

| | |
|---|---|
| Zusammensetzung: | |
| Wirksubstanz | 50 mg |
| Hartfett (Adeps solidus) q.s. ad | 1700 mg |

### Herstellung:

Hartfett bei ca, 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

| | |
|---|---|
| Zusammensetzung: | |
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. CGRP-Antagonisten der allgemeinen Formel I 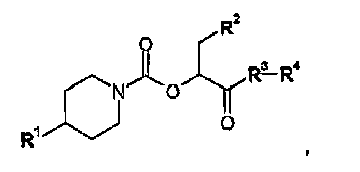 in der
**R¹** eine Gruppe ausgewählt aus 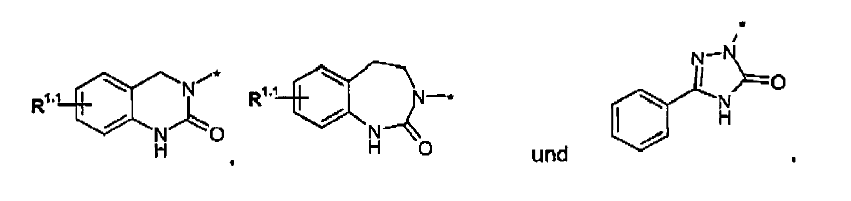 worin
**R^{1.1}** H oder H₃C-O- darstellt,
R² eine Gruppe der allgemeinen Formel II 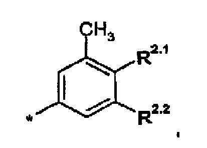 worin
**R^{2.1}** HO-, H₃CO-, H-C(O)-O- oder H₃C-C(O)-O- und
**R^{2,2}** C₁₋₂-Alkyl- oder H₃CO- darstellt,
**R³ R^{3.1}**-C₁₋₈-alkylenyl-NH- und
**R^{3.1}** H, H₂N-, C₁₋₃-Alkyl-NH-. (C₁₋₃-Alkyl)₂-N- oder C₁₋₈-Alkyl-O-C(O)-NH- darstellt oder
**R³** eine Gruppe der allgemeinen Formeln III 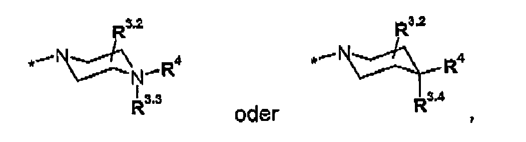 worin
**R^{3.2}** H oder C₁₋₃-Alkyl-,
**R^{3.3}** ein freies Elektronenpaar oder, wenn R⁴ nicht H darstellt oder über ein Kohlenstoffatom an **R³** gebunden ist, das Sauerstoffatom,
**R^{3.4}** H oder C₁₋₃-Alkyl- darstellt und
**R⁴** H, C₁₋₆-Alkyl-, H₂N-C₂₋₆-alkylenyl-, C₁₋₃-Alkyl-NH-C₂₋₆-alkylenyl-, (C₁₋₃-Alkyl)₂-N-C₁₋₆-alkylenyl-, C₃₋₆-Cycloalkyl-C₁₋₃-alkylenyl-, NH₂-, C₁₋₃-Alkyl-NH-oder (C₁₋₃-Alkyl)₂-N-, oder
**R⁴** eine Gruppe ausgewählt aus 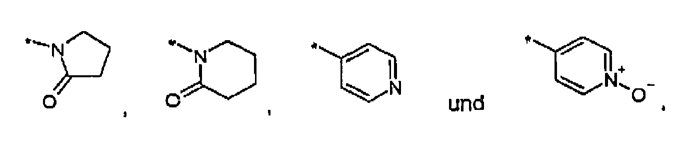 oder
**R**⁴ eine Gruppe der allgemeinen Formeln IV 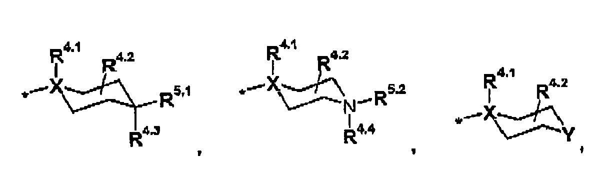 worin
**X** C und
**R^{4,1}** H, OH oder C₁₋₃-Alkyl- darstellt oder
**X** N und
**R^{4,1}** ein freies Elektronenpaar oder das Sauerstoffatom darstellt,
mit der Maßgabe, dass **X** nicht N ist, wenn **R⁴** über N an den Rest **R³** gebunden ist,
**Y** O, S, S(O), S(O)₂, wenn X = N ist, oder
**Y** S, S(O), S(O)₂, wenn X = C ist,
**R^{4.1}** H oder C₁₋₃-Alkyl-,
**R^{4.2}** H oder C₁₋₃-Alkyl-,
**R^{4.3}** H oder C₁₋₃-Alkyl-,
**R^{4.4}** ein freies Elektronenpaar oder, wenn **R^{5.2}** nicht H ist, das Sauerstoffatom,
**R^{5.1}** H, CN, OH, C₁₋₃-Alkyl-, C₁₋₃-Alkyl-C(O)-O-, C₁₋₃-Alkyl-O-, **R^{5.1.1}**-O-C(O)-, **R^{5.1.1}**-O-C(O)-C₂₋₄-alkylenyl- oder **R^{5.1.1}**-O-C(O)-C₁₋₃-alkylenyl-O-,
**R^{5.1.1}** H, C₁₋₆-Alkyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder C₁₋₃-Alkyl-O-C(O)-O-C₁₋₃-alkylenyl-,
**R^{5.2}** H, C₁₋₃-Alkyl-, C₃₋₆-Cycloalkyl-, C₃₋₆-Cycloalkyl-C₁₋₃-alkylenyl-, C₁₋₃-Alkyl-C(O)-, R^{5.2.1}-O-C(O)-C₁₋₃-alkylenyl- oder R^{5.2.2}-C₂₋₄-alkylenyl-4-C(O)-C₁₋₃-alkylenyl-,
**R^{5.2.1}** H, C₁₋₆-Alkyl-, C₁₋₆-Alkyl-C(O)-O-C₁₋₃-alkylenyl-, C₁₋₃-Alkyl-O-C(O)-O-C₁₋₃-alkylenyl-, C₃₋₇-Cycloalkyl-O-C(O)-O-C₁₋₃-alkylenyl-, H₂N-C(O)-C₁₋₃alkylenyl-, C₁₋₃-Alkyl-NH-C(O)-C₁₋₃-alkylenyl-, (C₁₋₃-Alkyl)₂-N-C(O)-C₁₋₃-alkylenyl- oder R^{5.2.1.1}-C(O)-C₁₋₃-alkylenyl- und
**R^{5.2.1.1}** eine Gruppe ausgewählt aus 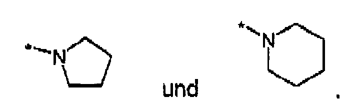 oder
**R^{5.2.1}** eine Gruppe ausgewählt aus 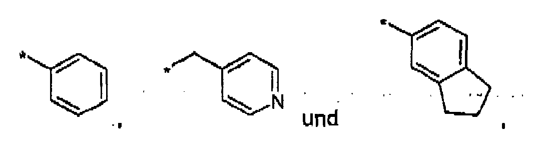
**R^{5.2.2}** H₂N, C₁₋₃-Alkyl-NH-, (C₁₋₃-Alkyl)₂-N-, C₁₋₃-Alkyl-O- oder C₁₋₃-Alkyl-O-C₂₋₄-alkylenyl-O-, oder
**R^{5.2.2}** eine Gruppe darstellt ausgewählt aus 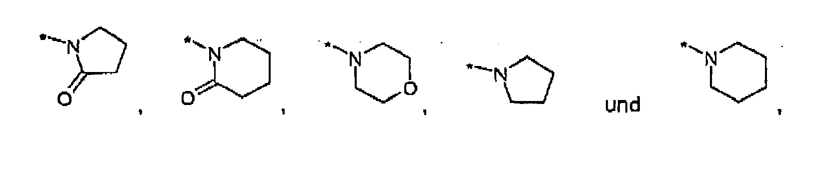
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

2. Verbindungen der allgemeinen Formel I gemäß Anspruch 1, in denen
**R¹** eine Gruppe ausgewählt aus 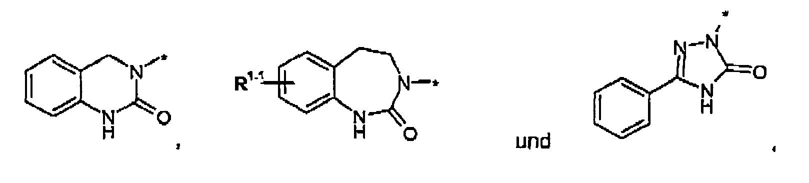 worin
**R^{1.1}** H oder H₃C-O- darstellt,
**R²** eine Gruppe der allgemeinen Formel II 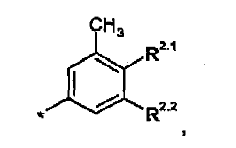 worin
**R^{2.1}** HO-, H₃CO-, H-C(O)-O- oder H₃C-C(O)-O- und
**R^{2.2}** C₁₋₂-Alkyl- oder H₃CO- darstellt,
**R³ R^{3.1}-**C₂₋₈-alkylenyl-NH- und
**R^{3.1}** H₂N-, C₁₋₃-Alkyl-NH-, (C₁₋₃-Alkyl)₂-N- oder C₁₋₆-Alkyl-O-C(O)-NH-, oder
**R³** eine Gruppe der allgemeinen Formeln III 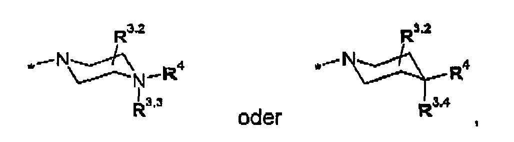 worin
**R^{3.2}** H,
**R^{3.3}** ein freies Elektronenpaar oder, wenn R⁴ nicht H darstellt oder über ein Kohlenstoffatom an R³ gebunden ist, das Sauerstoffatom,
**R^{3.4}** H oder C₁₋₃-Alkyl- darstellt,
**R⁴** H, C₁₋₆-Alkyl-, H₂N-C₂₋₆-alkylenyl-, C₁₋₃-Alkyl-NH-C₂₋₆-alkylenyl-, (C₁₋₃-Alkyl)₂-N-C₁₋₆-alkylenyl-, C₃₋₆-Cycloalkyl-C₁₋₃-alkylenyl-, H₂N-, C₁₋₃-Alkyl-NH- oder (C₁₋₃-Alkyl)₂-N-, oder
**R⁴** eine Gruppe ausgewählt aus 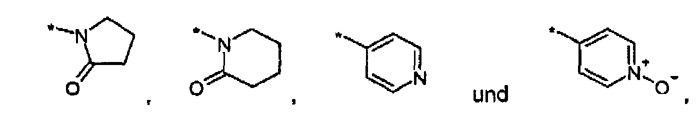 oder
**R⁴** eine Gruppe der allgemeinen Formeln IV 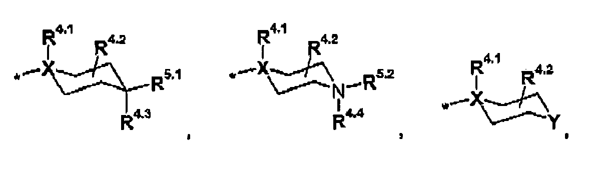 worin
**X** C und
**R^{4,1}** H, OH, C₁₋₃-Alkyl- oder
**X** N und
**R^{4.1}** ein freies Elektronenpaar oder das Sauerstoffatom darstellt,
mit der Maßgabe, dass **X** nicht N ist, wenn **R⁴** über N an den Rest **R³** gebunden ist,
**Y** O, S, S(O), S(O)₂, wenn **X** = N ist, oder
**Y** S, S(O), S(O)₂, wenn **X =** C ist,
**R^{4.1}** H oder C₁₋₃-Alkyl-,
**R^{4.2}** H,
**R^{4.3}** H oder C₁₋₃-Alkyl-,
**R^{4.4}** ein freies Elektronenpaar oder, wenn R^{5.2} nicht H ist, das Sauerstoffatom,
**R^{5.1}** H, CN, OH, C₁₋₃-Alkyl-, C₁₋₃-Alkyl-C(O)-O-, C₁₋₃-Alkyl-O-, R^{6.1.1}-O-C(O)-, R^{5.1.1}-O-C(O)-C₂₋₄-alkylenyl- oder R^{5.1.1}-O-C(O)-C₁₋₃-alkylenyl-O-,
**R^{5.1.1}** H, C₁₋₆-Alkyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder C₁₋₃-Adkyl-O-C(O)-O-C₁₋₃-alkylenyl-,
**R^{5.2}** H. C₁₋₃-Alkyl-, C₃₋₆-Cycloalkyl-, C₃₋₆-Cycloalkyl-C₁₋₃-alkylenyl-, C₁₋₃-Alkyl-C(O)-, R^{5.2.1}-O-C(O)-C₁₋₃-alkylenyl- oder R^{5.2.2}-C₂₋₄-alkylenyl-O-C(O)-C₁₋₃-alkylenyl-,
**R^{5.2.1}** H, C₁₋₆-Alkyl-, C₁₋₆-Alkyl-C(O)-O-C₁₋₃-alkylenyl-, C₁₋₃-Alkyl-O-C(O)-O-C₁₋₃-alkylenyl-, C₃₋₇-Cycloalkyl-O-C(O)-O-C₁₋₃-alkylenyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder R^{5.2.1.1}-C(O)-C₁₋₃-alkylenyl- und
**R^{5.2.1.1}** eine Gruppe ausgewählt aus 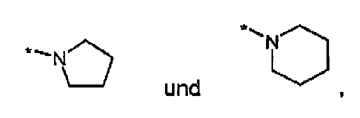 oder
**R^{5.2.1}** eine Gruppe ausgewählt aus 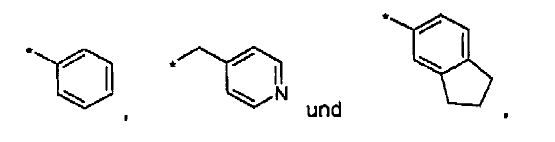
**R^{5.2.2}** H₂N, C₁₋₃-Alkyl-NH-, (C₁₋₃-Alkyl)₂-N-, C₁₋₃-Alkyl-O- oder C₁₋₃-Alkyl-O-C₂₋₄-alkylenyl-O-, oder
**R^{5.2.2}** eine Gruppe darstellt ausgewählt aus 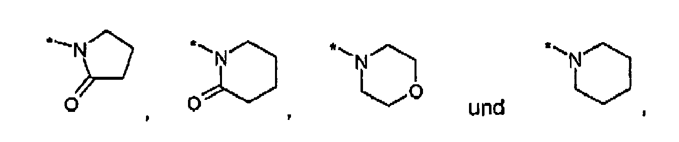
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel I gemäß Anspruch 1, in denen
**R¹** eine Gruppe ausgewählt aus 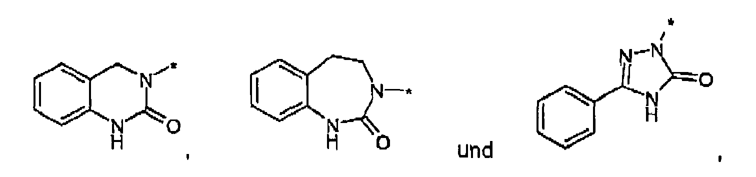
**R²** eine Gruppe ausgewählt aus 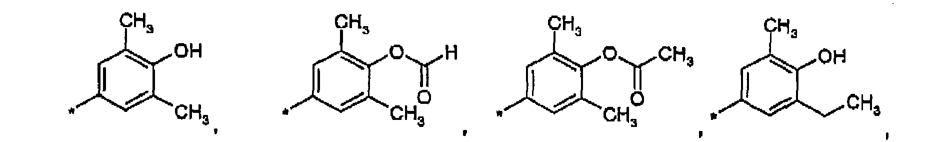 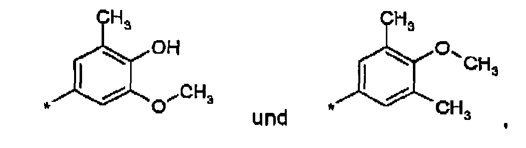
**R³** R^{3.1}-C₁₋₈-alkylenyl-NH- und
R^{3.1} H, H₂N-, (C₁₋₃-Alkyl)₂-N- oder C₁₋₄-Alkyl-O-C(O)-NH- darstellt, oder
**R³** eine Gruppe ausgewählt aus 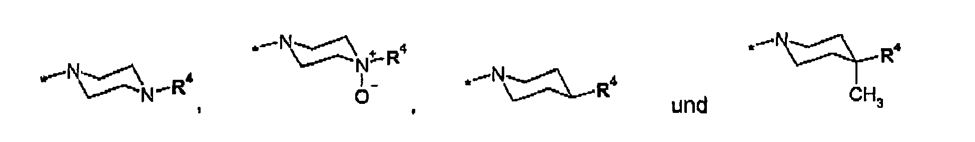 und
**R⁴** H, C₁₋₆-Alkyl-, (C₁₋₃-Alkyl)₂-N-C₁₋₆-alkylenyl-, C₃₋₆-Cycloalkyl-C₁₋₃-alkylenyl- oder (C₁₋₃-Alkyl)₂-N-, oder
**R⁴** eine Gruppe ausgewählt aus 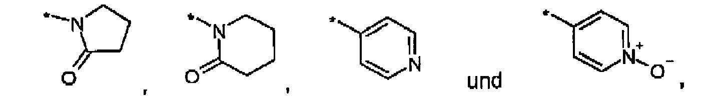 oder
**R⁴** eine Gruppe ausgewählt aus 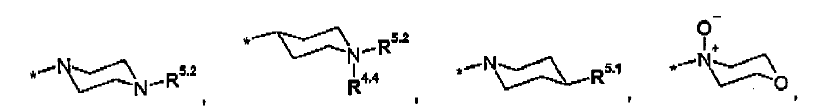 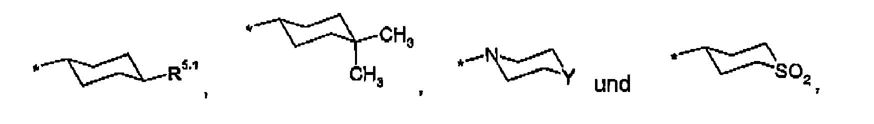 worin
**Y** O, S, S(O), S(O)₂,
**R^{5.1}** H, CN, OH, C₁₋₃-Alkyl-, C₁₋₃-Alkyl-C(O)-O-, C₁₋₃-Alkyl-O-, R^{5.1.1}-O-C(O)-, R^{5.1.1}-O-C(O)-C₂₋₄-alkylenyl- oder **R^{5.1.1}**-O-C(O)-C₁₋₃-alkylenyl-O-,
**R^{5.1.1}** H, C₁₋₆-Alkyl-, H₂N-C(O)-C₁₋₃-alkylenyl-, C₁₋₃-Alkyl-NH-C(O)-C₁₋₃-alkylenyl-, (C₁₋₃-Alkyl)₂-N-C(O)-C₁₋₃-alkylenyl- oder C₁₋₃-Alkyl-O-C(O)-O-C₁₋₃-alkylenyl-,
**R^{5.2}** H, C₁₋₃-Alkyl-, Cyclopropyl-, Cyclapropyl-methylenyl-, C₁₋₃-Alkyl-C(O)-, R^{5.2.1}-O-C(O)-C₁₋₃-alkylenyl- oder R^{5.2.2}-C₂₋₄-alkylenyl-O-C(O)-C₁₋₃-alkylenyl-,
**R^{5.2.1}** H, C₁₋₆-Alkyl-, C₁₋₆-Alkyl-C(O)-O-C₁₋₃-alkylenyl-, C₁₋₃Alkyl-O-C(O)-O-C₁₋₃-alkylenyl-, (C₁₋₃-Alkyl)₂-N-C(O)-C₁₋₃-alkylenyl- oder R^{5.2.1.1}-C(O)-C₁₋₃-alkylenyl- und
**R^{5.2.1.1}** eine Gruppe ausgewählt aus 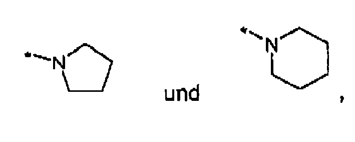 oder
**R^{5.2.1}** eine Gruppe ausgewählt aus 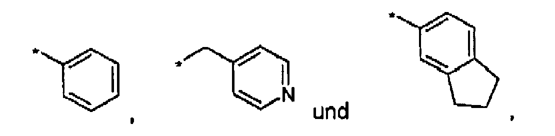
**R^{5.2.2}** (C₁₋₃-Alkyl)₂-N-, C₁₋₃-Alkyl-O- oder C₁₋₃-Alkyl-O-C₂₋₄-alkylenyl-O-, oder
**R^{5.2.2.}** eine Gruppe darstellt ausgewählt aus 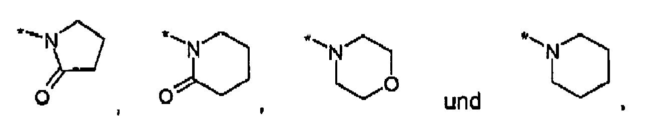
mit der Maßgabe, dass **R³** und **R⁴** nicht gleichzeitig über N aneinander gebunden sind,
bedeuten,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Physiologisch verträgliche Salze der Verbindungen nach einem der Ansprüche 1 bis 3 mit anorganischen oder organischen Säuren oder Basen.

5. Arzneimittel, enthaltend eine Verbindung nach mindestens einem der Ansprüche 1 bis 3 oder ein physiologisch verträgliches Salz gemäß Anspruch 4 neben gegebenenfalls einem oder mehreren inerten Trägerstoffen und/oder Verdonnungsmitteln.

6. Verwendung einer Verbindung nach mindestens einem der Ansprüche 1 bis 4 zur Herstellung eines Arzneimittels zur akuten und prophylaktischen Behandlung von Kopfschmerzen, insbesondere Migräne-, Cluster-Kopfschmerz sowie Spannungskopfschmerzen.

7. Verwendung einer Verbindung nach mindestens einem der Ansprüche 1 bis 4 zur Herstellung eines Arzneimittels zur Bekämpfung von nicht-insulinabhängigem Diabetesmellitus (NIDDM).

8. Verwendung einer Verbindung nach mindestens einem der Ansprüche 1 bis 4 zur Herstellung eines Arzneimittels zur Behandlung von cardiovaskulären Erkrankungen, von Morphintoleranz, von Chlostridiumtoxin-bedingten Durchfallerkrankungen, von Erkrankungen der Haut, insbesondere von thermischen und strahlungsbedingten Schäden inklusive Sonnenbrand, Lichen, prurigo, Pruriginöse Toxidermien sowie schwerer Juckreiz, von entzündlichen Erkrankungen, z.B. entzündlichen Gelenkerkrankungen wie Osteoarthritis, rheumatoide Arthritis oder neurogene Arthritis, generalisierten Weichteilrheumatismus (Fihromyalgie), von neurogenen Entzündungen der oralen Mucosa, von entzündlichen Lungenerkrankungen, von allergischer Rhinitis, von Asthma und COPD, von Erkrankungen, die mit einer überschießenden Gefäßerweiterung und **dadurch** bedingter verringerter Gefäßdurchblutung, wie insbesondere Schock oder Sepsis, einhergehen, von chronischen Schmerzerkrankungen wie z.B. diabetische Neuropathien, durch Chemotherapien induzierte Neuropathien, HIV-induzierte Neuropathien, postherpetische Neuropathien, durch Gewebetraumata induzierte Neuropathien, trigeminale Neuralgien, von temporomandibulären Dysfunktionen, von CRPS, Rückenschmerzen, von viszeralen Erkrankungen wie z.B, IBS (irritable bowel Syndrome) oder inflammable bowel syndrome, zur Linderung von Schmerzzuständen im allgemeinen oder zu präventiven oder akut therapeutischen Beeinflussung der durch Gefäßerweiterung und erhöhten Blutfluss verursachten Symptomatik von Hitzewallungen menopausaler, östrogendefizienter Frauen sowie hormonbeharldelter Prostatakarzinompatienten und Kastraten.

9. Verfahren zur Herstellung eines Arzneimittels gemäß Anspruch 5, **dadurch gekennzeichnet, dass** auf nichtchemischem Weg eine Verbindung nach mindestens einem der Ansprüche 1 bis 4 in einen oder mehrere inerte Trägerstoffe und/oder Verdünnungsmittel eingearbeitet wird.
